# EUROPEAN PATENT SPECIFICATION

(11) **EP 4 581 160 B1**
(45) Date of publication and mention of the grant of the patent: **01.07.2026**
(21) Application number: 24769493.8
(22) Date of filing: 22.08.2024
(51) Int. Cl.: C12Q 1/6827, C12Q 1/6837, C12Q 1/6841

(54) **METHODS, KITS, AND COMPOSITIONS FOR SPATIAL DETECTION OF GENETIC VARIANTS**
VERFAHREN, KITS UND ZUSAMMENSETZUNGEN FÜR DEN RÄUMLICHEN NACHWEIS GENETISCHER VARIANTEN
PROCÉDÉS, KITS ET COMPOSITIONS AUX FINS D'UNE DÉTECTION SPATIALE DE VARIANTS GÉNÉTIQUES

(30) Priority: 24.08.2023 US 202363578622 P
(43) Date of publication of application: 09.07.2025
(73) Proprietor: 10X Genomics, Inc., Pleasanton, CA 94588-3260 (US)
(72) Inventor: MIGNARDI, Marco, 114 33 Stockholm (SE); BORGSTROM, Erik Leonard Henrik, 114 33 Stockholm (SE); BJORNINEN, Anton Jan, 114 33 Stockholm (SE)
(74) Representative: J A Kemp LLP
(86) International application number: PCT/US2024/043399
(87) International publication number: WO 2025/043076

(56) References cited:
- WO-A1-2021/133849
- WO-A1-2021/216708
- WO-A2-2011/071923

## Description

### BACKGROUND

Cells within a tissue of a subject have differences in cell morphology and/or function due to varied analyte levels (e.g., gene and/or protein expression) within the different cells. The specific position of a cell within a tissue (e.g., the cell's position relative to neighboring cells or the cell's position relative to the tissue microenvironment) can affect, e.g., the cell's morphology, differentiation, fate, viability, proliferation, behavior, signaling and cross-talk with other cells in the tissue.

Spatial heterogeneity has been previously studied using techniques that only provide data for a small handful of analytes in the context of an intact tissue or a portion of a tissue, or provides substantial analyte data for dissociated tissue (i.e., single cells), but fail to provide information regarding the position of the single cell in a parent biological sample (e.g., tissue sample).

Generally, spatial analysis takes advantage of targeting a particular analyte such as mRNA in a sample using a capture probe with a poly(T) capture domain. However, this approach is capable of detecting a high number of off-target analytes. Similarly, detecting genetic variants via this method can be challenging, particularly if the genetic variant is near the 5' end of the target nucleic acid. Methods including templated-ligation are generally more specific since probe pairs are designed for specific target nucleic acids. Nevertheless, there remains a need to spatially detect genetic variants with improved methods for template-ligation.

WO 2011/071923 presents a method for determining the presence of multiple nucleotide sequences of interest in multiple samples while preserving the identity of each sample, by contacting the samples with a plurality of probe sets.

WO 2021/216708 presents methods for spatial analysis using targeted RNA depletion.

### SUMMARY OF THE INVENTION

This disclosure features improved methods of templated-ligation (e.g., RNA-templated ligation (RTL)) to increase detection of genetic variants within a target nucleic acid. Templated ligation seeks to increase the possibility of detecting target-specific mutations in a target nucleic acid through hybridization of multiple (e.g., two) oligonucleotides, or probes, that are ligated to form one oligonucleotide product that can be captured by a capture probe on a spatial array. More specifically, this disclosure features improvements to templated ligation that increases the efficiency of detecting genetic variants such as single-nucleotide polymorphisms, insertions, and/or deletions. Generally, probe pairs hybridize to adjacent or nearly adjacent sequences on a target nucleic acid and are ligated to one another, or, in the case where the probes are non-adjacent a gap-filling reaction can be performed prior to ligation. However, some limitations of this method include non-specific binding of one of the probes near a genetic variant. The present disclosure features improved methods where a first probe hybridizes to a target nucleic acid and is extended with a reversible terminator nucleotide. This single nucleotide extension step can provide increased specificity over current templated-ligation methods. Various reversible terminator nucleotides can be included in the methods described herein, including 3' blocked and unblocked reversible terminator nucleotides. In some examples, the probe can be extended with a second, a third, a fourth, a fifth, or more reversible terminator nucleotides.

Thus provided herein are methods for determining presence or absence of a genetic variant in a biological sample, the method including: (a) providing an array comprising a plurality of capture probes, wherein a capture probe of the plurality of capture probes comprises: (i) a spatial barcode and (ii) a capture domain; (b) contacting the biological sample with a plurality of first probes, wherein a first probe of the plurality of first probes comprises a sequence substantially complementary to a first sequence of a target nucleic acid in the biological sample; (c) hybridizing the first probe to the target nucleic acid and extending the first probe using a reversible terminator nucleotide, thereby generating an extended first probe; (d) contacting the biological sample with a plurality of second probes, wherein a second probe of the plurality of second probes comprises a sequence substantially complementary to a second sequence of the target nucleic acid in the biological sample; (e) hybridizing the second probe to the target nucleic acid and ligating the extended first probe to the second probe, thereby generating a ligation product; and (f) determining the presence or absence of the ligation product, thereby determining the presence or absence of the genetic variant in the biological sample.

In some embodiments, the extending includes use of a polymerase. In some embodiments, the polymerase includes Bst 3 polymerase or Klenow polymerase.

In some embodiments, the first probe includes DNA. In some embodiments, the second probe includes DNA.

In some embodiments, the biological sample is contacted with the second plurality of probes simultaneously with the first plurality of probes. In some embodiments, the biological sample is contacted with the second plurality of probes after the extending in (c).

In some embodiments, the reversible terminator nucleotide comprises a 3' blocking group. In some embodiments, the 3' blocking group is a 3'-O-blocked reversible terminator nucleotide. In some embodiments, the 3'-O-blocked reversible terminator nucleotide includes a 3'-azidomethyl dNTP, a 3'-O-allyl dNTP, a 3'-O-nitrobenzyl dNTP, a 3'-O-(2-nitrobenzyl) dNTP, or a 3'-O-dithiomethyl dNTP.

In some embodiments, the reversible terminator nucleotide comprises an unblocked reversible terminator nucleotide. In some embodiments, the unblocked reversible terminator nucleotide comprises a 5-hydroxymethyl-2'-deoxy triphosphate derivative.

In some embodiments, the ligating in (e) is performed using a ligase, and optionally, the ligase is a PBCV-1 ligase, a *Chlorella* DNA ligase, a single stranded DNA ligase, or a T4 DNA ligase. In some embodiments, the ligase is a T4 DNA ligase or a *Chlorella* DNA ligase.

In some embodiments, the target nucleic acid is DNA. In some embodiments, the target nucleic acid is RNA. In some embodiments, the RNA is mRNA.

In some embodiments, the method further comprises removing the 3' blocking group after the extending in (c). In some embodiments, the removing comprises reducing the 3' blocking group using a reducing agent. In some embodiments, the reducing agent includes tris(2-carboxyethyl)phosphine (TCEP) or dithiothreitol (DTT). In some embodiments, the removing comprises cleaving the 3' blocking group from the reversible terminator nucleotide.

In some embodiments, the method further comprises removing the target nucleic acid from the ligation product. In some embodiments, the removing includes use of an RNase. In some embodiments, the removing includes denaturation. In some embodiments, denaturation includes use of potassium hydroxide or heat.

In some embodiments, the genetic variant is one or more single nucleotide polymorphisms. In some embodiments, the genetic variant is an insertion or deletion mutation.

In some embodiments, the determining in (f) comprises sequencing. In some embodiments, the determining comprises fluorescent detection. In some embodiments, the fluorescent detection includes use of one or more labeled probes.

In some embodiments, the capture probe comprises one or more functional domains, a cleavage domain, a unique molecular identifier (UMI), or a combination thereof. In some embodiments, the one or more functional domains includes a primer binding site or a sequencing specific site.

In some embodiments, the second probe includes a capture probe capture domain. In some embodiments, the capture probe capture domain is substantially complementary to the capture domain of the capture probe.

In some embodiments, the biological sample is disposed on the array. In some embodiments, the biological sample is disposed on a substrate. In some embodiments, the method further comprises aligning the substrate including the biological sample with the array, such that at least a portion of the biological sample is aligned with at least a portion of the array.

In some embodiments, the method includes migrating the target nucleic acid from the biological sample to the array, and optionally, the migrating includes electrophoresis.

In some embodiments, the capture domain of the capture probe includes a poly(T) sequence. In some embodiments, the capture domain of the capture probe includes a fixed sequence.

In some embodiments, the method further comprises hybridizing the ligation product to the capture domain of the capture probe. In some embodiments, the capture probe is extended using the ligation product as a template. In some embodiments, the ligation product is extended using the capture probe as a template.

In some embodiments, the method further comprises determining (i) all or a portion of the sequence of the ligation product, or a complement thereof, and (ii) the sequence of the spatial barcode, or a complement thereof, and using the determined sequences of (i) and (ii) to determine a location of the genetic variant in the biological sample. In some embodiments, the determining includes sequencing. In some embodiments, the sequencing includes high-throughput sequencing.

In some embodiments, the method includes washing the biological sample after (e) to remove unhybridized first probes and unhybridized second probes.

In some embodiments, the method includes permeabilizing the biological sample. In some embodiments, the permeabilizing includes use of a protease. In some embodiments, the protease includes pepsin. In some embodiments, the protease includes proteinase K.

In some embodiments, the biological sample is fixed. In some embodiments, the biological sample is methanol-fixed, acetone-fixed, paraformaldehyde-fixed, or formalin-fixed paraffin-embedded (FFPE).

In some embodiments, the method further comprises staining the biological sample. In some embodiments, the staining includes use of immunofluorescence, immunohistochemistry, hematoxylin, or eosin.

In some embodiments, the method further comprises imaging the biological sample. In some embodiments, the biological sample is a tissue sample. In some embodiments, the tissue sample is a fresh-frozen tissue sample. In some embodiments, the biological sample is a tissue section. In some embodiments, the tissue section is a fresh-frozen tissue section.

To the extent publications, patents, patent applications, and items of information contradict the disclosure contained in the specification, the specification is intended to supersede and/or take precedence over any such contradictory material.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated.

The term "about" or "approximately" as used herein means within an acceptable error range for the particular value as determined by one of ordinary skill in the art, which will depend in part on how the value is measured or determined, i.e., the limitations of the measurement system. For example, "about" can mean within an acceptable standard deviation, per the practice in the art. Alternatively, "about" can mean a range of up to ±20%, preferably up to ±10%, more preferably up to ±5%, and more preferably still up to ±1% of a given value. Alternatively, particularly with respect to biological systems or processes, the term can mean within an order of magnitude, preferably within 2-fold, of a value. Where particular values are described in the application and claims, unless otherwise stated, the term "about" is implicit and in this context means within an acceptable error range for the particular value.

The term "substantially complementary" used herein means that a first sequence is at least 50%, 55%, 60%, 65%, 70%, 75%, 80%, 85%, 90%, 95%, 97%, 98%, or 99% identical to the complement of a second sequence over a region of 2, 3, 4, 5, 6, 7, 8, 9, 10, 11, 12, 13, 14, 15, 16, 17, 18, 19, 20-40, 40-60, 60-100, or more nucleotides, or that the two sequences hybridize under stringent hybridization conditions. Substantially complementary also means that a sequence in one strand is not completely and/or perfectly complementary to a sequence in an opposing strand, but that sufficient bonding occurs between bases on the two strands to form a stable hybrid complex in set of hybridization conditions (e.g., salt concentration and temperature). Such conditions can be predicted by using the sequences and standard mathematical calculations known to those skilled in the art.

The term "each," when used in reference to a collection of items, is intended to identify an individual item in the collection but does not necessarily refer to every item in the collection, unless expressly stated otherwise, or unless the context of the usage clearly indicates otherwise.

### DESCRIPTION OF DRAWINGS

The following drawings illustrate certain embodiments of the features and advantages of this disclosure. Like reference symbols in the drawings indicate like elements.
**FIG. 1A** shows an exemplary sandwiching process where a first substrate (e.g., a slide), including a biological sample, and a second substrate (e.g., array slide) are brought into proximity with one another.
**FIG. 1B** shows a fully formed sandwich configuration creating a chamber formed from the one or more spacers, the first substrate, and the second substrate.
**FIG. 2A** shows a perspective view of an exemplary sample handling apparatus in a closed position.
**FIG. 2B** shows a perspective view of an exemplary sample handling apparatus in an open position.
**FIG. 3A** shows the first substrate angled over (superior to) the second substrate.
**FIG. 3B** shows that as the first substrate lowers, and/or as the second substrate rises, the dropped side of the first substrate may contact a drop of reagent medium.
**FIG. 3C** shows a full closure of the sandwich between the first substrate and the second substrate with one or more spacers contacting both the first substrate and the second substrate.
**FIG. 4A** shows a side view of the angled closure workflow.
**FIG. 4B** shows a top view of the angled closure workflow.
**FIG. 5** is a schematic diagram showing an example of a barcoded capture probe, as described herein.
**FIG. 6** shows a schematic illustrating a cleavable capture probe.
**FIG. 7** shows exemplary capture domains on capture probes.
**FIG. 8** shows an exemplary arrangement of barcoded features within an array.
**FIG. 9A** shows an exemplary workflow for performing templated capture and producing a ligation product.
**FIG. 9B** shows an exemplary workflow for capturing a ligation product from **FIG. 9A** on a substrate.
**FIG. 10** is a schematic diagram of an exemplary analyte capture agent.
**FIG. 11** is a schematic diagram depicting an exemplary interaction between a feature-immobilized capture probe **1124** and an analyte capture agent **1126.**
**FIG. 12** is a schematic diagram depicting genetic variant detection after hybridization of a first probe, extension with a reversible terminator nucleotide, hybridization of a second probe, and ligation.
**FIGs. 13A-13B** are graphs showing unique molecular identifier counts for various genes with different polymerases, different hybridization durations, and hybridization temperatures during 3' blocking group removal using TCEP. **FIG. 13A** shows results after five hours of hybridization and **FIG. 13B** shows results after overnight hybridization.
**FIGs. 14A-14B** show probe ligation efficiency (i.e., sensitivity) (**FIG. 14A**) and specificity (**FIG. 14B**) for various genes with different polymerases at different hybridization temperatures by the method of **FIG. 12****.**
**FIG. 15** is a schematic diagram showing the experimental set up of **FIG. 12** with different polymerases and with either reversible terminator nucleotides or dNTPs.
**FIGs. 16A-16B** are graphs showing unique molecular identifier percent capture efficiency with Bst 3 polymerase and reversible terminator nucleotides.
**FIGs. 17A-17B** are graphs showing the specificity of the method shown in **FIG. 12****.**
**FIGs. 18A-18C** are graphs showing mutation detection in specific genes. The graphs show detection of various mutant alleles including wildtype (control).
**FIG. 19** shows an exemplary spike-in probe design for studying specificity and efficiency of single nucleotide polymorphism (SNP) spike-in probes to a transcriptomic spatial assay. The control probes included 800 RTL wildtype (wt) selected probe pairs and 400 spike-in probes pairs wherein the LHS RTL probe includes a mismatch at the 3' end. RTL wt probes were used for data normalization.
**FIG. 20** shows two replicate specificity plots demonstrating percent specificity of spike-in genetic variant probes compared to wt probes (100% line).
**FIG. 21** shows two replicate efficiency of ligation plots for genetic variant probes compared to wt probes (100% line).
**FIG. 22** describes the experimental workflow for performing the two-step hybridization scenario for mismatch detection.

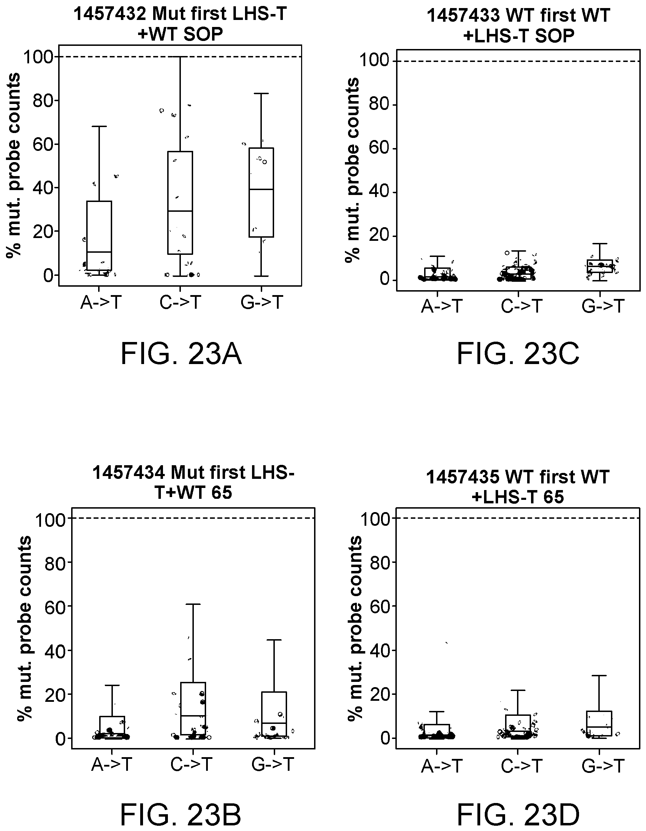

**FIGs. 23A-23D** shows exemplary specific graphs when running a two-step hybridization scenario for genetic variant detection. **FIG. 23A****:** mismatch probes were hybridized first, followed by post-ligation wash temperature of 57°C, followed by wt probe hybridization, **FIG. 23B****:** same as for **FIG. 23A** except the post-ligation wash was at 65°C, **FIG. 23C****:** wt RTL probes were hybridized first, followed by post-ligation wash of 57°C, followed by RTL genetic variant probe hybridization, **FIG. 23D****:** same as for **FIG. 23C****,** except the first post-ligation wash was at 65°C.
**FIGs. 24A-24D** shows exemplary efficiency graphs when running a two-step hybridization scenario for genetic variant detection. **FIG. 24A-D** conditions were the same as for **FIG. 23A-23D****.**
**FIGs. 25A-25B** shows the experimental probe design for **A)** asymmetric probe scenario, and **B)** gap fill probe scenario, for genetic variant detection.
**FIGs. 26A-26B** shows exemplary specificity and efficiency graphs where asymmetric RTL probes were used for genetic variant detection. The asymmetric probe scenario includes a LHS probe that is 19nt long, compared to the RHS probe that is 25nt long. The LHS shorter probe has one of three sequence options; **FIG. 26A****)** LHS probe with an internal mismatch at -9nt from the 3' end, **FIG. 26B****)** LHS probe with a 3' end mismatch, or wt sequence (control).
**FIG. 27** shows exemplary specificity and efficiency plots using a gap fill scenario for genetic variant detection. The LHS and RHS probes of the RTL probe pairs are both 20nt long, there is a gap between where the LHS probe hybridizes to the target nucleic acid and where the RHS probe hybridizes to the target nucleic acid, and there is a third probe that hybridizes in the gap, wherein the third probe includes wt or one or more mismatch nucleotides.

### DETAILED DESCRIPTION OF THE DISCLOSURE

The technical information set out below may in some respects go beyond the disclosure of the invention, which is defined by the appended claims. The additional technical information is provided to place the invention in a broader technical context and to illustrate possible related technical developments. Such additional technical information which does not fall within the scope of the appended claims is not part of the invention. Accordingly, any aspects, embodiments and examples of the present disclosure which do not fall under the scope of the appended claims do not form part of the invention and are merely provided for illustrative purposes.

### A. Spatial Analysis Methods

Spatial analysis methodologies described herein can provide a vast amount of analyte and/or expression data for a variety of analytes within a biological sample at high spatial resolution, while retaining native spatial context. Spatial analysis methods can include, e.g., the use of a capture probe including a spatial barcode (e.g., a nucleic acid sequence that provides information as to the location or position of an analyte within a cell or a tissue sample (e.g., mammalian cell or a mammalian tissue sample) and a capture domain that is capable of binding to an analyte (e.g., a protein and/or a nucleic acid) produced by and/or present in a cell. Spatial analysis methods and compositions can also include the use of a capture probe having a capture domain that captures an intermediate agent for indirect detection of an analyte. For example, the intermediate agent can include a nucleic acid sequence (e.g., a barcode) associated with the intermediate agent. Detection of the intermediate agent is therefore indicative of the analyte in the cell or tissue sample.

Non-limiting aspects of spatial analysis methodologies and compositions are described in U.S. Patent Nos. 11,447,807, 11,352,667, 11,168,350, 11,104,936, 11,008,608, 10,995,361, 10,913,975, 10,774,374, 10,724,078, 10,640,816, 10,494,662, 10,480,022, 10,364,457, 10,317,321, 10,059,990, 10,041,949, 10,030,261, 10,002,316, 9,879,313, 9,783,841, 9,727,810, 9,593,365, 8,951,726, 8,604,182, and 7,709,198; U.S. Patent Application Publication Nos. 2020/0239946, 2020/0080136, 2020/0277663, 2019/0330617, 2020/0256867, 2020/0224244, 2019/0085383, and 2013/0171621; PCT Publication Nos. WO2018/091676, WO2020/176788, WO2017/144338, and WO2016/057552; Non-patent literature references Rodriques et al., Science 363(6434):1463-1467, 2019; Lee et al., Nat. Protoc. 10(3):442-458, 2015; Trejo et al., PLoS ONE 14(2):e0212031, 2019; Chen et al., Science 348(6233):aaa6090, 2015; Gao et al., BMC Biol. 15:50, 2017; and Gupta et al., Nature Biotechnol. 36:1197-1202, 2018; and the Visium Spatial Gene Expression Reagent Kits User Guide (e.g., Rev F, dated January 2022); and/or the Visium Spatial Gene Expression Reagent Kits - Tissue Optimization User Guide (e.g., Rev E, dated February 2022), both of which are available at the 10x Genomics Support Documentation website, and can be used herein in any combination. Further non-limiting aspects of spatial analysis methodologies and compositions are described herein.

Some general terminology that may be used in this disclosure can be found in Section (I)(b) of PCT Publication No. WO2020/176788 and/or U.S. Patent Application Publication No. 2020/0277663. Typically, a "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes. For the purpose of this disclosure, an "analyte" can include any biological substance, structure, moiety, or component to be analyzed. The term "target" can similarly refer to an analyte of interest.

Analytes can be broadly classified into one of two groups: nucleic acid analytes and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral proteins (e.g., viral capsid, viral envelope, viral coat, viral accessory, viral glycoproteins, viral spike, etc.), extracellular and intracellular proteins, antibodies, and antigen binding fragments. In some embodiments, the analyte(s) can be localized to subcellular location(s), including, for example, organelles, e.g., mitochondria, Golgi apparatus, endoplasmic reticulum, chloroplasts, endocytic vesicles, exocytic vesicles, vacuoles, lysosomes, etc. In some embodiments, analyte(s) can be peptides or proteins, including without limitation antibodies and enzymes. Additional examples of analytes can be found in Section (I)(c) of PCT Publication No. WO2020/176788 and/or U.S. Patent Application Publication No. 2020/0277663. In some embodiments, an analyte can be detected indirectly, such as through detection of an intermediate agent, for example, a ligation product or an analyte capture agent (e.g., an oligonucleotide-conjugated antibody), such as those described herein.

A "biological sample" is typically obtained from the subject for analysis using any of a variety of techniques including, but not limited to, biopsy, surgery, and laser capture microscopy (LCM), and generally includes cells and/or other biological material from the subject. In some embodiments, the biological sample is a tissue sample. In some embodiments, the biological sample (e.g., tissue sample) is a tissue microarray (TMA). A tissue microarray contains multiple representative tissue samples - which can be from different tissues or organisms - assembled on a single histologic slide. The TMA can therefore allow for high throughput analysis of multiple specimens at the same time. Tissue microarrays are paraffin blocks produced by extracting cylindrical tissue cores from different paraffin donor blocks and re-embedding these tissue cores into a single recipient (microarray) block at defined array coordinates.

The biological sample as used herein can be any suitable biological sample described herein or known in the art. In some embodiments, the biological sample is a tissue sample. In some embodiments, the tissue sample is a solid tissue sample. In some embodiments, the biological sample is a tissue section. In some embodiments, the tissue is flash-frozen and sectioned. Any suitable method described herein or known in the art can be used to flash-freeze and section the tissue sample. In some embodiments, the biological sample, e.g., the tissue, is flash-frozen using liquid nitrogen before sectioning. In some embodiments, the biological sample, e.g., a tissue sample, is flash-frozen using nitrogen (e.g., liquid nitrogen), isopentane, or hexane.

In some embodiments, the biological sample, e.g., the tissue, is embedded in a matrix e.g., optimal cutting temperature (OCT) compound to facilitate sectioning. OCT compound is a formulation of clear, water-soluble glycols and resins, providing a solid matrix to encapsulate biological (e.g., tissue) specimens. In some embodiments, the sectioning is performed by cryosectioning, for example using a microtome. In some embodiments, the methods further comprise a thawing step, after the cryosectioning.

The biological sample can be from a mammal. In some instances, the biological sample is from a human, mouse, or rat. In addition to the subjects described above, the biological sample can be obtained from non-mammalian organisms (e.g., a plant, an insect, an arachnid, a nematode (e.g., *Caenorhabditis elegans),* a fungus, an amphibian, or a fish (e.g., zebrafish)). A biological sample can be obtained from a prokaryote such as a bacterium, e.g., *Escherichia coli, Staphylococci* or *Mycoplasma pneumoniae;* an archaeon; a virus, such as Hepatitis C virus or human immunodeficiency virus; or a viroid. A biological sample can be obtained from a eukaryote, such as a patient derived organoid (PDO) or patient derived xenograft (PDX). The biological sample can include organoids, a miniaturized and simplified version of an organ produced *in vitro* in three dimensions that shows realistic micro-anatomy. Organoids can be generated from one or more cells from a tissue, embryonic stem cells, and/or induced pluripotent stem cells, which can self-organize in three-dimensional culture owing to their self-renewal and differentiation capacities. In some embodiments, an organoid is a cerebral organoid, an intestinal organoid, a stomach organoid, a lingual organoid, a thyroid organoid, a thymic organoid, a testicular organoid, a hepatic organoid, a pancreatic organoid, an epithelial organoid, a lung organoid, a kidney organoid, a gastruloid, a cardiac organoid, or a retinal organoid. Subjects from which biological samples can be obtained can be healthy or asymptomatic individuals, individuals that have or are suspected of having a disease (e.g., cancer) or a pre-disposition to a disease, and/or individuals that are in need of therapy or suspected of needing therapy.

Biological samples can be derived from a homogeneous culture or population of the subjects or organisms mentioned herein or alternatively from a collection of several different organisms, for example, in a community or ecosystem.

Biological samples can include one or more diseased cells. A diseased cell can have altered metabolic properties, gene expression, protein expression, and/or morphologic features. Examples of diseases include inflammatory disorders, metabolic disorders, nervous system disorders, and cancer. Cancer cells can be derived from solid tumors, hematological malignancies, cell lines, or obtained as circulating tumor cells.

In some embodiments, the biological sample, e.g., the tissue sample, is fixed in a fixative including alcohol, for example methanol. In some embodiments, instead of methanol, acetone, or an acetone-methanol mixture can be used. In some embodiments, the fixation is performed after sectioning. In some instances, when the biological sample is fixed using a fixative including an alcohol (e.g., methanol or acetone-methanol mixture), the biological sample is not decrosslinked afterward. In some preferred embodiments, the biological sample is fixed using a fixative including an alcohol (e.g., methanol or an acetone-methanol mixture) after freezing and/or sectioning. In some instances, the biological sample is flash-frozen, and then the biological sample is sectioned and fixed (e.g., using methanol, acetone, or an acetone-methanol mixture). In some instances when methanol, acetone, or an acetone-methanol mixture is used to fix the biological sample, the sample is not decrosslinked at a later step. In instances when the biological sample is frozen (e.g., flash frozen using liquid nitrogen and embedded in OCT) followed by sectioning and alcohol (e.g., methanol, acetone-methanol) fixation or acetone fixation, the biological sample is referred to as "fresh frozen". In some embodiments, fixation of the biological sample e.g., using acetone and/or alcohol (e.g., methanol, acetone-methanol) is performed while the sample is mounted on a substrate (e.g., glass slide, such as a positively charged glass slide).

In some embodiments, the biological sample, e.g., the tissue sample, is fixed e.g., immediately after being harvested from a subject. In such embodiments, the fixative is preferably an aldehyde fixative, such as paraformaldehyde (PFA) or formalin. In some embodiments, the fixative induces crosslinks within the biological sample. In some embodiments, after fixing e.g., by formalin or PFA, the biological sample is dehydrated via sucrose gradient. In some instances, the fixed biological sample is treated with a sucrose gradient and then embedded in a matrix e.g., OCT compound. In some instances, the fixed biological sample is not treated with a sucrose gradient, but rather is embedded in a matrix e.g., OCT compound after fixation. In some embodiments, when a fixed frozen tissue sample is treated with a sucrose gradient, the sample can be rehydrated with an ethanol gradient. In some embodiments, the PFA or formalin fixed biological sample, which can be optionally dehydrated via sucrose gradient and/or embedded in OCT compound, is then frozen e.g., for storage or shipment. In such instances, the biological sample is referred to as "fixed frozen". In preferred embodiments, a fixed frozen biological sample is not treated with methanol. In preferred embodiments, a fixed frozen biological sample is not paraffin embedded. Thus, in preferred embodiments, a fixed frozen biological sample is not deparaffinized. In some embodiments, a fixed frozen biological sample is rehydrated using an ethanol gradient.

In some instances, the biological sample (e.g., a fixed frozen tissue sample) is treated with a citrate buffer. Citrate buffer can be used to decrosslink antigens and fixation medium in the biological sample for antigen retrieval. Thus, any suitable decrosslinking agent can be used in addition to or alternatively to citrate buffer. In some embodiments, for example, the biological sample (e.g., a fixed frozen tissue sample) is decrosslinked using TE buffer.

In any of the foregoing, the biological sample can further be stained, imaged, and/or destained. For example, in some embodiments, a fresh frozen tissue sample or fixed frozen tissue sample is stained (e.g., via eosin and/or hematoxylin), imaged, destained (e.g., via HCl), or a combination thereof. In some embodiments, when a fresh frozen tissue sample is fixed in methanol, the sample is treated with isopropanol prior to being stained (e.g., via eosin and/or hematoxylin), imaged, destained (e.g., via HCl), or a combination thereof. In some embodiments when a fixed frozen tissue sample is treated with a sucrose gradient, the sample can be rehydrated using an ethanol gradient before being stained, (e.g., via eosin and/or hematoxylin), imaged, destained (e.g., via HCl), decrosslinked (e.g., via TE buffer or citrate buffer), or a combination thereof. In some embodiments, the biological sample can undergo further fixation (e.g., while mounted on a substrate), stained, imaged, and/or destained. For example, a fixed frozen biological sample may be subject to an additional fixing step (e.g., using PFA) before optional ethanol rehydration, staining, imaging, and/or destaining.

In any of the foregoing, the biological sample can be fixed using PAXgene. For example, the biological sample can be fixed using PAXgene in addition, or alternatively to, a fixative disclosed herein (e.g., alcohol, acetone, acetone-alcohol, formalin, paraformaldehyde). PAXgene is a non-cross-linking mixture of different alcohols, an acid, and a soluble organic compound that preserves morphology of biomolecules. PAXgene provides a two-reagent fixative system in which tissue is firstly fixed in a solution containing methanol and acetic acid then stabilized in a solution containing ethanol. See, Ergin B. et al., J Proteome Res. 2010 Oct 1;9(10):5188-96; Kap M. et al., PLoS One.; 6(11):e27704 (2011); and Mathieson W. et al., Am J Clin Pathol.; 146(1):25-40 (2016) for a description and evaluation of PAXgene for tissue fixation. Thus, in some embodiments, when the biological sample, e.g., the tissue sample, is fixed in a fixative including alcohol, the fixative is PAXgene. In some embodiments, a fresh frozen tissue sample is fixed with PAXgene. In some embodiments, a fixed frozen tissue sample is fixed with PAXgene.

In some embodiments, the biological sample, e.g., the tissue sample is fixed, for example in methanol, acetone, acetone-methanol, PFA, PAXgene or is formalin-fixed and paraffin-embedded (FFPE). In some embodiments, the biological sample comprises intact cells. In some embodiments, the biological sample is a cell pellet, e.g., a fixed cell pellet, e.g., an FFPE cell pellet. FFPE samples are used in some instances in the RNA-templated ligation (RTL) methods disclosed herein. A limitation of direct RNA capture for fixed samples is that the RNA integrity of fixed (e.g., FFPE) samples can be lower than a fresh sample. As such, capturing RNA directly from fixed samples, e.g., by capture of a common sequence, such as a poly(A) tail of an mRNA molecule, can be more difficult. By utilizing RTL probes that hybridize to RNA target sequences in the transcriptome, RNA analytes can be captured without requiring that both a poly(A) tail and target sequences remain intact. Accordingly, RTL probes can be utilized to beneficially improve capture and spatial analysis of fixed samples. The biological sample, e.g., tissue sample, can be stained, and imaged prior, during, and/or after each step of the methods described herein. Any of the methods described herein or known in the art can be used to stain and/or image the biological sample. In some embodiments, the imaging occurs prior to destaining the sample. In some embodiments, the biological sample is stained using an H&E staining method. In some embodiments, the tissue sample is stained and imaged for about 10 minutes to about 2 hours (or any of the subranges of this range described herein). Additional time may be needed for staining and imaging of different types of biological samples.

The tissue sample can be obtained from any suitable location in a tissue or organ of a subject, e.g., a human subject. In some instances, the sample is a mouse sample. In some instances, the sample is a human sample. In some embodiments, the sample can be derived from skin, brain, breast, lung, liver, kidney, prostate, tonsil, thymus, testes, bone, lymph node, ovary, eye, heart, or spleen. In some instances, the sample is a human or mouse breast tissue sample. In some instances, the sample is a human or mouse brain tissue sample. In some instances, the sample is a human or mouse lung tissue sample. In some instances, the sample is a human or mouse tonsil tissue sample. In some instances, the sample is a human or mouse liver tissue sample. In some instances, the sample is a human or mouse bone, skin, kidney, thymus, testes, or prostate tissue sample. In some embodiments, the tissue sample is derived from normal or diseased tissue. In some embodiments, the sample is an embryo sample. The embryo sample can be a non-human embryo sample. In some instances, the sample is a mouse embryo sample.

Non-limiting examples of stains include histological stains (e.g., hematoxylin and/or eosin) and immunological stains (e.g., fluorescent stains). The biological sample can be stained using Can-Grunwald, Giemsa, hematoxylin and eosin (H&E), Jenner's, Leishman, Masson's trichrome, Papanicolaou, Romanowsky, silver, Sudan, Wright's, and/or Periodic Acid Schiff (PAS) staining techniques. In some instances, PAS staining is performed after formalin or acetone fixation. In some embodiments, a biological sample (e.g., a fixed and/or stained biological sample) can be imaged. Biological samples are also described in Section (I)(d) of PCT Publication No. WO2020/176788 and/or U.S. Patent Application Publication No. 2020/0277663.

The following embodiments can be used with any of the methods described herein. In some embodiments, the biological sample is imaged. In some embodiments, the biological sample is visualized or imaged using bright field microscopy. In some embodiments, the biological sample is visualized or imaged using fluorescence microscopy. The biological sample can be visualized or imaged using additional methods of visualization and imaging are known in the art. Non-limiting examples of visualization and imaging include expansion microscopy, bright field microscopy, dark field microscopy, phase contrast microscopy, electron microscopy, fluorescence microscopy, reflection microscopy, interference microscopy and confocal microscopy. In some embodiments, the sample is stained and imaged prior to adding reagents for analyzing captured analytes as disclosed herein to the biological sample.

In some embodiments, the method includes staining the biological sample. In some embodiments, the staining includes the use of hematoxylin and/or eosin. In some embodiments, a biological sample can be stained using any number of biological stains including, but not limited to, acridine orange, Bismarck brown, carmine, coomassie blue, cresyl violet, DAPI (4',6-diamidino-2-phenylindole), eosin, ethidium bromide, acid fuchsine, hematoxylin, Hoechst stains, iodine, methyl green, methylene blue, neutral red, Nile blue, Nile red, osmium tetroxide, propidium iodide, rhodamine, or safranin. In some instances, the biological sample can be stained using known staining techniques, including Can-Grunwald, Giemsa, hematoxylin and eosin (H&E), Jenner's, Leishman, Masson's trichrome, Papanicolaou, Romanowsky, silver, Sudan, Wright's, and/or Periodic Acid Schiff (PAS) staining techniques. PAS staining is typically performed after formalin or acetone fixation.

In some embodiments, the staining includes the use of a detectable label, such as a radioisotope, a fluorophore, a chemiluminescent compound, a bioluminescent compound, or a combination thereof.

In some embodiments, a biological sample is permeabilized with one or more permeabilization reagents. For example, permeabilization of a biological sample can facilitate analyte capture. Exemplary permeabilization agents and conditions are described in Section (I)(d)(ii)(13) or the Exemplary Embodiments Section of PCT Publication No. WO2020/176788 and/or U.S. Patent Application Publication No. 2020/0277663. Briefly, in any of the methods described herein, the method includes a step of permeabilizing the biological sample. For example, the biological sample can be permeabilized to facilitate transfer of the extension products to the capture probes on the array. In some embodiments, the permeabilizing includes the use of an organic solvent (e.g., acetone, ethanol, or methanol), a detergent (e.g., saponin, Triton X-100^{™}, Tween-20^{™}, or sodium dodecyl sulfate (SDS)), an enzyme (e.g., an endopeptidase, an exopeptidase, or a protease), or a combination thereof. In some embodiments, the permeabilizing includes the use of an endopeptidase, a protease, SDS, polyethylene glycol tert-octylphenyl ether, polysorbate 80, and polysorbate 20, N-lauroylsarcosine sodium salt solution, saponin, Triton X-100^{™}, Tween-20^{™}, or a combination thereof. In some embodiments, the endopeptidase is pepsin. In some embodiments, the endopeptidase is Proteinase K. Additional methods for sample permeabilization are described, for example, in Jamur et al., Method Mol. Biol. 588:63-66, 2010.

Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of the analytes within the biological sample. The spatial location of an analyte within the biological sample is determined based on the feature to which the analyte is bound (e.g., directly or indirectly) on the array, and the feature's relative spatial location within the array.

A "capture probe" refers to any molecule capable of capturing (directly or indirectly) and/or labelling an analyte (e.g., an analyte of interest) in a biological sample. In some embodiments, the capture probe is a nucleic acid or a polypeptide. In some embodiments, the capture probe includes a barcode (e.g., a spatial barcode and/or a unique molecular identifier (UMI) and a capture domain). In some instances, the capture probe includes a homopolymer sequence, such as a poly(T) sequence. In some embodiments, a capture probe can include a cleavage domain and/or a functional domain (e.g., a primer-binding site, such as for next-generation sequencing (NGS)). See, e.g., Section (II)(b) (e.g., subsections (i)-(vi)) of PCT Publication No. WO2020/176788 and/or U.S. Patent Application Publication No. 2020/0277663. Generation of capture probes can be achieved by any appropriate method, including those described in Section (II)(d)(ii) of PCT Publication No. WO2020/176788 and/or U.S. Patent Application Publication No. 2020/0277663.

In some instances, a capture probe and a nucleic acid analyte interaction (or any other nucleic acid to nucleic acid interaction) occurs because the sequences of the two nucleic acids are substantially complementary to one another. By "substantial," "substantially" and the like, two nucleic acid sequences can be complementary when at least 60% of the nucleotide residues of one nucleic acid sequence are complementary to nucleotide residues of the other nucleic acid sequence. The complementary residues within a particular complementary nucleic acid sequence need not always be contiguous with each other, but can be interrupted by one or more non-complementary residues within the complementary nucleic acid sequence. In some embodiments, at least 60%, but less than 100%, of the residues of one of the two complementary nucleic acid sequences are complementary to residues of the other nucleic acid sequence. In some embodiments, at least 70%, 80%, 90%, 95%, or 99% of the residues of one nucleic acid sequence are complementary to residues of the other nucleic acid sequence. Sequences are said to be "substantially complementary" when at least 60% (e.g., at least 70%, at least 80%, or at least 90%) of the residues of one nucleic acid sequence are complementary to residues in the other nucleic acid sequence.

In some embodiments, the biological sample is mounted on a first substrate and the substrate comprising the array of capture probes is a second substrate. In this configuration, one or more analytes or analyte derivatives (e.g., intermediate agents, e.g., ligation products) can then be released from the biological sample and migrate to the second substrate comprising an array of capture probes. In some embodiments, the release and migration of the analytes or analyte derivatives to the second substrate comprising the array of capture probes occurs in a manner that preserves the original spatial context of the analytes in the biological sample. This method can be referred to as a sandwiching process, which is described e.g., in U.S. Patent Application Publication No. 2021/0189475 and PCT Publication Nos. WO 2021/252747 A1, WO 2022/061152 A2, and WO 2022/140028 A1.

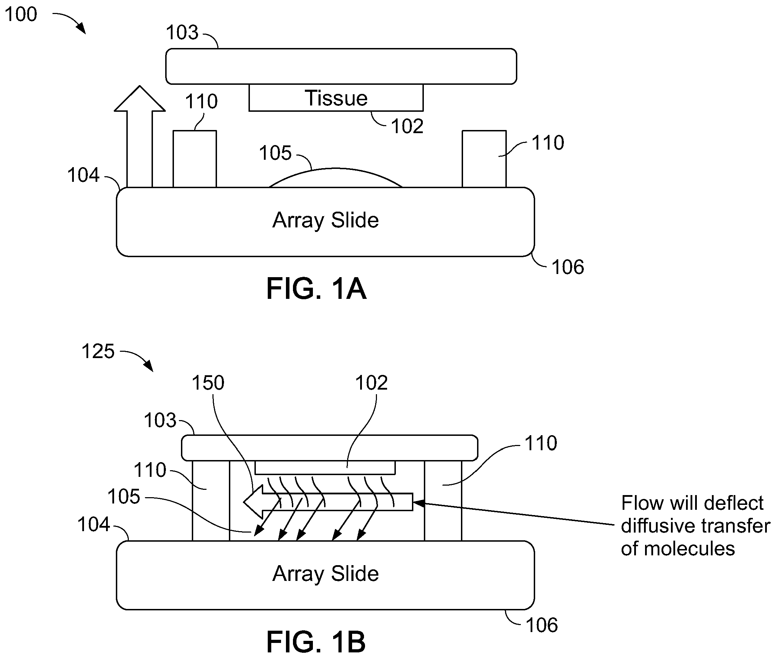

**FIG. 1A** shows an exemplary sandwiching process **100** where a first substrate (e.g., slide **103**), including a biological sample **102,** and a second substrate (e.g., array slide **104** including an array having spatially barcoded capture probes **106**) are brought into proximity with one another. As shown in **FIG. 1A****,** a liquid reagent drop (e.g., permeabilization solution **105**) is introduced on the second substrate in proximity to the capture probes **106** and in between the biological sample **102** and the second substrate (e.g., slide **104** including an array having spatially barcoded capture probes **106**). The permeabilization solution **105** may release analytes or analyte derivatives (e.g., intermediate agents, e.g., ligation products) that can be captured by the capture probes of the array **106**.

During the exemplary sandwiching process, the first substrate is aligned with the second substrate, such that at least a portion of the biological sample is aligned with at least a portion of the capture probes (e.g., aligned in a sandwich configuration). As shown, the second substrate (e.g., array slide **104**) is in an inferior position to the first substrate (e.g., slide **103**). In some embodiments, the first substrate (e.g., slide **103**) may be positioned superior to the second substrate (e.g., slide **104**). A reagent medium **105** within a gap between the first substrate (e.g., slide **103**) and the second substrate (e.g., slide **104**) creates a liquid interface between the two substrates. The reagent medium may be a permeabilization solution which permeabilizes and/or digests the biological sample **102**. In some embodiments, wherein the biological sample **102** has been pre-permeabilized, the reagent medium is not a permeabilization solution. In some embodiments, analytes (e.g., mRNA transcripts) and/or analyte derivatives (e.g., intermediate agents, e.g., ligation products) of the biological sample **102** may release from the biological sample, and actively or passively migrate (e.g., diffuse) across the gap toward the capture probes on the array **106**. Alternatively, in certain embodiments, migration of the analyte or analyte derivative (e.g., intermediate agent; e.g., ligation product) from the biological sample is performed actively (e.g., electrophoretic, by applying an electric field to promote migration). Exemplary methods of electrophoretic migration are described in WO 2020/176788, and U.S. Patent Application Publication No. 2021/0189475.

As further shown, one or more spacers **110** may be positioned between the first substrate (e.g., slide **103**) and the second substrate (e.g., array slide **104** including spatially barcoded capture probes **106**). The one or more spacers **110** may be configured to maintain a separation distance between the first substrate and the second substrate. While the one or more spacers **110** is shown as disposed on the second substrate, the spacer may additionally or alternatively be disposed on the first substrate.

In some embodiments, the one or more spacers 110 is configured to maintain a separation distance between first and second substrates that is between about 2 microns and 1 mm (e.g., between about 2 microns and 800 microns, between about 2 microns and 700 microns, between about 2 microns and 600 microns, between about 2 microns and 500 microns, between about 2 microns and 400 microns, between about 2 microns and 300 microns, between about 2 microns and 200 microns, between about 2 microns and 100 microns, between about 2 microns and 25 microns, or between about 2 microns and 10 microns), measured in a direction orthogonal to the surface of first substrate that supports the biological sample. In some instances, the separation distance is about 2, 3, 4, 5, 6, 7, 8, 9, 10, 11, 12, 13, 14, 15, 16, 17, 18, 19, 20, 21, 22, 23, 24, or 25 microns. In some embodiments, the separation distance is less than 50 microns. In some embodiments, the separation distance is less than 25 microns. In some embodiments, the separation distance is less than 20 microns. The separation distance may include a distance of at least 2 µm.

**FIG. 1B** shows a fully formed sandwich configuration **125** creating a chamber **150** formed from the one or more spacers **110,** the first substrate (e.g., the slide **103),** and the second substrate (e.g., the slide **104** including an array **106** having spatially barcoded capture probes) in accordance with some example implementations. In the example of **FIG. 1B****,** the liquid reagent (e.g., the permeabilization solution **105)** fills the volume of the chamber **150** and may create a permeabilization buffer that allows analytes (e.g., mRNA transcripts and/or other molecules) or analyte derivatives (e.g., intermediate agents, e.g., ligation products) to diffuse from the biological sample **102** toward the capture probes of the second substrate (e.g., slide **104).** In some aspects, flow of the permeabilization buffer may deflect transcripts and/or molecules from the biological sample **102** and may affect diffusive transfer of analytes or analyte derivatives (e.g., intermediate agents, e.g., ligation products) for spatial analysis. A partially or fully sealed chamber **150** resulting from the one or more spacers **110**, the first substrate (e.g., slide **103**)**,** and the second substrate (e.g., slide **104**) may reduce or prevent flow from undesirable movement (e.g., convective movement) of transcripts and/or molecules during the diffusive transfer from the biological sample **102** to the capture probes.

The sandwiching process methods described above can be implemented using a variety of hardware components. For example, the sandwiching process methods can be implemented using a sample holder (also referred to herein as a support device, a sample handling apparatus, and an array alignment device). Further details on support devices, sample holders, sample handling apparatuses, or systems for implementing a sandwiching process are described in, e.g., US. Patent Application Publication No. 2021/0189475, and PCT Publication No. WO 2022/061152 A2.

In some embodiments of a sample holder, the sample holder can include a first member including a first retaining mechanism configured to retain a first substrate comprising a biological sample. The first retaining mechanism can be configured to retain the first substrate disposed in a first plane. The sample holder can further include a second member including a second retaining mechanism configured to retain a second substrate disposed in a second plane. The sample holder can further include an alignment mechanism connected to one or both of the first member and the second member. The alignment mechanism can be configured to align the first and second members along the first plane and/or the second plane such that the sample contacts at least a portion of the reagent medium when the first and second members are aligned and within a threshold distance along an axis orthogonal to the second plane. The adjustment mechanism may be configured to move the second member along the axis orthogonal to the second plane and/or move the first member along an axis orthogonal to the first plane.

In some embodiments, the adjustment mechanism includes a linear actuator. In some embodiments, the linear actuator is configured to move the second member along an axis orthogonal to the plane of the first member and/or the second member. In some embodiments, the linear actuator is configured to move the first member along an axis orthogonal to the plane of the first member and/or the second member. In some embodiments, the linear actuator is configured to move the first member, the second member, or both the first member and the second member at a velocity of at least 0.1 mm/sec. In some embodiments, the linear actuator is configured to move the first member, the second member, or both the first member and the second member with an amount of force of at least 0.1 lbs.

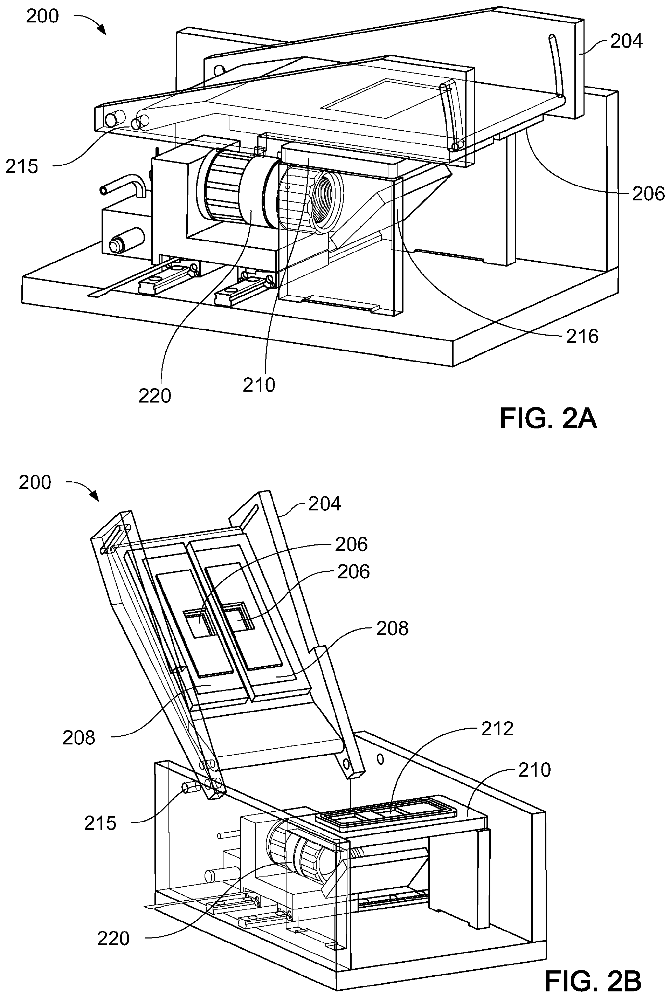

**FIG. 2A** is a perspective view of an example sample handling apparatus **200** in a closed position in accordance with some example implementations. As shown, the sample handling apparatus **200** includes a first member **204,** a second member **210,** optionally an image capture device **220,** a first substrate **206,** optionally a hinge **215,** and optionally a mirror **216.** The hinge **215** may be configured to allow the first member **204** to be positioned in an open or closed configuration by opening and/or closing the first member **204** in a clamshell manner along the hinge **215.**

**FIG. 2B** is a perspective view of the example sample handling apparatus **200** in an open position in accordance with some example implementations. As shown, the sample handling apparatus **200** includes one or more first retaining mechanisms **208** configured to retain one or more first substrates **206.** In the example of **FIG. 2B****,** the first member **204** is configured to retain two first substrates **206,** however the first member **204** may be configured to retain more or fewer first substrates **206.**

In some aspects, when the sample handling apparatus **200** is in an open position (e.g., in **FIG. 2B****),** the first substrate **206** and/or the second substrate **212** may be loaded and positioned within the sample handling apparatus **200,** such as within the first member **204** and the second member **210,** respectively. As noted, the hinge **215** may allow the first member **204** to close over the second member **210** and form a sandwich configuration.

In some aspects, after the first member **204** closes over the second member **210,** an adjustment mechanism of the sample handling apparatus **200** may actuate the first member **204** and/or the second member **210** to form the sandwich configuration for the permeabilization of the sample (e.g., bringing the first substrate **206** and the second substrate **212** closer to each other and within a threshold distance for the sandwich configuration). The adjustment mechanism may be configured to control a speed, an angle, a force, or the like of the sandwich configuration.

In some embodiments, the biological sample (e.g., sample **102** from **FIG. 1A****)** may be aligned within the first member **204** (e.g., via the first retaining mechanism **208)** prior to closing the first member **204** such that a desired region of interest of the sample is aligned with the barcoded array of the second substrate (e.g., the slide **104** from **FIG. 1A****),** e.g., when the first and second substrates are aligned in the sandwich configuration. Such alignment may be accomplished manually (e.g., by a user) or automatically (e.g., via an automated alignment mechanism). After or before alignment, spacers may be applied to the first substrate **206** and/or the second substrate **212** to maintain a minimum spacing between the first substrate **206** and the second substrate **212** during sandwiching. In some aspects, the permeabilization solution (e.g., permeabilization solution **305)** may be applied to the first substrate **206** and/or the second substrate **212.** The first member **204** may then close over the second member **210** and form the sandwich configuration. Analytes or analyte derivatives (e.g., intermediate agents, e.g., ligation products) may be captured by the capture probes of the array and may be processed for spatial analysis.

In some embodiments, during permeabilization, the image capture device **220** may capture images of the overlap area between the biological sample and the capture probes on the array **106.** If more than one first substrates **206** and/or second substrates **212** are present within the sample handling apparatus **200,** the image capture device **220** may be configured to capture one or more images of one or more overlap areas.

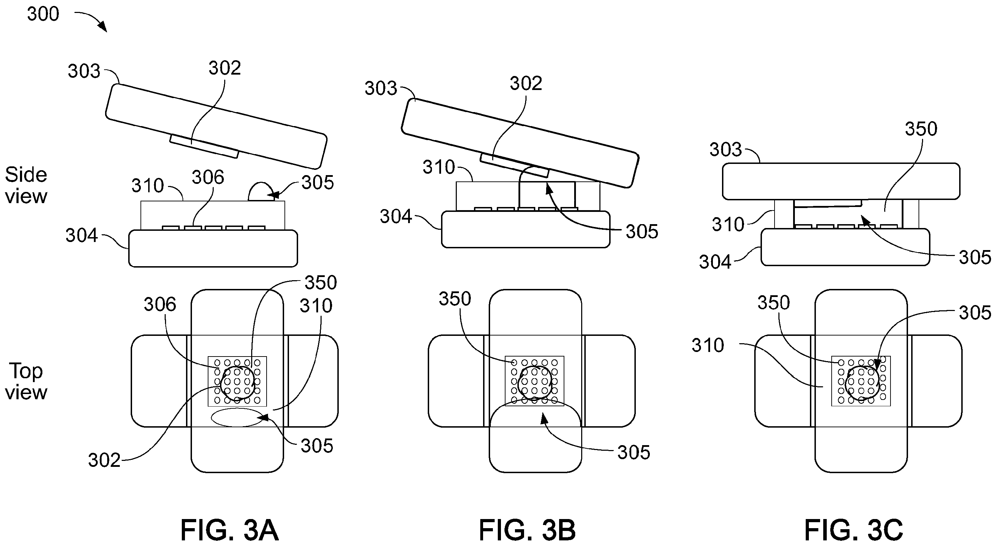

Described herein are methods for delivering a fluid to a biological sample disposed on an area of a first substrate and an array disposed on a second substrate. **FIGs. 3A-3C** depict a side view and a top view of an exemplary angled closure workflow **300** for sandwiching a first substrate (e.g., slide **303**) having a biological sample **302** and a second substrate (e.g., slide **304** having capture probes **306**) in accordance with some exemplary implementations.

**FIG. 3A** depicts the first substrate (e.g., slide **303** including a biological sample **302**) angled over (superior to) the second substrate (e.g., slide **304**). As shown, reagent medium (e.g., permeabilization solution) **305** is located on the spacer **310** toward the right-hand side of the side view in **FIG. 3A****.** While **FIG. 3A** depicts the reagent medium on the right-hand side of side view, it should be understood that such depiction is not meant to be limiting as to the location of the reagent medium on the spacer.

**FIG. 3B** shows that as the first substrate lowers, and/or as the second substrate rises, the dropped side of the first substrate (e.g., a side of the slide **303** angled toward the slide **304**) may contact the reagent medium **305**. The dropped side of the slide **303** may urge the reagent medium **305** toward the opposite direction (e.g., towards an opposite side of the spacer **310**, towards an opposite side of the slide **303** relative to the dropped side). For example, in the side view of **FIG. 3B** the reagent medium **305** may be urged from right to left as the sandwich is formed.

In some embodiments, the first substrate and/or the second substrate are further moved to achieve an approximately parallel arrangement of the first substrate and the second substrate.

**FIG. 3C** depicts a full closure of the sandwich between the first substrate and the second substrate with the spacer **310** contacting both the first substrate and the second substrate and maintaining a separation distance and optionally the approximately parallel arrangement between the two substrates. As shown in the top view of **FIG. 3C****,** the spacer **310** fully encloses and surrounds the biological sample **302** and the capture probes **306,** and the spacer **310** form the sides of chamber **350** which holds a volume of the reagent medium **305.**

While **FIG. 3C** depicts the first substrate (e.g., the slide **303** including biological sample **302**) angled over (superior to) the second substrate (e.g., slide **304**) and the second substrate comprising the spacer **310,** it should be understood that an exemplary angled closure workflow can include the second substrate angled over (superior to) the first substrate and the first substrate comprising the spacer **310.**

It may be desirable that the reagent medium be free from air bubbles between the substrates to facilitate transfer of target analytes with spatial information. Additionally, air bubbles present between the substrates may obscure at least a portion of an image capture of a desired region of interest. Accordingly, it may be desirable to ensure or encourage suppression and/or elimination of air bubbles between the two substrates (e.g., slide **303** and slide **304**) during a permeabilization step (e.g., step **104**). In some aspects, bubble formation between the substrates may be reduced or eliminated using a variety of filling methods and/or closing methods. In some instances, the first substrate and the second substrate are arranged in an angled sandwich assembly as described herein. For example, during the sandwiching of the two substrates (e.g., the slide **303** and the slide **304**), an angled closure workflow may be used to suppress or eliminate bubble formation.

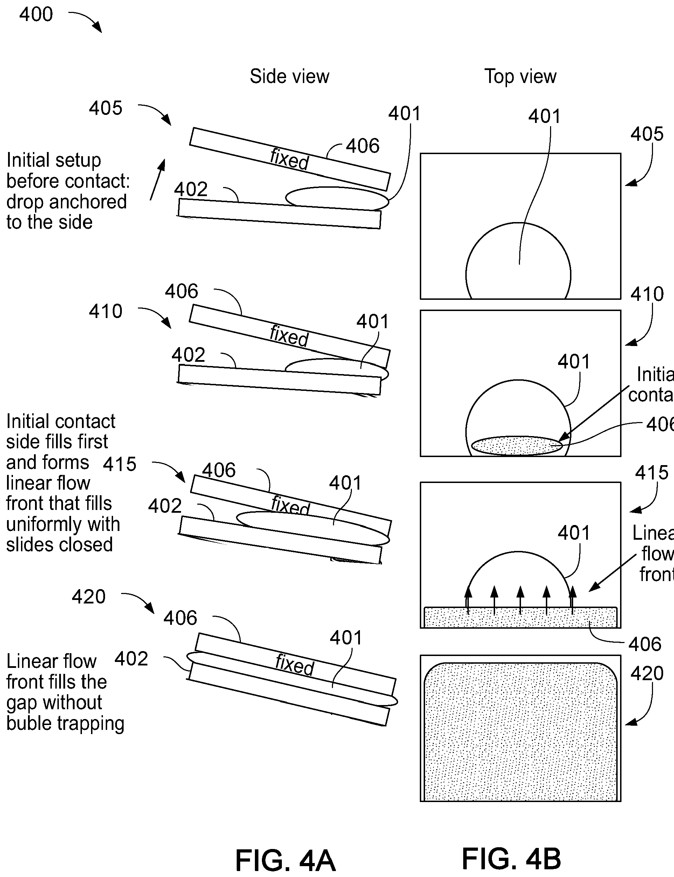

**FIG. 4A** is a side view of the angled closure workflow **400** in accordance with some exemplary implementations. **FIG. 4B** is a top view of the angled closure workflow **400** in accordance with some exemplary implementations. As shown at step **405,** reagent medium **401** is positioned to the side of the substrate **402.**

At step **410,** the dropped side of the angled substrate **406** contacts the reagent medium **401** first. The contact of the substrate **406** with the reagent medium **401** may form a linear or low curvature flow front that fills the gap between the two substrates **406** and **402** uniformly with the slides closed.

At step **415,** the substrate **406** is further lowered toward the substrate **402** (or the substrate **402** is raised up toward the substrate **406**) and the dropped side of the substrate **406** may contact and may urge the reagent medium toward the side opposite the dropped side, thereby creating a linear or low curvature flow front that may prevent or reduce bubble trapping between the substrates.

At step **420,** the reagent medium **401** fills the gap between the substrate **406** and the substrate **402.** The linear flow front of the liquid reagent may be formed by squeezing the reagent medium **401** volume along the contact side of the substrate **402** and/or the substrate **406.** Additionally, capillary flow may also contribute to filling the gap area.

In some embodiments, the reagent medium (e.g., **105** in **FIG 1A**) comprises a permeabilization agent. In some embodiments, following initial contact between the biological sample and a permeabilization agent, the permeabilization agent can be removed from contact with the biological sample (e.g., by opening the sample holder). Suitable agents for this purpose include, but are not limited to, organic solvents (e.g., acetone, ethanol, or methanol), cross-linking agents (e.g., paraformaldehyde), detergents (e.g., saponin, Triton X-100^{™}, Tween-20^{™}, or sodium dodecyl sulfate (SDS)), and enzymes (e.g., trypsin or other proteases (e.g., proteinase K). In some embodiments, the detergent is an anionic detergent (e.g., SDS or N-lauroylsarcosine sodium salt solution).

In some embodiments, the reagent medium comprises a lysis reagent. Lysis solutions can include ionic surfactants such as, for example, sarkosyl and sodium dodecyl sulfate (SDS). More generally, chemical lysis agents can include, without limitation, organic solvents, chelating agents, detergents, surfactants, and chaotropic agents. In some embodiments, the reagent medium comprises a protease. Exemplary proteases include, e.g., pepsin, trypsin, elastase, and proteinase K. In some embodiments, the reagent medium comprises a nuclease. In some embodiments, the nuclease comprises an RNase. In some embodiments, the RNase includes RNase A, RNase C, RNase H, or RNase I. In some embodiments, the reagent medium comprises sodium dodecyl sulfate (SDS) or a sodium salt thereof, proteinase K, pepsin, N-lauroylsarcosine, or RNase.

In some embodiments, the reagent medium comprises polyethylene glycol (PEG). In some embodiments, the PEG molecular weight is from about 2K to about 16K. In some embodiments, the PEG molecular weight is about 2K, about 3K, about 4K, about 5K, about 6K, about 7K, about 8K, about 9K, about 10K, about 11K, about 12K, about 13K, about 14K, about 15K, or about 16K. In some embodiments, the PEG is present at a concentration from about 2% to about 25%, from about 4% to about 23%, from about 6% to about 21%, or from about 8% to about 20% (v/v).

In certain embodiments, a dried permeabilization reagent is applied or formed as a layer on the first substrate, the second substrate, or both prior to contacting the biological sample with the array. For example, a permeabilization reagent can be deposited in solution on the first substrate or the second substrate or both and then dried.

In some instances, the aligned portions of the biological sample and the array are in contact with the reagent medium for about 1 minute, about 5 minutes, about 10 minutes, about 12 minutes, about 15 minutes, about 18 minutes, about 20 minutes, about 25 minutes, about 30 minutes, about 36 minutes, about 45 minutes, or about an hour. In some instances, the aligned portions of the biological sample and the array are in contact with the reagent medium for about 1-60 minutes.

In some instances, the device is configured to control a temperature of the first and second substrates. In some embodiments, the temperature of the first and second members is lowered to a first temperature that is below room temperature.

There are at least two methods to associate a spatial barcode with one or more neighboring cells, such that the spatial barcode identifies the one or more cells, and/or contents of the one or more cells, as associated with a particular spatial location in a biological sample. One method is to promote analytes or analyte proxies (e.g., intermediate agents) out of a cell and towards a spatially-barcoded array (e.g., including spatially-barcoded capture probes). Another method is to release or cleave spatially-barcoded capture probes from an array and promote the spatially-barcoded capture probes towards and/or into or onto the biological sample.

In some cases, capture probes may be configured to prime, replicate, and consequently yield optionally barcoded extension products from a template (e.g., a DNA or RNA template, such as an analyte or an intermediate agent (e.g., a ligation product or an analyte capture agent), or a portion thereof), or derivatives thereof (see, e.g., Section (II)(b)(vii) of PCT Publication No. WO2020/176788 and/or U.S. Patent Application Publication No. 2020/0277663 regarding extended capture probes). In some cases, capture probes may be configured to form ligation products with a template (e.g., a DNA or RNA template, such as an analyte or an intermediate agent, or portion thereof), thereby creating ligation products that serve as proxies for the template.

As used herein, an "extended capture probe" refers to a capture probe having additional nucleotides added to a terminus (e.g., a 3' or 5' end) of the capture probe thereby extending the overall length of the capture probe. For example, an "extended 3' end" indicates additional nucleotides were added to the most 3' nucleotide of the capture probe to extend the length of the capture probe, for example, by polymerization reactions used to extend nucleic acid molecules including templated polymerization catalyzed by a polymerase (e.g., a DNA polymerase or a reverse transcriptase). In some embodiments, extending the capture probe includes adding to a 3' end of a capture probe a nucleic acid sequence that is complementary to a nucleic acid sequence of an analyte or intermediate agent specifically bound to the capture domain of the capture probe. In some embodiments, the capture probe is extended by using a reverse transcriptase. In some embodiments, the capture probe is extended using one or more DNA polymerases. In some embodiments, the extended capture probes include the sequence of the capture domain, the sequence of the spatial barcode of the capture probe, and the complementary sequence of the template used for extension of the capture probe.

In some embodiments, extended capture probes are amplified (e.g., in bulk solution or on the array) to yield quantities that are sufficient for downstream analysis, e.g., sequencing. In some embodiments, extended capture probes (e.g., DNA molecules) can act as templates for an amplification reaction (e.g., a polymerase chain reaction).

Additional variants of spatial analysis methods, including in some embodiments, an imaging step, are described in Section (II)(a) of PCT Publication No. WO2020/176788 and/or U.S. Patent Application Publication No. 2020/0277663. Analysis of captured analytes (and/or intermediate agents or portions thereof), for example, including sample removal, extension of capture probes using the captured analyte as a template, sequencing (e.g., of a cleaved extended capture probe and/or a cDNA molecule complementary to an extended capture probe), sequencing on the array (e.g., using, for example, in situ hybridization or in situ ligation approaches), temporal analysis, and/or proximity capture, is described in Section (II)(g) of PCT Publication No. WO2020/176788 and/or U.S. Patent Application Publication No. 2020/0277663. Some quality control measures are described in Section (II)(h) of PCT Publication No. WO2020/176788 and/or U.S. Patent Application Publication No. 2020/0277663.

Spatial information can provide information of medical importance. For example, the methods described herein can allow for: identification of one or more biomarkers (e.g., diagnostic, prognostic, and/or for determination of efficacy of a treatment) of a disease or disorder; identification of a candidate drug target for treatment of a disease or disorder; identification (e.g., diagnosis) of a subject as having a disease or disorder; identification of stage and/or prognosis of a disease or disorder in a subject; identification of a subject as having an increased likelihood of developing a disease or disorder; monitoring of progression of a disease or disorder in a subject; determination of efficacy of a treatment of a disease or disorder in a subject; identification of a patient subpopulation for which a treatment is effective for a disease or disorder; modification of a treatment of a subject with a disease or disorder; selection of a subject for participation in a clinical trial; and/or selection of a treatment for a subject with a disease or disorder. Exemplary methods for identifying spatial information of biological and/or medical importance can be found in U.S. Patent Application Publication Nos. 2021/0140982, 2021/0198741, and 2021/0199660.

Spatial information can provide information of biological importance. For example, the methods described herein can allow for: identification of transcriptome and/or proteome expression profiles (e.g., in healthy and/or diseased tissue); identification of multiple analyte types in close proximity (e.g., nearest neighbor or proximity based analysis); determination of up-regulated and/or down-regulated genes and/or proteins in diseased tissue; characterization of tumor microenvironments; characterization of tumor immune responses; characterization of cells types and their co-localization in healthy and diseased tissue; and identification of genetic variants within tissues (e.g., based on gene and/or protein expression profiles associated with specific disease or disorder biomarkers).

For spatial array-based methods, a substrate may function as a support for direct or indirect attachment of capture probes to features of the array. A "feature" is an entity that acts as a support or repository for various molecular entities used in spatial analysis. In some embodiments, some or all of the features in an array are functionalized for analyte capture. Exemplary substrates are described in Section (II)(c) of PCT Publication No. WO2020/176788 and/or U.S. Patent Application Publication No. 2020/0277663. Exemplary features and geometric attributes of an array can be found in Sections (II)(d)(i), (II)(d)(iii), and (II)(d)(iv) of PCT Publication No. WO2020/176788 and/or U.S. Patent Application Publication No. 2020/0277663.

Generally, analytes and/or intermediate agents (or portions thereof) can be captured when contacting a biological sample with a substrate including capture probes (e.g., a substrate with capture probes embedded, spotted, printed, fabricated on the substrate, or a substrate with features (e.g., beads, wells) comprising capture probes). As used herein, "contact," "contacted," and/or "contacting," a biological sample with a substrate refers to any contact (e.g., direct or indirect) such that capture probes can interact (e.g., bind covalently or non-covalently (e.g., hybridize)) with analytes from the biological sample. Capture can be achieved actively (e.g., using electrophoresis) or passively (e.g., using diffusion). Analyte capture is further described in Section (II)(e) of PCT Publication No. WO2020/176788 and/or U.S. Patent Application Publication No. 2020/0277663.

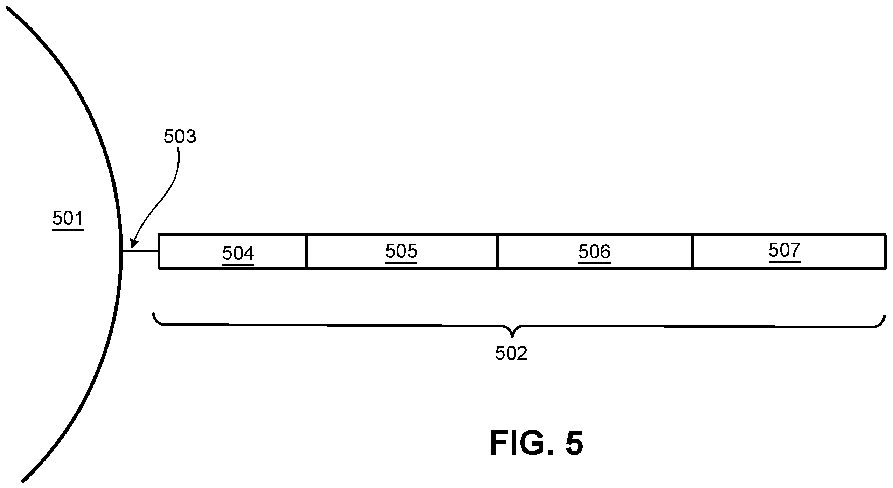

**FIG. 5** is a schematic diagram showing an exemplary capture probe, as described herein. As shown, the capture probe **502** is optionally coupled to a feature **501** by a cleavage domain **503,** such as a disulfide linker. The capture probe can include a functional sequence **504** that is useful for subsequent processing. The functional sequence **504** can include all or a part of sequencer specific flow cell attachment sequence (e.g., a P5 or P7 sequence), all or a part of a sequencing primer sequence, (e.g., a R1 primer binding site, a R2 primer binding site), or a combination thereof. The capture probe can also include a spatial barcode **505.** The capture probe can also include a unique molecular identifier (UMI) sequence **506.** While **FIG. 5** shows the spatial barcode **505** as being located upstream (5') of UMI sequence **506,** it is to be understood that capture probes wherein UMI sequence **506** is located upstream (5') of the spatial barcode **505** is also suitable for use in any of the methods described herein. The capture probe can also include a capture domain **507** to facilitate capture of a target analyte. The capture domain can have a sequence complementary to a sequence of a nucleic acid analyte. The capture domain can have a sequence complementary to a connected probe described herein. The capture domain can have a sequence complementary to an analyte capture sequence present in an analyte capture agent. The capture domain can have a sequence complementary to a splint oligonucleotide. A splint oligonucleotide, in addition to having a sequence complementary to a capture domain of a capture probe, can have a sequence complementary to a sequence of a nucleic acid analyte, a portion of a connected probe described herein, a capture handle sequence described herein, and/or a methylated adaptor described herein.

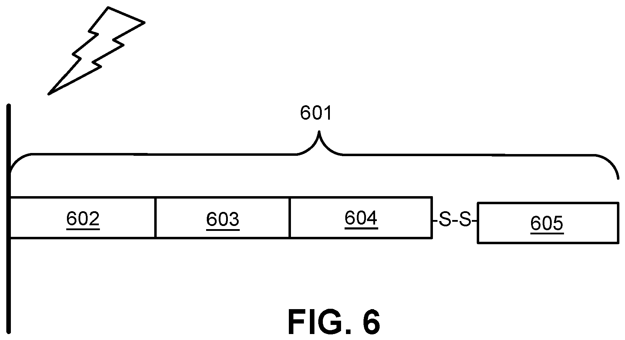

**FIG. 6** is a schematic illustrating a cleavable capture probe, wherein the cleaved capture probe can enter into a non-permeabilized cell and bind to analytes within the cell. The capture probe **601** can contain a cleavage domain **602,** a cell penetrating peptide **603,** a reporter molecule **604,** and a disulfide bond (-S-S-). **605** represents all other parts of a capture probe, for example a spatial barcode and a capture domain.

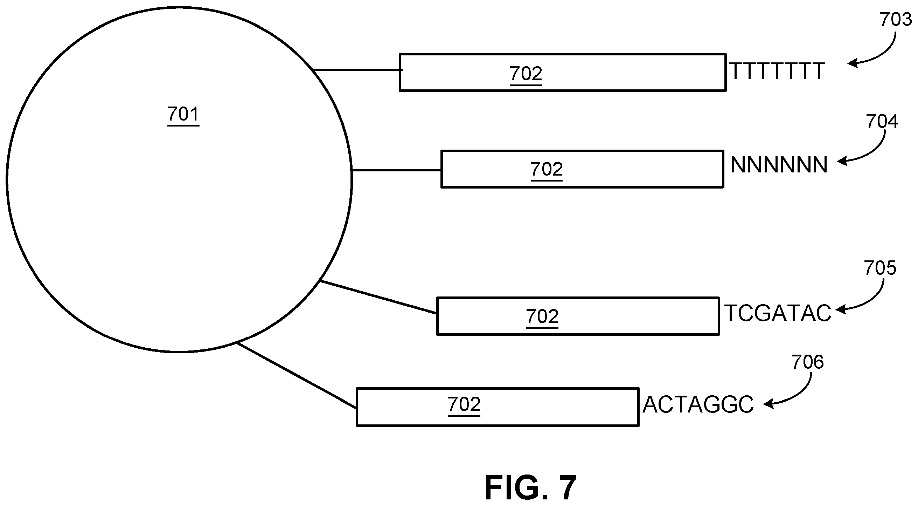

**FIG. 7** is a schematic diagram of an exemplary multiplexed spatially-barcoded feature. In **FIG.** 7, the feature **701** can be coupled to spatially-barcoded capture probes, wherein the spatially-barcoded probes of a particular feature can possess the same spatial barcode, but have different capture domains designed to associate the spatial barcode of the feature with more than one target analyte. For example, a feature may include four different types of spatially-barcoded capture probes, each type of spatially-barcoded capture probe possessing the spatial barcode **702.** One type of capture probe associated with the feature can include the spatial barcode **702** in combination with a poly(T) capture domain **703,** designed to capture mRNA target analytes. A second type of capture probe associated with the feature can include the spatial barcode **702** in combination with a random N-mer capture domain **704** for gDNA analysis. A third type of capture probe associated with the feature can include the spatial barcode **702** in combination with a capture domain complementary to the analyte capture agent of interest **705.** A fourth type of capture probe associated with the feature can include the spatial barcode **702** in combination with a capture probe that can specifically bind a nucleic acid molecule **706** that can function in a CRISPR assay (e.g., CRISPR/Cas9). While only four different capture probe-barcoded constructs are shown in **FIG. 7****,** capture-probe barcoded constructs can be tailored for analyses of any given analyte associated with a nucleic acid and capable of binding with such a construct. For example, the schemes shown in **FIG. 7** can also be used for concurrent analysis of other analytes disclosed herein, including, but not limited to: (a) mRNA, a lineage tracing construct, cell surface or intracellular proteins and/or metabolites, and gDNA; (b) mRNA, accessible chromatin (e.g., ATAC-seq, DNase-seq, and/or MNase-seq), cell surface or intracellular proteins and metabolites, and a perturbation agent (e.g., a CRISPR crRNA/sgRNA, TALEN, zinc finger nuclease, and/or antisense oligonucleotide as described herein); (c) mRNA, cell surface or intracellular proteins and/or metabolites, a barcoded labelling agent (e.g., the MHC multimers described herein), and a V(D)J sequence of an immune cell receptor (e.g., T-cell receptor). In some embodiments, a perturbation agent can be a small molecule, an antibody, a drug, an aptamer, a miRNA, a physical environmental (e.g., temperature) change, or any other known perturbation agents.

The functional sequences can generally be selected for compatibility with any of a variety of different sequencing systems, e.g., Ion Torrent Proton or PGM, Illumina sequencing instruments, PacBio, Oxford Nanopore, etc., and the requirements thereof. In some embodiments, functional sequences can be selected for compatibility with non-commercialized sequencing systems. Examples of such sequencing systems and techniques, for which suitable functional sequences can be used include, but are not limited to, Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the spatial barcode **505** and functional sequences **504** are common to all of the probes attached to a given feature. In some embodiments, the UMI sequence **506** of a capture probe attached to a given feature is different from the UMI sequence of a different capture probe attached to the given feature.

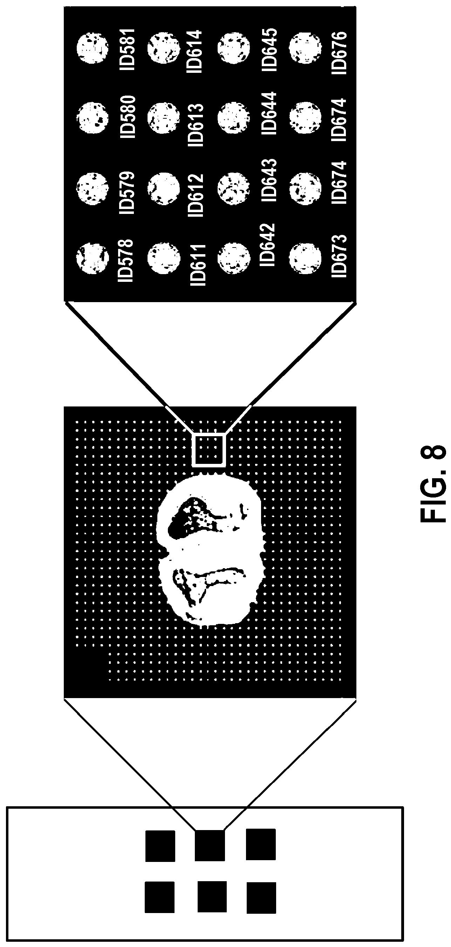

**FIG. 8** depicts an exemplary arrangement of barcoded features within an array. From left to right, **FIG. 8** shows (left) a slide including six spatially-barcoded arrays, (center) an enlarged schematic of one of the six spatially-barcoded arrays, showing a grid of barcoded features in relation to a biological sample, and (right) an enlarged schematic of one section of an array, showing the specific identification of multiple features within the array (e.g., labelled as ID578, ID579, ID580, etc.).

In some embodiments, more than one analyte type (e.g., nucleic acids and proteins) from a biological sample can be detected (e.g., simultaneously or sequentially) using any appropriate multiplexing technique, such as those described in Section (IV) of PCT Publication No. WO2020/176788 and/or U.S. Patent Application Publication No. 2020/0277663.

In some cases, spatial analysis can be performed by attaching and/or introducing a molecule (e.g., a peptide, a lipid, or a nucleic acid molecule) having a barcode (e.g., a spatial barcode) to a biological sample (e.g., to a cell in a biological sample). In some embodiments, a plurality of molecules (e.g., a plurality of nucleic acid molecules) having a plurality of barcodes (e.g., a plurality of spatial barcodes) are introduced to a biological sample (e.g., to a plurality of cells in a biological sample) for use in spatial analysis. In some embodiments, after attaching and/or introducing a molecule having a barcode to a biological sample, the biological sample can be physically separated (e.g., dissociated) into single cells or cell groups for analysis. Some such methods of spatial analysis are described in Section (III) of PCT Publication No. WO2020/176788 and/or U.S. Patent Application Publication No. 2020/0277663.

In some cases, spatial analysis can be performed by detecting multiple oligonucleotides that hybridize to an analyte. In some instances, for example, spatial analysis can be performed using RNA-templated ligation (RTL). Methods of RTL have been described previously. See, e.g., Credle et al., Nucleic Acids Res. 2017 Aug 21; 45(14):e128. Typically, RTL includes hybridization of two oligonucleotides to adjacent sequences on an analyte (e.g., an RNA molecule, such as an mRNA molecule). In some instances, the oligonucleotides are DNA molecules. In some instances, one of the oligonucleotides includes at least two ribonucleic acid bases at the 3' end and/or the other oligonucleotide includes a phosphorylated nucleotide at the 5' end. In some instances, one of the two oligonucleotides includes a capture domain (e.g., a poly(A) sequence or a non-homopolymeric sequence). After hybridization to the analyte, a ligase (e.g., a T4 RNA ligase (Rnl2), a PBCV-1 DNA Ligase or *Chlorella* virus DNA Ligase, a single-stranded DNA ligase, or a T4 DNA ligase) ligates the two oligonucleotides together, creating a ligation product. In some instances, the two oligonucleotides hybridize to sequences that are not adjacent to one another. For example, hybridization of the two oligonucleotides creates a gap between the hybridized oligonucleotides. In some instances, a polymerase (e.g., a DNA polymerase) can extend one of the oligonucleotides prior to ligation. After ligation, the ligation product is released from the analyte. In some instances, the ligation product is released using an endonuclease (e.g., RNase H). In some instances, the ligation product is removed using heat. In some instances, the ligation product is removed using potassium hydroxide (KOH). The released ligation product can then be captured by capture probes (e.g., instead of direct capture of an analyte) on an array, optionally amplified, and sequenced, thus determining the location and optionally the abundance of the analyte in the biological sample.

In some instances, one or both of the oligonucleotides may hybridize to genomic DNA (gDNA) which can lead to false positive sequencing data from ligation events on gDNA (off target) in addition to the desired (on target) ligation events on target nucleic acids (e.g., mRNA). Thus, in some embodiments, the disclosed methods can include contacting the biological sample with a deoxyribonuclease (DNase). The DNase can be an endonuclease or exonuclease. In some embodiments, the DNase digests single-stranded and/or double-stranded DNA. Suitable DNases include, without limitation, a DNase I and a DNase II. Use of a DNase as described can mitigate false positive sequencing data from off target gDNA ligation events.

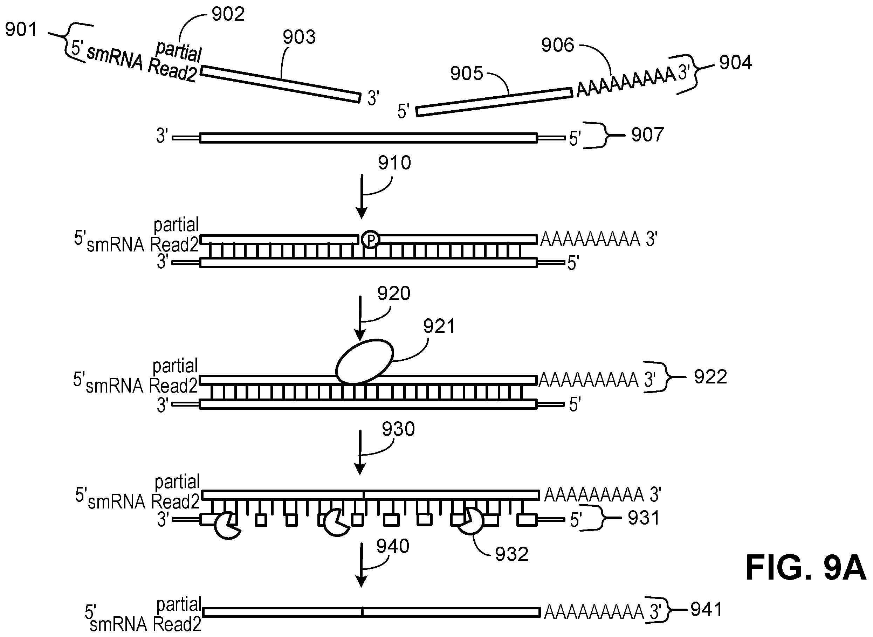

A non-limiting example of templated ligation methods disclosed herein is depicted in **FIG. 9A****.** After a biological sample is contacted with a substrate including a plurality of capture probes and contacted with (a) a first probe **901** having a target-hybridization sequence **903** and a primer sequence **902** and (b) a second probe **904** having a target-hybridization sequence **905** and a capture domain (e.g., a poly(A) sequence) **906,** the first probe **901** and the second probe **904** hybridize **910** to an analyte **907.** A ligase **921** ligates **920** the first probe **901** to the second probe **904,** thereby generating a ligation product **922.** The ligation product **922** is then released **930** from the analyte **931** by digesting the analyte **907** using an endoribonuclease **932.** The sample is permeabilized **940** and the ligation product **941** is able to hybridize to a capture probe on the substrate. Methods and composition for spatial detection using templated ligation have been described in PCT Publication No. WO 2021/133849 A1, U.S. Patent Nos. 11,332,790 and 11,505,828.

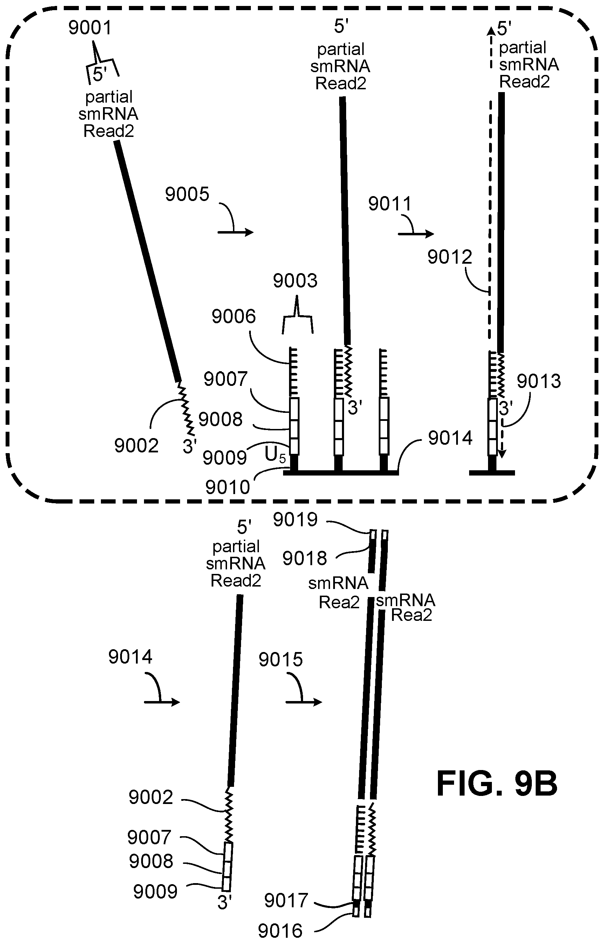

In some embodiments, as shown in **FIG. 9B****,** the ligation product **9001** includes a capture probe capture domain **9002,** which can bind to a capture probe **9003** (e.g., a capture probe immobilized, directly or indirectly, on a substrate **9004**). In some embodiments, methods described herein include contacting **9005** a biological sample with a substrate **9004,** wherein the capture probe **9003** is affixed to the substrate (e.g., immobilized to the substrate, directly or indirectly). In some embodiments, the capture probe capture domain **9002** of the ligated product **9001** specifically binds to the capture domain **9006.** The capture probe can also include a unique molecular identifier (UMI) **9007,** a spatial barcode **9008,** a functional sequence **9009,** and a cleavage domain **9010.**

In some embodiments, methods described herein include permeabilization of the biological sample such that the capture probe can more easily bind to target analytes (i.e., compared to no permeabilization). In some embodiments, reverse transcription (RT) reagents can be added to permeabilize biological samples. Incubation with the RT reagents can be used to extend the capture probes **9011** to produce spatially-barcoded full-length cDNA **9012** and **9013** from the captured analytes (e.g., polyadenylated mRNA). Second strand reagents (e.g., second strand primers, enzymes, etc.) can be added to the biological sample to initiate second strand synthesis.

In some embodiments, methods described herein include permeabilization of the biological sample such that the capture probe can more easily capture the ligation products (i.e., compared to no permeabilization). In some embodiments, reverse transcription (RT) reagents can be added to permeabilize biological samples. Incubation with the RT reagents can be used to extend the capture probes **9011** to produce spatially-barcoded full-length cDNA **9012** and **9013** from the captured ligation products (e.g., polyadenylated ligation products).

In some embodiments, the extended ligation products can be denatured **9014,** released from the capture probe and transferred (e.g., to a clean tube) for amplification, and/or library construction. The spatially-barcoded ligation products can be amplified **9015** via PCR prior to library construction. P5 **9016** and P7 **9019** sequences can be used for sequencing, while i5 **9017** and i7 **9018** sequences can be used as sample indexes. The amplicons can then be sequenced using paired-end sequencing using TruSeq Read 1 and TruSeq Read 2 as sequencing primer sites.

In some embodiments, detection of one or more analytes (e.g., protein analytes) can be performed using one or more analyte capture agents. As used herein, an "analyte capture agent" refers to an agent that interacts with an analyte (e.g., an analyte in a biological sample) and with a capture probe (e.g., a capture probe attached to a substrate or a feature) to identify the analyte. In some embodiments, the analyte capture agent includes: (i) an analyte binding moiety (e.g., that binds to an analyte), for example, an antibody or antigen-binding fragment thereof; (ii) analyte binding moiety barcode; and (iii) an analyte capture sequence. As used herein, the term "analyte binding moiety barcode" refers to a barcode that is associated with or otherwise identifies the analyte binding moiety. As used herein, the term "analyte capture sequence" refers to a region or moiety configured to hybridize to, bind to, couple to, or otherwise interact with a capture domain of a capture probe. In some cases, an analyte binding moiety barcode (or portion thereof) may be able to be removed (e.g., cleaved) from the analyte capture agent. Additional description of analyte capture agents can be found in Section (II)(b)(ix) of PCT Publication No. WO2020/176788 and/or Section (II)(b)(viii) U.S. Patent Application Publication No. 2020/0277663.

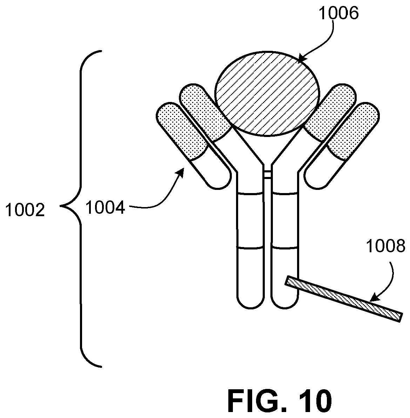

**FIG. 10** is a schematic diagram of an exemplary analyte capture agent **1002** comprised of an analyte binding moiety **1004** and an analyte binding moiety barcode domain **1008.** The exemplary analyte binding moiety **1004** is a molecule capable of binding to an analyte **1006** and the analyte capture agent **1002** is capable of interacting with a spatially-barcoded capture probe. The analyte binding moiety **1004** can bind to the analyte **1006** with high affinity and/or with high specificity. The analyte capture agent **1002** can include: (i) an analyte binding moiety barcode domain **1008** which serves to identify the analyte binding moiety, and (ii) an analyte capture sequence, which can hybridize to at least a portion or an entirety of a capture domain of a capture probe. The analyte binding moiety **1004** can include a polypeptide and/or an aptamer. The analyte binding moiety **1004** can include an antibody or antibody fragment (e.g., an antigen-binding fragment).

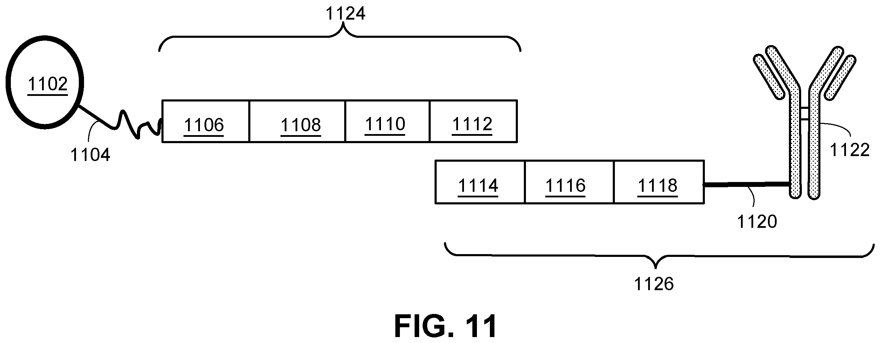

**FIG. 11** is a schematic diagram depicting an exemplary interaction between a feature-immobilized capture probe **1124** and an analyte capture agent **1126.** The feature-immobilized capture probe **1124** can include a spatial barcode **1108** as well as functional sequences **1106** and a UMI **1110,** as described elsewhere herein. The capture probe can be affixed **1104** to a feature such as a bead **1102.** The capture probe **1124** can also include a capture domain **1112** that is capable of binding to an analyte capture agent **1126.** The analyte binding moiety barcode domain of the analyte capture agent **1126** can include a functional sequence **1118,** analyte binding moiety barcode **1116,** and an analyte capture sequence **1114** that is capable of binding (e.g., hybridizing) to the capture domain **1112** of the capture probe **1124.** The analyte capture agent **1126** can also include a linker **1120** that allows the analyte binding moiety barcode domain (e.g., including the functional sequence **1118,** analyte binding moiety barcode **1116,** and analyte capture sequence **1114**) to couple to the analyte binding moiety **1122.** In some embodiments, the linker **1120** is a cleavable linker. In some embodiments, the cleavable linker is a photo-cleavable linker, a UV-cleavable linker, chemical-cleavable linker, thermal-cleavable linker, or an enzyme cleavable linker. In some instances, the cleavable linker is a disulfide linker. A disulfide linker can be cleaved by use of a reducing agent, such as dithiothreitol (DTT), beta-mercaptoethanol (BME), or tris(2-carboxyethyl)phosphine (TCEP).

During analysis of spatial information, sequence information for a spatial barcode associated with an analyte is obtained, and the sequence information can be used to provide information about the spatial distribution of the analyte in the biological sample. Various methods can be used to obtain the spatial information. In some embodiments, specific capture probes and the analytes they capture are associated with specific locations in an array of features on a substrate. For example, specific spatial barcodes can be associated with specific array locations prior to array fabrication, and the sequences of the spatial barcodes can be stored (e.g., in a database) along with specific array location information, so that each spatial barcode uniquely maps to a particular array location.

Alternatively, specific spatial barcodes can be deposited at predetermined locations in an array of features during fabrication such that at each location, only one type of spatial barcode is present so that each spatial barcode is uniquely associated with a single feature of the array. Where necessary, the arrays can be decoded using any of the methods described herein so that spatial barcodes are uniquely associated with array feature locations, and this mapping can be stored as described above.

When sequence information is obtained for capture probes and/or analytes during analysis of spatial information, the locations of the capture probes and/or analytes can be determined by referring to the stored information that uniquely associates each spatial barcode with an array feature location. In this manner, specific capture probes and captured analytes are associated with specific locations in the array of features. Each array feature location represents a position relative to a coordinate reference point (e.g., an array location, a fiducial marker) for the array. Accordingly, each feature location has an "address" or location in the coordinate space of the array.

Some exemplary spatial analysis workflows are described in the Exemplary Embodiments section of PCT Publication No. WO2020/176788 and/or U.S. Patent Application Publication No. 2020/0277663. See, for example, the Exemplary embodiment starting with "In some non-limiting examples of the workflows described herein, the sample can be immersed..." of PCT Publication No. WO2020/176788 and/or U.S. Patent Application Publication No. 2020/0277663. See also, e.g., the Visium Spatial Gene Expression Reagent Kits User Guide (e.g., Rev F, dated January 2022); and/or the Visium Spatial Gene Expression Reagent Kits - Tissue Optimization User Guide (e.g., Rev E, dated February 2022).

In some embodiments, spatial analysis can be performed using dedicated hardware and/or software, such as any of the systems described in Sections (II)(e)(ii) and/or (V) of PCT Publication No. WO2020/176788 and/or U.S. Patent Application Publication No. 2020/0277663, or any of one or more of the devices or methods described in Sections *Control Slide for Imaging, Methods of Using Control Slides and Substrates for, Systems of Using Control Slides and Substrates for Imaging,* and/or *Sample and Array Alignment Devices and Methods, Informational labels* of PCT Publication No. WO2020/123320.

Suitable systems for performing spatial analysis can include components such as a chamber (e.g., a flow cell or a sealable, fluid-tight chamber) for containing a biological sample. The biological sample can be mounted, for example, in a biological sample holder. One or more fluid chambers can be connected to the chamber and/or the sample holder via fluid conduits, and fluids can be delivered into the chamber and/or sample holder via fluidic pumps, vacuum sources, or other devices coupled to the fluid conduits that create a pressure gradient to drive fluid flow. One or more valves can also be connected to fluid conduits to regulate the flow of reagents from reservoirs to the chamber and/or sample holder.

The systems can optionally include a control unit that includes one or more electronic processors, an input interface, an output interface (such as a display), and a storage unit (e.g., a solid state storage medium such as, but not limited to, a magnetic, optical, or other solid state, persistent, writeable, and/or re-writeable storage medium). The control unit can optionally be connected to one or more remote devices via a network. The control unit (and components thereof) can generally perform any of the steps and functions described herein. Where the system is connected to a remote device, the remote device (or devices) can perform any of the steps or features described herein. The systems can optionally include one or more detectors (e.g., CCD or CMOS) used to capture images. The systems can also optionally include one or more light sources (e.g., LED-based, diode-based, or lasers) for illuminating a sample, a substrate with features, analytes from a biological sample captured on a substrate, and various control and calibration media.

The systems can optionally include software instructions encoded and/or implemented in one or more of tangible storage media and hardware components such as application specific integrated circuits. The software instructions, when executed by a control unit (and in particular, an electronic processor) or an integrated circuit, can cause the control unit, integrated circuit, or other component executing the software instructions to perform any of the method steps or functions described herein.

In some cases, the systems described herein can detect (e.g., register an image) the biological sample on the array. Exemplary methods to detect the biological sample on an array are described in PCT Publication No. WO2021/102003 and/or U.S. Patent Application Publication No. 2021/0150707.

Prior to transferring analytes from the biological sample to the array of features on the substrate, the biological sample can be aligned with the array. Alignment of a biological sample and an array of features including capture probes can facilitate spatial analysis, which can be used to detect differences in analyte presence and/or level within different positions in the biological sample, for example, to generate a three-dimensional map of the analyte presence and/or level. Exemplary methods to generate a two-dimensional and/or three-dimensional map of the analyte presence and/or level are described in PCT Publication No. WO2020/053655 and spatial analysis methods are generally described in PCT Publication No. WO2021/102039 and/or U.S. Patent Application Publication No. 2021/0155982.

In some cases, a map of analyte presence and/or level can be aligned to an image of a biological sample using one or more fiducial markers, e.g., objects placed in the field of view of an imaging system which appear in the image produced, as described in the *Substrate Attributes* Section, *Control Slide for Imaging* Section of PCT Publication Nos. WO2020/123320, WO 2021/102005, and/or U.S. Patent Application Publication No. 2021/0158522. Fiducial markers can be used as a point of reference or measurement scale for alignment (e.g., to align a sample and an array, to align two substrates, to determine a location of a sample or array on a substrate relative to a fiducial marker) and/or for quantitative measurements of sizes and/or distances.

### Biological Samples

The methods disclosed herein can be performed on any type of biological sample. In some embodiments, the biological sample is a fresh tissue sample. In some embodiments, the biological sample is a frozen tissue sample. In some embodiments, the biological sample was previously frozen. In some embodiments, the biological sample is a fixed tissue sample. In some embodiments, the fixed tissue sample is a formalin-fixed, paraffin embedded (FFPE) sample.

In some embodiments, the biological sample is a tissue section. In some embodiments, the sample is a fresh tissue section. In some embodiments, the biological sample is a frozen tissue section. In some embodiments, the biological sample was previously frozen. In some embodiments, the biological sample is a fixed tissue section. In some embodiments, the fixed tissue section is a formalin-fixed, paraffin embedded (FFPE) section.

Subjects from which biological samples can be obtained can be healthy or asymptomatic individuals, individuals that have or are suspected of having a disease (e.g., cancer) or a pre-disposition to a disease, and/or individuals that are in need of therapy or suspected of needing therapy. In some instances, the biological sample can include one or more diseased cells. A diseased cell can have altered metabolic properties, gene expression, protein expression, and/or morphologic features. Examples of diseases include inflammatory disorders, metabolic disorders, nervous system disorders, cancer, and/or diseases associated with a genetic variant (e.g., any of the genetic variants described herein). In some instances, the biological sample comprises nucleic acids with one or more genetic variants. In some instances, the one or more genetic variants is associated with a disease or disease state. In some instances, the biological sample includes cancer or tumor cells. Cancer cells can be derived from solid tumors, hematological malignancies, cell lines, or obtained as circulating tumor cells. In some instances, the biological sample is a heterogeneous sample. In some instances, the biological sample is a heterogeneous sample that includes tumor or cancer cells and/or stromal cells.

FFPE samples can be heavily cross-linked and fragmented, and therefore, this type of sample allows for limited RNA recovery using conventional detection techniques. In certain embodiments, methods of targeted RNA capture described herein are less affected by RNA degradation associated with FFPE fixation than other methods (e.g., methods that involve oligo-dT capture and reverse transcription of mRNA). In certain embodiments, methods described herein enable sensitive measurement of specific genes of interest that otherwise might be missed using a poly(A) capture based whole transcriptomic approach.

In some embodiments, the FFPE sample or section is deparaffinized, permeabilized, equilibrated, and blocked before target probe oligonucleotides are added. In some embodiments, deparaffinization includes using xylenes. In some embodiments, deparaffinization includes multiple washes with xylenes. In some embodiments, deparaffinization includes multiple washes with xylenes followed by removal of xylenes using multiple rounds of graded alcohol followed by washing the sample with water. In some aspects, the water is deionized water.

### Arrays

The present disclosure also includes methods, compositions, and kits including a spatial array. For example, a spatial array is an array including a plurality of capture probes, where a capture probe of the plurality of capture probes includes: (i) a spatial barcode and (ii) a capture domain. In some embodiments, the capture probe includes one or more functional domains, a cleavage domain, a unique molecular identifier (UMI), or a combination thereof. In some embodiments, the one or more functional domains includes a primer binding site or a sequencing specific site.

In some embodiments, the second probe includes a capture probe capture domain. In some embodiments, the capture probe capture domain is substantially complementary to the capture domain of the capture probe. In some embodiments, the capture probe capture domain includes a poly(A) sequence. In some embodiments, the capture domain of the capture probe includes a poly(T) sequence. In some embodiments, the capture domain of the capture probe and the capture probe capture domain include fixed sequences. The capture probe capture domain and the capture domain of the capture probe can be any sequence so long as the two domains are substantially complementary for hybridization to occur.

In some embodiments, the biological sample is disposed on the array. In some embodiments, the biological sample is disposed on a substrate. In some embodiments, the method includes aligning the substrate including the biological sample with the array (e.g., sandwiching as described herein), such that at least a portion of the biological sample is aligned with at least a portion of the array.

In some embodiments, the method includes migrating the target nucleic acid from the biological sample to the array. Alternatively, in some embodiments, the method includes migrating capture probes from the array to the biological sample. In some embodiments, migrating includes electrophoresis.

In some embodiments, the method includes determining (i) all or a portion of the sequence of the ligation product, or a complement thereof, and (ii) the sequence of the spatial barcode, or a complement thereof, and using the determined sequences of (i) and (ii) to determine a location of the genetic variant in the biological sample. In some embodiments, the determining step includes sequencing. In some embodiments, sequencing is high-throughput sequencing. In some embodiments, the determining step includes fluorescent detection (e.g., fluorescently labeled probes).

### Library Preparation

In some embodiments, the target nucleic acids or complements thereof and other proxies of target nucleic acids (e.g., ligation products), and/or amplicons of such products, can be prepared for downstream applications, such as generation of a sequencing library and next-generation sequencing. For example, the target nucleic acids, proxies of target nucleic acids and/or complements thereof can be purified and collected for downstream amplification. The amplification products can be amplified using PCR, where primer binding sites flank the spatial barcode and target nucleic acid, or a complement thereof, generating a library associated with a particular spatial barcode. In some embodiments, the library preparation can be quantitated and/or quality controlled to verify the success of the library preparation steps. The library amplicons can be sequenced and analyzed to decode spatial information of the target nucleic acid or proxy thereof (e.g., a ligation product).

Alternatively or additionally, the amplicons can then be enzymatically fragmented and/or size-selected to enrich for a desired amplicon size. In some embodiments, when utilizing an Illumina^{®} library preparation methodology, for example, P5 and P7 sequences can be added to the amplicons, thereby allowing capture of the library preparation on a sequencing flow cell (e.g., on Illumina sequencing instruments). Additionally, i7 and i5 index sequences can be added as sample indexes if multiple libraries are to be pooled and sequenced together. Further, Read 1 and Read 2 sequences can be added to the library preparation for sequencing purposes. The aforementioned sequences can be added to a library preparation sample, for example, via End Repair, A-tailing, Adaptor Ligation, and/or PCR. The cDNA fragments can then be sequenced using, for example, paired-end sequencing using TruSeq Read 1 and TruSeq Read 2 as sequencing primer sites.

### B. Spatial Detection of Genetic Variants in Target Nucleic Acids

This disclosure features methods of templated ligation (e.g., RNA-templated ligation (RTL)) to increase detection and determine location of genetic variants within a target nucleic acid in a biological sample. Templated ligation can increase target-specific genetic variant detection in a target nucleic acid through hybridization of multiple (e.g., two) and/or differentially length oligonucleotides, or probes, that are subsequently ligated to form one ligation product that can be captured by a capture probe on a spatial array. More specifically, this disclosure features templated ligation methods that increase the efficiency of detecting genetic variants, such as single-nucleotide polymorphisms, insertions, and/or deletions ("indels"). Generally, probe pairs that hybridize to adjacent or nearly adjacent sequences on a target nucleic acid can be ligated to one another. In the case where the probes are non-adjacent, a gap-filling reaction can be performed prior to ligation. However, some limitations of this method can include non-specific binding of one of the probes near a genetic variant.

### Scenario for Using Reversible Terminators to Detect Genetic Variants

The present disclosure features methods where a first probe hybridizes to a target nucleic acid and is then extended using a reversible terminator nucleotide. This single nucleotide extension step can provide increased specificity over standard templated ligation methods. Various reversible terminator nucleotides can be used in the methods described herein, including 3' blocked and unblocked reversible terminator nucleotides. In some examples, the probe can be extended with a second, a third, a fourth, a fifth, or more reversible terminator nucleotides.

In some examples, the target nucleic acids detected by the methods described herein include one or more genetic variants. Thus described herein are methods for determining presence or absence of a genetic variant in a biological sample, the method including: (a) contacting the biological sample with a plurality of first probes, where a first probe of the plurality of first probes includes a sequence substantially complementary to a sequence of a target nucleic acid in the biological sample; (b) hybridizing the first probe to the target nucleic acid and extending the first probe using a reversible terminator nucleotide, thereby generating an extended first probe; (c) contacting the biological sample with a plurality of second probes, where a second probe of the plurality of second probes includes a sequence substantially complementary to a sequence of the target nucleic acid; (d) hybridizing the second probe to the target nucleic acid and ligating the extended first probe to the second probe, thereby generating a ligation product; and (e) determining the presence or absence of the ligation product, thereby determining the presence or absence of the genetic variant in the biological sample.

Non-limiting examples of target nucleic acids include nucleic acids such as DNA or RNA. Non-limiting examples of DNA analytes include genomic DNA, methylated DNA, specific methylated DNA sequences, fragmented DNA, mitochondrial DNA, *in situ* synthesized PCR products, and viral DNA.

Non-limiting examples of RNA analytes include various types of coding and noncoding RNA. Examples of the different types of RNA analytes include messenger RNA (mRNA), ribosomal RNA (rRNA), transfer RNA (tRNA), microRNA (miRNA), and viral RNA. The RNA can be a transcript (e.g., present in a tissue section). The RNA can be small (e.g., less than 200 nucleic acid bases in length) or large (e.g., RNA greater than 200 nucleic acid bases in length). Small RNAs mainly include 5.8S ribosomal RNA (rRNA), 5S rRNA, transfer RNA (tRNA), microRNA (miRNA), small interfering RNA (siRNA), small nucleolar RNA (snoRNAs), PIWI-interacting RNA (piRNA), tRNA-derived small RNA (tsRNA), and small rDNA-derived RNA (srRNA). The RNA can be double-stranded RNA or single-stranded RNA. The RNA can be circular RNA. The RNA can be a bacterial rRNA (e.g., 16S rRNA or 23 S rRNA). The RNA can be from an RNA virus, for example, RNA viruses from Group III, IV, or V of the Baltimore classification system. The RNA can be from a retrovirus, such as a virus from Group VI of the Baltimore classification system.

In some embodiments, the first probe is extended with a polymerase. In some embodiments, the polymerase is Bst 3 polymerase. In some embodiments, the polymerase is Klenow polymerase. In some embodiments, the first probe is extended with a reverse transcriptase. Any suitable reverse transcriptase can be used. In some embodiments, the reverse transcriptase is KOD reverse transcriptase *(Thermococcus kodakarensis* reverse transcriptase).

In some embodiments, the first probe is a DNA probe. In some embodiments, the second probe is a DNA probe. In some embodiments, the second plurality of probes is contacted with the biological sample at about the same time (e.g., concurrently or simultaneously) as the first plurality of probes. In some embodiments, the second plurality of probes is contacted with the biological sample after the first plurality of probes. For example, the first plurality of probes can be contacted with the biological sample and allowed to hybridize to the target nucleic acids. In some embodiments, the second plurality of probes can be contacted with the biological sample before the first probes are extended (e.g., extended with a reversible terminator nucleotide). In some embodiments, the second plurality of probes can be contacted with the biological sample after the first probe has been extended (e.g., extended with a reversible terminator nucleotide).

In some embodiments, the first probe is extended with a reversible terminator nucleotide. Suitable reversible terminator nucleotides are described in WO 2004/018493; WO 2003/020261; WO 2005/084367; WO 2007/053719; WO 1999/049082; WO 2008/005673; WO 1996/007669; U.S. Patent No. 8,030,466; and U.S. Patent No. 9,650,406. In some embodiments, the first probe is extended with a reversible terminator nucleotide that includes a 3' blocking group, thereby generating an extended first probe. The blocking group can reduce the likelihood of further extension after incorporation into the first probe. In some embodiments, the 3' blocking group is a 3'-O-blocked reversible terminator nucleotide. Non-limiting examples of 3'-O-blocked reversible terminator nucleotides include 3'-azidomethyl dNTP, a 3'-O-allyl dNTP, a 3'-O-nitrobenzyl dNTP, a 3'-O-(2-nitrobenzyl) dNTP, and 3'-O-dithiomethyl dNTP. In some embodiments, the blocking group is removed. For example, after removing the blocking group, the extended first probe can be extended by one, two, three, four, five, or more reversible terminator nucleotides. In some embodiments, the blocking group is removed using a reducing agent. In some embodiments, the reducing agent is tris(2-carboxyethyl)phosphine (TCEP). In some embodiments, the reducing agent is dithiothreitol (DTT). In some embodiments, removing the blocking group includes cleaving the blocking group.

In some embodiments, the reversible terminator nucleotide is an unblocked reversible terminator nucleotide. 3'-O-blocked reversible terminators include a blocking group linked to the oxygen atom of the 3' OH of the pentose moiety (e.g., deoxyribose) of the nucleotide. In contrast, unblocked reversible terminator nucleotides include a reversible termination group linked to the base of the nucleotide. Non-limiting examples of unblocked reversible terminator nucleotides include 5-hydroxymethyl-2'-deoxyuridine triphosphate (HOMedUTP) *(See* e.g., Litosh, V.A., et al., Improved nucleotide sensitivity and termination of 3'-OH unblocked reversible terminators by molecular tuning of 2-nitrobenzyl alkylated HOMedU triphosphates; Nucleic Acids Research, 339 (6) (2011)) and those described in WO 2013/040257.

In some embodiments, the 3' end of the extended probe (e.g., extended first probe) can be ligated to the 5' end of the second probe, thereby generating a proxy of the target nucleic acid (e.g., a ligation product). In some embodiments, the extended probe and the second probe can be ligated with a ligase. In some embodiments, the ligase can be any ligase capable of ligating RNA and DNA together. In some embodiments, the ligase can be any DNA ligase. In some embodiments, the ligase can be T4 RNA ligase 2. Other enzymes suitable for the ligation step include, e.g., Tth DNA ligase, Taq DNA ligase, Thermococcus sp. (strain 9°N) DNA ligase (9°N^{™} DNA ligase, New England Biolabs), AmpligaseTM (available from Lucigen, Middleton, WI), SplintR (available from New England Biolabs, Ipswich, MA) (also known as PBCV-1 DNA Ligase), or *Chlorella* virus DNA Ligase.

In some embodiments, prior to contacting the biological sample with a spatial array and after ligation of the extended probe and the second probe, the biological sample can be treated with an RNase. In some embodiments, the biological sample is contacted with an RNase while disposed on the spatial array. In some embodiments, the RNase includes RNase A, RNase I, RNase H, or RNase P. In some embodiments, the RNase is RNase H.

In some embodiments, the unligated probes (e.g., unligated first probes and/or extended first probes and unligated second probes) can be removed (e.g., washed) from the array. In some embodiments, the unligated probes (e.g., unligated first probes and unligated second probes) can be blocked from binding to the capture probes of the array. For example, the unligated second probe including the capture probe capture domain (e.g., the sequence complementary to the capture domain) can bind (e.g., hybridize) to the capture domain (e.g., any of the capture domains described herein). In some embodiments, when the unligated second probe binds to the capture domain, the unligated second probe can block ligated probes (analytes of interest) from binding (e.g., hybridizing) to the capture domain. To reduce the likelihood of undesirable binding of unligated probes to the capture domain, the capture sequence of the second probe can be blocked until the portion complementary to the target nucleic acid is hybridized to the target nucleic acid, and optionally, ligated to the first probe. Then, the blocking probe can be removed to allow binding of the ligation product to the capture probes of the array.

In some embodiments, after generating a ligation product, the ligation product is removed from the target nucleic acid. In some embodiments, a ligation product is removed from the target nucleic acid using an endoribonuclease. In some embodiments, the endoribonuclease is RNase H, RNase A, RNase C, or RNase I. In some embodiments, the endoribonuclease is RNase H. RNase H is an endoribonuclease that specifically hydrolyzes the phosphodiester bonds of RNA when hybridized to DNA. RNase H is part of a conserved family of ribonucleases that are present in many different organisms. There are two primary classes of RNase H: RNase H1 and RNase H2. Retroviral RNase H enzymes are similar to the prokaryotic RNase H1 enzymes. Each of these enzymes shares the characteristic ability to cleave the RNA component of an RNA:DNA heteroduplex. In some embodiments, the RNase H is RNase H1 or RNase H2. In some embodiments, the RNase H includes, but is not limited to, RNase HII from *Pyrococcus furiosus,* RNase HII from *Pyrococcus horikoshi,* RNase HI from *Thermococcus litoralis,* RNase HI from *Thermus thermophilus,* RNAse HI from *E. coli,* or RNase HII from *E. coli.*

In some embodiments, the target nucleic acid is removed from the ligation product via denaturation. In some embodiments, denaturation includes the use of heat. In some embodiments, the denaturation includes the use of potassium hydroxide. In some embodiments, denaturation includes the use of both heat and potassium hydroxide. In some embodiments, the method includes washing the biological sample after (e) to remove first probes and unhybridized second probes.

In some embodiments, the array includes one or more features. In some embodiments, features are directly or indirectly attached or fixed to a substrate. In some embodiments, the features are not directly or indirectly attached or fixed to a substrate, but instead, for example, are disposed within an enclosed or partially enclosed three-dimensional space (e.g., wells or divots). For example, the plurality of capture probes can be located on features on a substrate. In some embodiments, features include, but are not limited to, a spot, an inkjet spot, a masked spot, a pit, a post, a well, a ridge, a divot, a hydrogel pad, and a bead (e.g., a hydrogel bead).

In some embodiments, the biological sample can be stained. In some embodiments, the biological sample is stained after fixation. In some embodiments, the biological sample is stained before fixation. In some embodiments, the staining includes optical labels as described herein, including, but not limited to, fluorescent (e.g., fluorophore), radioactive (e.g., radioisotope), chemiluminescent (e.g., a chemiluminescent compound), bioluminescent (e.g., bioluminescent compound), calorimetric, or colorimetric detectable labels. In some embodiments, the staining includes contacting a fluorescent antibody directed to a target analyte (e.g., cell surface or intracellular proteins) with the biological sample. In some embodiments, the staining includes an immunohistochemistry stain directed to a target analyte (e.g., cell surface or intracellular proteins) in the biological sample. In some embodiments, the staining includes a chemical stain, such as hematoxylin and/or eosin (H&E) or periodic acid-schiff (PAS). In some embodiments, staining the biological sample includes the use of a biological stain including, but not limited to, acridine orange, Bismarck brown, carmine, coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, safranin, or any combination thereof. In some embodiments, significant time (e.g., days, months, or years) can elapse between staining and/or imaging the biological sample.

In some embodiments, the biological sample is imaged. In some embodiments, the biological sample is imaged after fixation. In some embodiments, the biological sample is imaged before fixation. In some embodiments, imaging includes expansion microscopy, brightfield microscopy, dark field microscopy, phase contrast microscopy, electron microscopy, fluorescence microscopy, reflection microscopy, interference microscopy, or confocal microscopy.

In some embodiments, the biological sample is permeabilized. Permeabilization of a biological sample can occur on a substrate where the substrate is aligned with the array such that at least a portion of the biological sample is aligned with at least a portion of the array or directly on an array including a plurality of capture probes. In some embodiments, the biological sample is permeabilized with a protease. In some embodiments, the protease comprises pepsin, Proteinase K, or collagenase.

The biological sample can be applied to any of the variety of arrays described herein. In some embodiments, the plurality of capture probes includes, from a 5' to a 3' direction, a spatial barcode and a capture domain. In some embodiments, the capture domain hybridizes to a capture sequence. In some embodiments, the capture domain is a poly(T) capture domain. In some embodiments, the capture domain is not a poly(T) sequence. In some embodiments, the capture domain is a fixed sequence. As used herein, a "fixed sequence" is a non-random sequence. For example, the capture domain and the capture sequence (i.e., capture probe capture domain) of the second probe can be any sequence so long as the sequences are substantially complementary to one another to facilitate hybridization.

In some embodiments, a capture probe can include one or more functional domains and/or a cleavage domain. A functional domain can include a functional nucleotide sequence used for a downstream analytical step in the overall analysis procedure. In some embodiments, the functional domain can include a sequencing handle. In some embodiments, the functional domain can include an amplification (e.g., PCR) handle. In some embodiments, a capture probe can include a unique molecular identifier as described herein. In some embodiments, the unique molecular identifier is located 5' to the capture domain in the capture probe.

### Scenario with Spike-in mismatch probe set

In some embodiments, methods for genetic variant detection and/or localization in a biological sample comprise two sets of RNA templated ligation (RTL) probes.

Also described herein are methods for determining presence or absence of a genetic variant in a biological sample, the method including: (a) contacting the biological sample with a first and a second population of first probes, where: (i) a probe of the first population of first probes includes: (1) a sequence substantially complementary to a sequence of a target nucleic acid in the biological sample, (2) a 3' end mismatched nucleotide complementary to the genetic variant, and (3) a functional domain, and (ii) a probe of the second population of first probes includes: (1) a sequence substantially complementary to the sequence of the target nucleic acid and (2) a functional domain; (b) hybridizing the probe of the first population of first probes or the probe of the second population of first probes to the target nucleic acid; (c) contacting the biological sample with a plurality of second probes, where a second probe of the plurality of second probes includes a sequence substantially complementary to a sequence of the target nucleic acid; (d) hybridizing the second probe to the target nucleic acid and ligating: (i) the probe of the first population of first probes including the 3' end mismatched nucleotide, or (ii) the probe of the second population of first probes, to the second probe, thereby generating a ligation product; and (e) determining the presence or absence of the ligation product, thereby determining the presence or absence of the genetic variant in the biological sample.

In one embodiment, a first set of RTL probes comprises a plurality of probe pairs that targets (is complementary to a sequence of) the transcriptome, without any sequence mismatches. In another embodiment, a second set of RTL probes comprises a plurality of probe pairs, wherein the LHS probe comprises a mismatch at the 3' end. In some embodiments, the RHS of the RTL probe pairs comprise on the 5' end sequence complementary to a target nucleic acid and a capture sequence on the 3' end, wherein the capture sequence is complementary to a capture domain of a capture probe on a spatial array. In some embodiments, the LHS of the RTL probe pair comprises further comprises a functional sequence at the 5' end.

For determining the presence of a genetic variant in a target nucleic acid, the two probe pairs (i.e., mismatch and wild type ("wt") probe pairs) can be added simultaneously to a biological sample. The RTL probe pairs can then hybridize to the target nucleic acids and subsequently ligate together to generate ligation products, which can be released from the target nucleic acid. The biological sample can be permeabilized, thereby facilitating the migration of the released ligation products to the array to hybridize to the capture domains of the capture probes on the array.

In some embodiments, the two RTL probe pairs, one pair with a LHS probe having a mismatch at the 3' end and one pair with a LHS probe having wt sequence at the 3' end, overlap at one or more nucleotides of the target nucleic acid. In some embodiments, the two RTL probe pairs do not overlap on the target nucleic acid. In preferred embodiments, the two RTL probe pairs do not overlap, and as such, hybridize to different sequences on the target nucleic acid.

### Scenario for two-step hybridization and ligation

In some embodiments, methods for genetic variant detection and/or localization in a biological sample comprise a two-step hybridization scenario. The two-step hybridization scenario uses the two probe pairs, in a step wise fashion, for identifying the presence and location of a genetic variant in a target nucleic acid from a biological sample.

Also described herein are methods for determining presence or absence of a genetic variant in a biological sample, the method including:(a) contacting the biological sample with: (i) a first population of first probes, where a probe of the first population of first probes includes: (1) a sequence substantially complementary to a sequence of a target nucleic acid in the biological sample, (2) a 3' end mismatched nucleotide, and (3) a functional domain, and (ii) a plurality of second probes, where a second probe of the plurality of second probes includes a sequence substantially complementary to a sequence of the target nucleic acid; (b) hybridizing (i) the probe of the first population of first probes and (ii) the second probe of the plurality of second probes to the target nucleic acid, thereby generating a hybridized probe of the first population of first probes and a hybridized second probe of the plurality of second probes; (c) removing unhybridized probes of the first population of first probes and unhybridized probes of the plurality of second probes; (d) ligating the hybridized probe of the first population of first probes to the hybridized second probe of the plurality of second probes, thereby generating a first ligation product; (f) contacting the biological sample with: (i) a second population of first probes, where a probe of the second population of first probes includes: (1) a sequence substantially complementary to the sequence of the target nucleic acid and (2) a functional domain, and (ii) a second plurality of second probes, where a second probe of the second plurality of second probes includes a sequence substantially complementary to a sequence of the target nucleic acid, where probes of the plurality of second probes and probes of the second plurality of second probes are identical; (g) hybridizing (i) the probe of the second population of first probes and (ii) the second probe of the second plurality of second probes to the target nucleic acid, thereby generating a hybridized probe of the second population of first probes and a hybridized second probe of the second plurality of second probes; (h) ligating the hybridized probe of the second population of first probes to the hybridized second probe of the second plurality of second probes, thereby generating a second ligation product; and (i) determining the presence or absence of the first ligation product, and optionally, the second ligation product, thereby determining the presence or absence of the genetic variant in the biological sample.

In one embodiment, the LHS of the RTL probe pair comprises a mismatch at the 3' end and a functional domain at the 5' end, with target nucleic acid complementary sequence in between. In some embodiments, the RHS of the RTL probe pair comprises on the 5' end sequence complementary to a target nucleic acid and a capture sequence on the 3' end, wherein the capture sequence is complementary to a capture domain of a capture probe on a spatial array. In some embodiments, the LHS of the RTL probe pair comprises the wt nucleotide at the 3' end instead of a mismatch nucleotide. In some embodiments, a first RTL probe pair comprises a LHS probe with a mismatch at the 3' end and a RHS probe. In some embodiments, a second RTL probe pair comprises a LHS probe with a wt nucleotide at the 3' end and a RHS probe.

In some embodiments, RTL probe pairs wherein the LHS probe includes a 3' mismatch are added to a biological sample and allowed to hybridize to the target nucleic acids. In some embodiments, the hybridized probes are ligated together. In some embodiments, the biological sample is washed post-ligation at 57°C. In some embodiments, the biological sample is washed post-ligation at 65°C. Following the post-ligation wash, the second RTL probe pairs where the LHS probe includes a wt nucleotide at the 3' end are added to the biological sample and allowed to hybridize to the target nucleic acids. After hybridization of the second RTL probe pairs, the probes are ligated together, followed by post-ligation wash at 57°C. Following all ligations and washes, the ligation products are released from the target nucleic acids and the biological sample is permeabilized, thereby facilitating the migration of the released ligation products to the array where they are available to hybridize to the capture domains of the capture probes on the array.

In some embodiments, RTL probe pairs wherein the LHS probe includes a wt sequence at the 3' end are added to a biological sample and allowed to hybridize to the target nucleic acids. In some embodiments, the hybridized probes are ligated together. In some embodiments, the biological sample is washed post-ligation ligation at 57°C. In some embodiments, the biological sample is washed post-ligation at 65°C. Following the post-ligation wash, the second RTL probe pairs where the LHS probe includes a mismatch at the 3' end are added to the biological sample and allowed to hybridize to the target nucleic acids. After hybridization of the second RTL probe pairs, the probes are ligated together, followed by post-ligation wash at 57°C. Following all ligations and washes, the ligation products are released from the target nucleic acids and the biological sample is permeabilized, thereby facilitating the migration of the released ligation products to the array where they are available to hybridize to the capture domains of the capture probes on the array.

In some embodiments, the two RTL probe pairs, one with the LHS probe having a mismatch at the 3' end and the other LHS probe having wt sequence at the 3' end, overlap at one or more nucleotides of the target nucleic acid. In some embodiments, the two RTL probe pairs do not overlap on the target nucleic acid. In preferred embodiments, the two RTL probe pairs do not overlap and as such hybridize to different sequences on the target nucleic acid.

### Scenario for asymmetric probe design

In one embodiment, methods for identifying the presence or absence of a genetic variant in a target nucleic acid and its location in a biological sample comprises the use of asymmetric RTL probes.

Also described herein are methods for determining presence or absence of a genetic variant in a biological sample, the method including: (a) contacting the biological sample with a first, a second, and a third population of first probes, where: (i) a probe of the first population of first probes includes: (i) a sequence substantially complementary to a sequence of a target nucleic acid in the biological sample, (ii) an internal mismatched nucleotide complementary to the genetic variant, and (iii) a functional domain, and where the probe of the first population of first probes is at least 5 nucleotides shorter than a second probe of a plurality of second probes; (ii) a probe of the second population of first probes includes the sequence substantially complementary to the sequence of the target nucleic acid, and where the probe of the second population of first probes is at least 5 nucleotides shorter than the second probe of the plurality of second probes; and (iii) a probe of the third population of first probes includes: (i) a sequence complementary to the target nucleic acid, (ii) a 3' end mismatched nucleotide, and (iii) a functional domain, and the where the probe of the third population of first probes is at least 5 nucleotides shorter than the second probe of the plurality of second probes; (b) hybridizing: (i) the probe of the first population of first probes to the target nucleic acid, (ii) the probe of the second population of first probes to the target nucleic acid, or (iii) the probe of the third population of first probes to the target nucleic acid; (c) contacting the biological sample with the plurality of second probes, where the second probe of the plurality of second probes includes a sequence substantially complementary to a sequence of the target nucleic acid; (d) hybridizing the second probe to the target nucleic acid and ligating: (i) the probe of the first population of first probes to the second probe, thereby generating a first ligation product, (ii) the probe of the second population of first probes to a different second probe of the plurality of second probes, thereby generating a second ligation product; or (iii) the probe of the third population of first probes to a different second probe of the plurality of second probes, thereby generating a third ligation product; and (e) determining the presence or absence of the first ligation product, the second ligation product, or the third ligation product thereby determining the presence or absence of the genetic variant in the biological sample.

In some embodiments, the RHS probe is the longer of the two probes, for example, having 25nt that are complementary to a target nucleic acid and additional nucleotides that are non-complementary to the target nucleic acid sequence, but complementary to a capture domain of a capture probe on a spatial array. The LHS probe is shorter of the two probes, for example, having 19nt. In some embodiments, the LHS probe further includes either the wt sequence at the 3' end or a mismatch nucleotide at the 3' end, wherein both the mismatch and the wt LHS probes are added to the biological sample concurrently with the RHS probe, thereby creating a competitive hybridization reaction between the mismatch and the wt LHS probes. In some embodiments, the shorter LHS probe further comprises a non-complementary sequence to the target nucleic acid, an additional sequence that serves as a functional domain, for example, a primer domain or a sequencing domain.

In some embodiments, the shorter LHS probe includes a wt sequence throughout the entire probe that is complementary to the target nucleic acid, whereas the shorter, mismatch LHS probe includes the mismatch internal to the probe sequence and not at the 3' end. For example, an internal mismatch to identify the presence or absence of a genetic variant in a target nucleic acid could be -1, -2, -3, -4, -5, -6, -7, -8, -9, -10, -11, -12, -13, -14, -15, -16, - 17, -18, or -19 nucleotides from the 3' end. As described herein, an example RTL probe pair can include an asymmetric 19nt LHS probe with a mismatch at -9 nucleotides from the 3' end. As with the shorter LHS probes where a mismatch is located at the 3' end, the internal mismatch probe and the shorter wt LHS probe can be added to the biological sample concurrently with the RHS probe, thereby creating a competitive hybridization reaction between the mismatch and the wt LHS probes. In some embodiments, the shorter LHS probe further comprises a non-complementary sequence to the target nucleic acid, an additional sequence that serves as a functional domain, for example, a primer domain or a sequencing domain.

In some embodiments, the shorter LHS probes and the longer RHS probes hybridize adjacently (i.e., to adjacent sequences) on the target nucleic acid. After hybridization, the shorter LHS probe (with a mismatch or with wt sequence) can be ligated to the hybridized RHS probe, thereby generating a ligation product. Ligation can be performed using a ligase, for example, a PBCV-1 ligase, a *Chlorella* ligase, or T4 DNA ligase, etc. The ligation product can then be released from the target nucleic acid, thereby allowing the ligation product to migrate and hybridize to a capture probe on a spatial array.

The ligation product can be released from the target nucleic acid via RNase digestion of the template nucleic acid. In some embodiments, RNase H digestion of the template nucleic acid (e.g., mRNA) releases the ligation product from the hybridized duplex. In some embodiments, the biological sample is concurrently, previously, or subsequently permeabilized, thereby facilitating the migration of the ligation product from the biological sample to the arrayed substrate, wherein the capture sequence of the ligation product can hybridize to the capture domain of the capture probe on the array surface. Permeabilization can be performed as previously described, for example, by addition of a detergent and/or a protease to the biological sample before, during, or after the addition of the RTL probes to the biological sample.

The ligation products that are captured by the capture domains of the capture probes on the array can therefore be used as proxies of the target nucleic acid and act as an indirect method of determining the sequence of the target nucleic acid and whether the target nucleic acid comprises a genetic variant at a queried location. Upon hybridization of the ligation product to the capture domain of the capture probe, the probe can be extended using the ligation product as a template and the ligation product can also be extended using the capture probe as a template. The extended ligation product can be harvested from the array prior to generation of sequencing libraries generated and sequencing. The sequencing of the ligation products can be used to determine the presence or absence of a genetic variant in a biological sample. Further, as the array is a spatial array, the sequence data can also be used to determine the location of the genetic variant in the biological sample, thereby spatially correlating the gene and its variant, or wt, to its original location in the biological sample.

In some embodiments, the asymmetric probe scenario for genetic variant detection and location in a biological sample is performed concurrently with a whole transcriptome query of the biological sample as previously described. As such, genetic variants can both be identified, if present, and spatially determined in context of spatial determinations of the whole transcriptome of the biological sample.

### Scenario for third oligonucleotide gap fill & ligation

In one embodiment, methods for identifying the presence or absence of a genetic variant in a target nucleic acid and its location in a biological sample comprise the use of a third oligonucleotide in combination with the first and second RTL probes.

Also described herein are methods for determining presence or absence of a genetic variant in a biological sample, the method including: (a) contacting the biological sample with: (i) a plurality of first probes, where a first probe of the plurality of first probes includes: (1) a sequence substantially complementary to a sequence of a target nucleic acid in the biological sample and (2) a functional domain, and (ii) a plurality of second probes, where a second probe of the plurality of second probes includes: (1) a sequence substantially complementary to a sequence of the target nucleic acid and (2) a functional domain, where the first probe and second probe hybridize to sequences on the target nucleic acid that are at least 16 nucleotides apart; (b) hybridizing the first probe and the second probe to the target nucleic acid, thereby generating a hybridized first probe and a hybridized second probe; (c) contacting the biological sample with a first and a second population of third probes, where: (i) a probe of the first population of third probes includes a sequence complementary to the target nucleic acid between the first probe and the second probe and a mismatched nucleotide and (ii) a probe of the second population of third probes includes a sequence complementary to the target nucleic acid; (d) hybridizing the probe of the first population and the probe of the second population in (c) to the target nucleic acid, thereby generating a hybridized probe of the first population and a hybridized probe of the second population; (e) ligating: (i) the hybridized first probe to the hybridized probe of the first population of third probes and (ii) the hybridized probe of the first population of third probes to the hybridized second probe, thereby generating a first ligation product, and/or ligating: (i) a different hybridized first probe to the hybridized probe of the second population of third probes and (ii) the hybridized probe of the second population of third probes to a different hybridized second probe, thereby generating a second ligation product; and (f) determining the presence or absence of the first ligation product, and optionally, the second ligation product, thereby determining the presence or absence of the genetic variant in the biological sample.

In some embodiments, the LHS and RHS probes are each 20nt long and a third oligonucleotide is 16nt long. In some embodiments, the third oligonucleotide includes a mutation which is indicative of a genetic variant present in a target nucleic acid sequence or the third oligonucleotide includes the wt nucleotide at the position of a potential genetic variant. In this third oligonucleotide scenario, the LHS and RHS RTL probes do not hybridize adjacently on the target nucleic acid. Instead, the LHS and RHS RTL probes hybridize to target nucleic acid sequences that are at least 16nt apart thereby generating a gap on the target nucleic acid where the third oligonucleotide can hybridize.

The gap fill genetic variant detection scenario described herein includes a LHS RTL probe, a RHS RTL probe, an oligonucleotide with a mismatched nucleotide and an oligonucleotide with a wt nucleotide which are combined and added to a biological sample. The LHS and RHS RTL probes hybridize to their target nucleic acids while the oligonucleotides with a mismatch or wt nucleotide compete for hybridizing in the gap between the two RTL probes. In one embodiment, the oligonucleotide that comprises a nucleotide that is complementary to a genetic variant hybridizes in the gap between the LHS and RHS RTL probes on a target nucleic acid, thereby detecting the presence or absence of a genetic variant in the target nucleic acid. In other embodiments, the oligonucleotide that comprises a nucleotide that is complementary to a wildtype sequence hybridizes in the gap between the LHS and RHS RTL probes on a target nucleic acid, thereby determining an absence of a genetic variant in the target nucleic acid.

In some embodiments, the three hybridized probes/oligonucleotides are ligated together generating a ligation product using a ligase, for example, PBCV-1 ligase, a *Chlorella* species ligase, or T4 DNA ligase. In some embodiments, the LHS RTL probe comprises a functional sequence, whereas the RHS RTL probes comprises a capture sequence that is complementary to a capture domain on a capture probe on a spatial array.

The ligation product can be subsequently released from the target nucleic acid via RNase digestion of the template nucleic acid. In some embodiments, RNase H digestion of the template nucleic acid (e.g., mRNA) releases the ligation product from the hybridized duplex. In some embodiments, the biological sample is concurrently, previously, or subsequently permeabilized, thereby facilitating the migration of the ligation product from the biological sample to the arrayed substrate, wherein the capture sequence of the ligation product can hybridize to the capture domain of the capture probe on the array surface. Permeabilization can be performed as previously described, for example, by addition of a detergent and/or a protease to the biological sample before, during, or after the addition of the probes/oligonucleotides to the biological sample.

The ligation products that are captured by the capture domains of the capture probes on the array can therefore be used as proxies of the target nucleic acid and act as an indirect method of determining the sequence of the target nucleic acid and whether the target nucleic acid comprises a genetic variant at a queried location. Upon hybridization of the ligation product to the capture domain of the capture probe, the probe can be extended using the ligation product as a template and the ligation product can also be extended using the capture probe as a template. The extended ligation product can be harvested from the array prior to generation of sequencing libraries and sequencing. The sequencing of the ligation products can be used to determine the presence or absence of a genetic variant in a biological sample. Further, as the array is a spatial array, the sequence data can also be used to determine the location of the genetic variant in the biological sample, thereby spatially correlating the gene and it variant, or wt, to its original location in the biological sample.

In some embodiments, the gap fill scenario for genetic variant detection and location in a biological sample is performed concurrently with a whole transcriptome query of the biological sample as previously described. As such, genetic variants can both be identified, if present, and spatially determined in context of spatial determinations of the whole transcriptome of the biological sample.

### Kits

The present disclosure also features kits useful for the spatial detection of genetic variants in a biological sample (e.g., a tissue section). Thus described herein are kits including: (a) a plurality of first probes, where a first probe of the plurality of first probes includes a sequence substantially complementary to a target nucleic acid; (b) a plurality of second probes, where a second probe of the plurality of second probes includes: (i) a sequence substantially complementary to the target nucleic acid 5' to the first probe and (ii) a capture probe capture domain; (c) a plurality of reversible terminator nucleotides; (d) a polymerase; and (e) instructions for performing any of the methods described herein.

Also described herein are kits including: (a) a first and a second population of first probes, wherein: (i) a probe of the first population of first probes comprises a sequence substantially complementary to a sequence of a target nucleic acid and comprises a 3' end mismatched nucleotide and a functional domain, and (ii) a probe of the second population of first probes comprises a sequence substantially complementary to the sequence of the target nucleic acid and a functional domain; (b) a plurality of second probes, wherein a second probe of the plurality of second probes comprises a sequence substantially complementary to a sequence of the target nucleic acid; (c) a ligase; and (d) instructions for performing any of the methods described herein.

In some embodiments, the probe of the first population of first probes, the probe of the second population of first probes, and the second probe each comprise about 20 to about 35 nucleotides or more (e.g., about 20, about 21, about 22, about 23, about 24, about 25, about 26, about 27, about 28, about 20, about 30, about 31, about 32, about 33, about 34, about 35, or more nucleotides). In some embodiments, the probe of the first population of first probes, the probe of the second population of first probes, and the second probe each comprise 25 nucleotides.

In some embodiments, the probe of the first population of first probes and the probe of the second population of first probes each comprises 19 nucleotides and the second probe comprises 25 nucleotides.

Also described herein are kits including: (a) a first and a second population of first probes, wherein: (i) a probe of the first population of first probes comprises a sequence substantially complementary to a sequence of a target nucleic acid and comprises an internal mismatched nucleotide and a functional domain, and (ii) a probe of the second population of first probes comprises the sequence substantially complementary to the sequence of the target nucleic acid, and wherein the probe of the first population of first probes is at least 5 nucleotides shorter than a second probe; (b) a plurality of second probes, wherein the second probe of the plurality of second probes comprises a sequence substantially complementary to a sequence of the target nucleic acid; (c) a ligase; and (d) instructions for performing any of the methods described herein.

In some embodiments, the probe of the first population of first probes and the probe of the second population of first probes comprise about 15, about 16, about 17, about 18, about 19, or about 20 nucleotides and the second probe comprises about 20, about 21, about 22, about 23, about 24, or about 25 nucleotides, respectively. In some embodiments, the probe of the first population of first probes and the probe of the second population of first probes comprises 19 nucleotides and the second probe comprises 25 nucleotides.

In some embodiments, the kit includes a third population of first probes, wherein a probe of the third population of first probes comprises a sequence complementary to the target nucleic acid and comprises a 3' end mismatched nucleotide and a functional domain and the wherein the probe of the third population of first probes is at least 5 nucleotides shorter than the second probe.

Also described herein are kits including: (a) a plurality of first probes, wherein a first probe of the plurality of first probes comprises a sequence substantially complementary to a sequence of a target nucleic acid and a functional domain; (b) a plurality of second probes, wherein a second probe of the plurality of second probes comprises a sequence substantially complementary to a sequence of the target nucleic acid, wherein the first probe and second probe hybridize to sequences on the target nucleic acid at least 16 nucleotides apart; (c) a first and a second population of third probes, wherein: (i) a probe of the first population of third probes comprises a sequence complementary to the target nucleic acid between the first probe and the second probe and a mismatched nucleotide and (ii) a probe of the second population of third probes comprises a sequence complementary to the target nucleic acid; (d) a ligase; and (e) instructions for performing any of the methods described herein.

In some embodiments, the second probe includes a capture probe capture domain (e.g., a sequence substantially complementary to a capture domain of a capture probe on an array (e.g., a spatial array). In some embodiments, the second probe does not include a capture probe capture domain.

In some embodiments, the kit includes a hybridization buffer. In some embodiments, the kit includes a wash buffer.

In some embodiments, the kit includes a spatial array including a plurality of capture probes, where a capture of the plurality of capture probes includes: (i) a spatial barcode, and (ii) a capture domain (e.g., any of the capture domains described herein).

Capture probes of the spatial array can include additional domains. For example, a capture can include one or more functional domains, a cleavage domain, a unique molecular identifier (UMI), and a combination thereof. In some embodiments, the one or more functional domains includes a primer binding site or a sequencing specific site.

In some embodiments, the polymerase includes a DNA polymerase. Any suitable DNA polymerase can be included in the kits described herein. For example, in some embodiments, the DNA polymerase includes Bst 3 polymerase and/or Klenow polymerase. In some embodiments, the kit includes a reverse transcriptase. Non-limiting examples of reverse transcriptases include KOD reverse transcriptase or Moloney Murine Leukemia Virus (M-MulV) reverse transcriptase.

In some embodiments, the kit includes one or more permeabilization reagents. In some embodiments, the one or more permeabilization reagents includes one or more proteases, a DNase, an RNase, a lipase, a detergent, and a combination thereof. In some embodiments, the one or more proteases includes pepsin, Proteinase K, and/or collagenase.

The reversible terminator nucleotides of the present disclosure includes both the use of blocked (e.g., 3' blocked) reversible terminator nucleotides or unblocked reversible terminator nucleotides.

In some embodiments, the reversible terminator nucleotide includes a 3' blocking group. Non-limiting examples of reversible terminator nucleotides with a 3' blocking group includes: 3'-azidomethyl dNTP, 3'-O-allyl dNTP, 3'-O-nitrobenzyl dNTP, 3'-O-(2-nitrobenzyl) dNTP; or 3'-O-dithiomethyl dNTP.

In some embodiments, the reversible terminator nucleotide includes an unblocked reversible terminator nucleotide. In some embodiments, the unblocked reversible terminator nucleotide includes a 5-hydroxymethyl-2'-deoxy triphosphate derivative.

In some embodiments, the kit includes a ligase. Non-limiting examples of ligases include a PBCV-1 ligase, a *Chlorella* DNA ligase, a single stranded DNA ligase, and/or a T4 DNA ligase.

In some embodiments, the kit includes a reducing agent. In some embodiments, the reducing agent includes tris(2-carboxyethyl)phosphine (TCEP) or dithiothreitol (DTT).

### Compositions

The present disclosure also features compositions in addition to the methods and kits described herein useful for the spatial detection of genetic variants in a biological sample (e.g., a tissue section).

Thus described herein are compositions including: (a) a spatial array including a plurality of capture probes, where a capture probe of the plurality of capture probes includes: (i) a spatial barcode and (ii) a capture domain; (b) a plurality of first probes, where a first probe of the plurality of first probes includes a sequence substantially complementary to a target nucleic acid; (c) a plurality of second probes, where a second probe of the plurality of second probes includes: (i) a sequence substantially complementary to the target nucleic acid 5' to the first probe and (ii) a capture probe capture domain; (d) a plurality of reversible terminator nucleotides; and (e) a polymerase.

Also described herein are compositions including: (a) a plurality of probe pairs (e.g., a plurality of first probes and a plurality of second probes), where a first probe of the plurality of probe pairs includes a sequence substantially complementary to a target nucleic acid and where a second probe of the plurality of probes includes a sequence substantially complementary to the target nucleic acid 5' to the first probe (b) a plurality of reversible terminator nucleotides; and (c) a polymerase.

Also described herein, are compositions including (a) a plurality of probe pairs (e.g., a plurality of first probes and a plurality of second probes), where a first probe of the plurality of probe pairs is hybridized to a target nucleic acid and a second probe of the plurality of probes where the second probe includes a sequence substantially complementary to the target nucleic acid 5' to the first probe (b) a plurality of reversible terminator nucleotides; and (c) a polymerase.

Also described herein, are compositions including: (a) a plurality of probe pairs (e.g., a plurality of first probes and a plurality of second probes), where a first probe of the plurality of probe pairs is hybridized to a target nucleic acid and where a second probe of the plurality of probes is hybridized to the target nucleic acid 5' to the first probe (b) a plurality of reversible terminator nucleotides; and (c) a polymerase.

In some embodiments, the first probe is present in the compositions. In some embodiments, the first probe is hybridized to the target nucleic acid. In some embodiments, the first probe is extended by the polymerase using the target nucleic acid as a template thereby incorporating the reversible terminator nucleotide and generating an extended probe. In some embodiments, the first probe is extended with one or more (e.g., 2, 3, 4, 5 or more) reversible terminator nucleotides (e.g., any of the reversible terminator nucleotides described herein). In some embodiments, the first probe is extended prior to the second probe hybridizing to the target nucleic acid. In some embodiments, the first probe is extended after the second probe hybridizes to the target nucleic acid. For example, the second probe can be hybridized to the target nucleic acid prior to the extension of the first probe such that one nucleotide or more gap is present between the hybridized first probe and the hybridized second probe. Conversely, the second probe can be hybridized to the target nucleic acid after the extension of the first probe with one or more reversible terminator nucleotides.

In some embodiments, the composition includes a spatial array. For example, the spatial array can include a plurality of capture probes, where a capture of the plurality of capture probes includes: (i) a spatial barcode, and (ii) a capture domain (e.g., any of the capture domains described herein).

In some embodiments, the extended probe (e.g., extended by one or more reversible terminator nucleotides) and the second probe are ligated. In some embodiments, ligation comprises use of a ligase. Any suitable ligase can be used. Non-limiting examples of ligases include a PBCV-1 ligase, a *Chlorella* DNA ligase, a single stranded DNA ligase, and/or a T4 DNA ligase.

In some embodiments, the reversible terminator nucleotide includes a 3' blocking group. Any 3' blocking group can be used by any of the methods described herein. Non-limiting examples of 3' blocking groups include 3'-azidomethyl dNTP, 3'-O-allyl dNTP, 3'-O-nitrobenzyl dNTP, 3'-O-(2-nitrobenzyl) dNTP; or 3'-O-dithiomethyl dNTP.

In some embodiments, the reversible terminator nucleotide includes an unblocked reversible terminator nucleotide. In some embodiments, the unblocked reversible terminator nucleotide includes a 5-hydroxymethyl-2'-deoxy triphosphate derivative.

In embodiments, that include a spatial array with a plurality of capture probes, the capture probe can include additional domains. In some embodiments, the capture probe includes one or more functional domains, a cleavage domain, a unique molecular identifier (UMI), and a combination thereof. In some embodiments, the one or more functional domains includes a primer binding site or a sequencing specific site.

In some embodiments, the composition includes a polymerase. In some embodiments, the polymerase is a DNA polymerase. Any suitable DNA polymerase can be used. Non-limiting examples of DNA polymerases include Klenow polymerase and Bst 3 polymerase. In some embodiments, the composition includes a reverse transcriptase. In some embodiments, the reverse transcriptase is one or more of KOD reverse transcriptase or Moloney Murine Leukemia Virus (M-MulV) reverse transcriptase.

In some embodiments, the composition includes a protease. In some embodiments, the protease includes pepsin, Proteinase K, or collagenase. In some embodiments, the composition includes a DNase.

In some embodiments, the composition includes a reducing agent. In some embodiments, the reducing agent includes tris(2-carboxyethyl)phosphine (TCEP) or dithiothreitol (DTT).

Also described herein are compositions including: (a) a first and a second population of first probes, wherein: (i) a probe of the first population of first probes comprises a sequence substantially complementary to a sequence of a target nucleic acid and comprises a 3' end mismatched nucleotide and a functional domain, and (ii) a probe of the second population of first probes comprises a sequence substantially complementary to the sequence of the target nucleic acid and a functional domain; (b) a plurality of second probes, wherein a second probe of the plurality of second probes comprises a sequence substantially complementary to a sequence of the target nucleic acid; and (c) a ligase (e.g., any of the ligases described herein).

In some embodiments, the probe of the first population of first probes is hybridized to a target nucleic acid. In some embodiments, the probe of the second population of first probes is hybridized to a target nucleic acid. In such examples, the probe of the first population and the probe of the second population hybridize to target nucleic acids, however, the probe of the first population hybridizes to a target nucleic acid including a genetic variant (e.g., any of the genetic variants described herein). In some embodiments, the probe of the first population is ligated to the second probe, thereby generating a first ligation product. In some embodiments, the probe of the second population is ligated to the second probe, thereby generating a second ligation product. In some embodiments, the probe of the first population is ligated to a second probe and the probe of the first population is ligated to a different second probe.

In some embodiments, the probe of the first population of first probes, the probe of the second population of first probes, and the second probe each comprise about 20 to about 35 nucleotides or more (e.g., about 20, about 21, about 22, about 23, about 24, about 25, about 26, about 27, about 28, about 20, about 30, about 31, about 32, about 33, about 34, about 35, or more nucleotides). In some embodiments, the probe of the first population of first probes, the probe of the second population of first probes, and the second probe each comprise 25 nucleotides.

In some embodiments, the probe of the first population of first probes and the probe of the second population of first probes each comprises 19 nucleotides and the second probe comprises 25 nucleotides.

In some embodiments, the probe of the first population of first probes and the probe of the second population of first probes are contacted with the biological sample at about the same time. In some embodiments, the probe of the first population of first probes is contacted with the biological sample before the probe of second population of first probes is contacted with the biological sample and allowed to hybridize to target nucleic acids before the probe of the second population of first probes.

Also described herein are compositions including: (a) a first and a second population of first probes, wherein: (i) a probe of the first population of first probes comprises a sequence substantially complementary to a sequence of a target nucleic acid and comprises an internal mismatched nucleotide and a functional domain, and (ii) a probe of the second population of first probes comprises the sequence substantially complementary to the sequence of the target nucleic acid, and wherein the probe of the first population of first probes is at least 5 nucleotides shorter than a second probe; (b) a plurality of second probes, wherein the second probe of the plurality of second probes comprises a sequence substantially complementary to a sequence of the target nucleic acid; (c) a ligase; and (d) instructions for performing any of the methods described herein.

In some embodiments, the probe of the first population of first probes and the probe of the second population of first probes comprise about 15, about 16, about 17, about 18, about 19, or about 20 nucleotides and the second probe comprises about 20, about 21, about 22, about 23, about 24, or about 25 nucleotides, respectively. In some embodiments, the probe of the first population of first probes and the probe of the second population of first probes comprises 19 nucleotides and the second probe comprises 25 nucleotides.

In some embodiments, the composition includes a third population of first probes, wherein a probe of the third population of first probes comprises a sequence complementary to the target nucleic acid and comprises a 3' end mismatched nucleotide and a functional domain and the wherein the probe of the third population of first probes is at least 5 nucleotides shorter than the second probe.

In some embodiments, the probe of the first population of first probes is hybridized to a target nucleic acid. In some embodiments, the probe of the second population of first probes is hybridized to a target nucleic acid. In some embodiments, the probe of the third population of first probes is hybridized to a target nucleic acid. In some embodiments, the probe of the first population is ligated to the second probe, thereby generating a first ligation product. In some embodiments, the probe of the second population is ligated to the second probe, thereby generating a second ligation product. In some embodiments, the probe of the third population is ligated to the second probe, thereby generating a third ligation product. In some embodiments, the probe of the first population is ligated to a second probe, the probe of the first population is ligated to a different second probe, and the probe of the third population is ligated to another different second probe.

Also described herein are compositions including: (a) a plurality of first probes, wherein a first probe of the plurality of first probes comprises a sequence substantially complementary to a sequence of a target nucleic acid and a functional domain; (b) a plurality of second probes, wherein a second probe of the plurality of second probes comprises a sequence substantially complementary to a sequence of the target nucleic acid, wherein the first probe and second probe hybridize to sequences on the target nucleic acid at least 16 nucleotides apart; (c) a first and a second population of third probes, wherein: (i) a probe of the first population of third probes comprises a sequence complementary to the target nucleic acid between the first probe and the second probe and a mismatched nucleotide and (ii) a probe of the second population of third probes comprises a sequence complementary to the target nucleic acid; (d) a ligase; and (e) instructions for performing any of the methods described herein.

In some embodiments, the first probe of the plurality of first probes is hybridized to the target nucleic acid. In some embodiments, the second probe of the plurality of second probes is hybridized to the target nucleic acid. In some embodiments, the first and the second population of third probes, are hybridized to the target nucleic acids (e.g., different target nucleic acids).

In some embodiments, the first probe is ligated to the probe of the first population of third probes. In such embodiments, the product comprising the first probe and the probe of the first population of third probes can be ligated to the second probe, thereby generating a first ligation product.

In some embodiments, the first probe is ligated to the probe of the second population of third probes. In such embodiments, the product comprising the first probe and the probe of the second population of third probes can be ligated to the second probe, thereby generating a second ligation product.

In some embodiments, the second probe includes a capture probe capture domain (e.g., a sequence substantially complementary to a capture domain of a capture probe on an array (e.g., a spatial array). In some embodiments, the second probe does not include a capture probe capture domain.

In some embodiments, the composition includes a hybridization buffer. In some embodiments, the composition includes a wash buffer.

### EXAMPLES

### Example 1. Spatial Detection of Genetic Variants via Templated Ligation and Reversible Terminator Nucleotides.

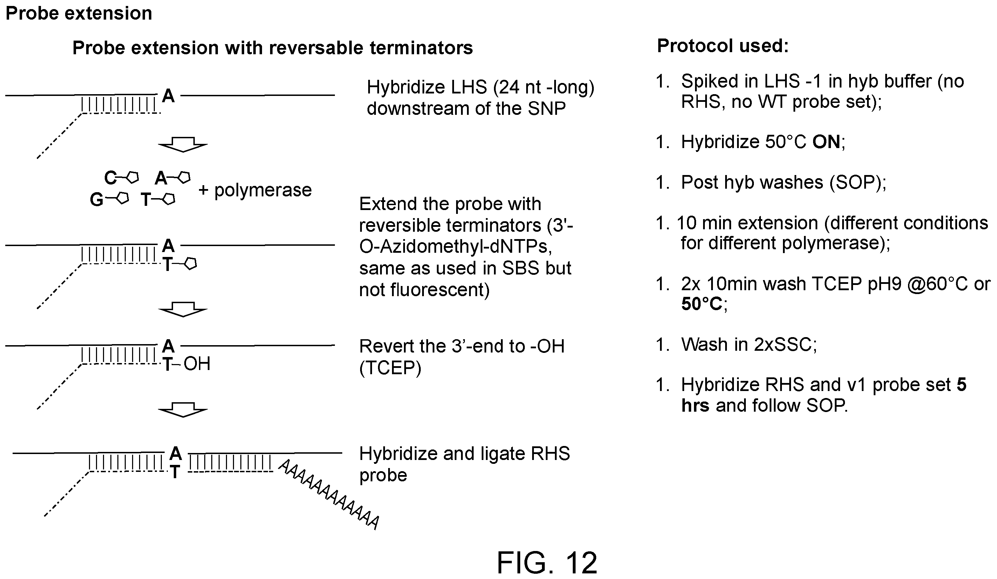

**FIG. 12** is a schematic diagram of a method described herein depicting genetic variant detection after hybridization of a first probe to a target nucleic acid in a biological sample, extension of the hybridized first probe using a reversible terminator nucleotide, hybridization of a second probe to the target nucleic acid, and ligation of the hybridized first probe and hybridized second probe. Various polymerases and temperatures were tested to identify the most effective experimental conditions.

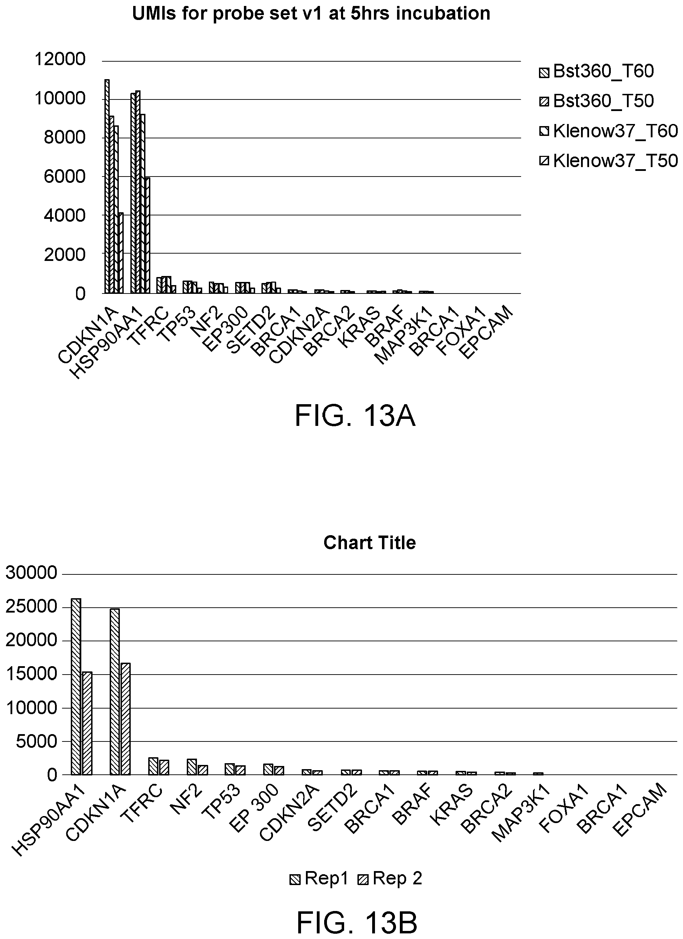

**FIGs. 13A-13B** are graphs showing unique molecular identifier counts for various genes using different polymerases at different hybridization temperatures and durations using the method shown in **FIG. 12****.** Specifically, two polymerases, Bst 3 polymerase and Klenow polymerase, were tested at different hybridization durations (5 hours and overnight). Removal of the blocking group using a reducing agent, such as TCEP, was also tested at both 50°C and 60°C. **FIG. 13A** shows results after 5 hours of hybridization and **FIG. 13B** shows results after overnight hybridization.

Experimentally, the first probe was contacted with the biological sample in a hybridization buffer and allowed to hybridize to the target nucleic acid at 50°C overnight or for 5 hours. The biological sample was washed in buffer, followed by a 10 minute extension reaction to incorporate the reversible terminator nucleotide to generate the extended first probe. The extension reactions were performed with either Bst 3 polymerase or Klenow polymerase, followed by removal of the blocking group at either 50°C or 60°C. Next, the second probe was contacted with the biological sample and allowed to hybridize to the target nucleic acid, followed by ligation to the extended first probe.

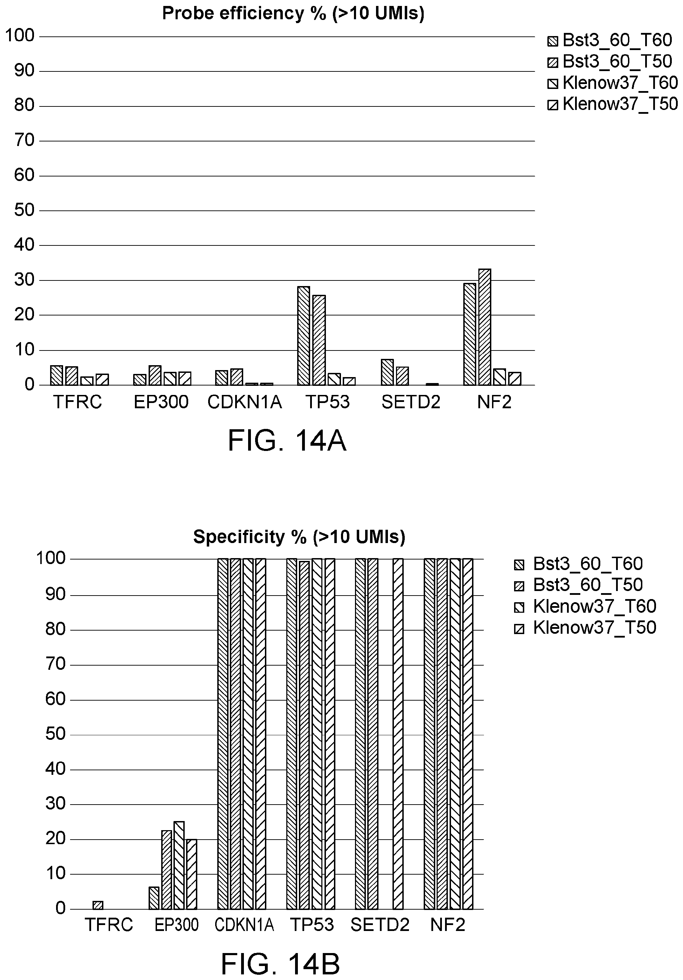

**FIGs. 14A-14B** show probe efficiency **(****FIG. 14A****)** and specificity **(****FIG. 14B****)** for various genes using either Bst 3 polymerase or Klenow polymerase at different reducing temperatures (e.g., 50°C or 60°C) using the method of **FIG. 12****.**

Collectively, the data demonstrate that Bst 3 polymerase generally performs better than Klenow polymerase, and a blocking group removal temperature of either 50°C or 60°C works comparably well.

### Example 2. Spatial Detection of Genetic Variants via Templated Ligation and either Reversible Terminator Nucleotides or dNTPs.

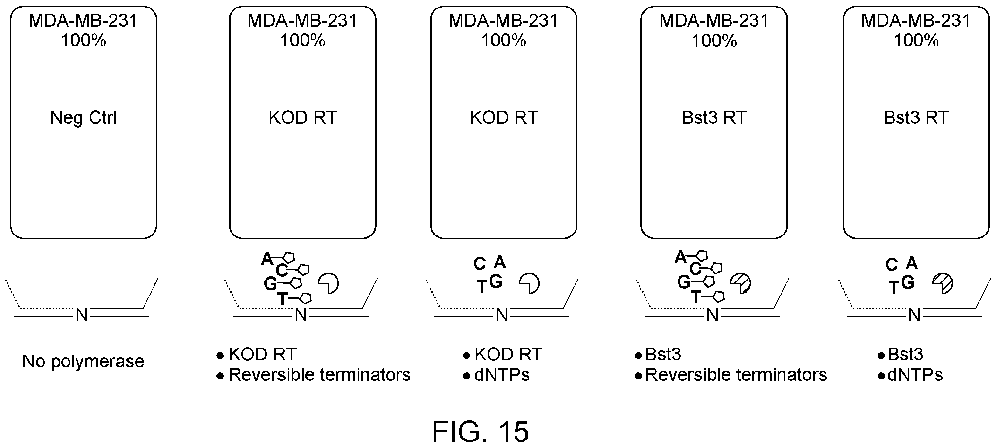

**FIG. 15** is a schematic diagram showing the method of **FIG. 12** using Bst 3 polymerase and KOD reverse transcriptase with either reversible terminator nucleotides or dNTPs.

Experimentally, the same protocol was followed as described in Example 1, but with the additional process of incorporating dNTPs or reversible terminator nucleotides during the extension of the first probe. Bst 3 polymerase in combination with reversible terminator nucleotides generated usable sequencing data (89.1% Fraction Reads Usable; 105 Median genes per spot (5k mapped spot-reads per spot); and 107 Median genes per spot; data not shown) relative to KOD reverse transcriptase with either reversible terminator nucleotides or traditional dNTPs.

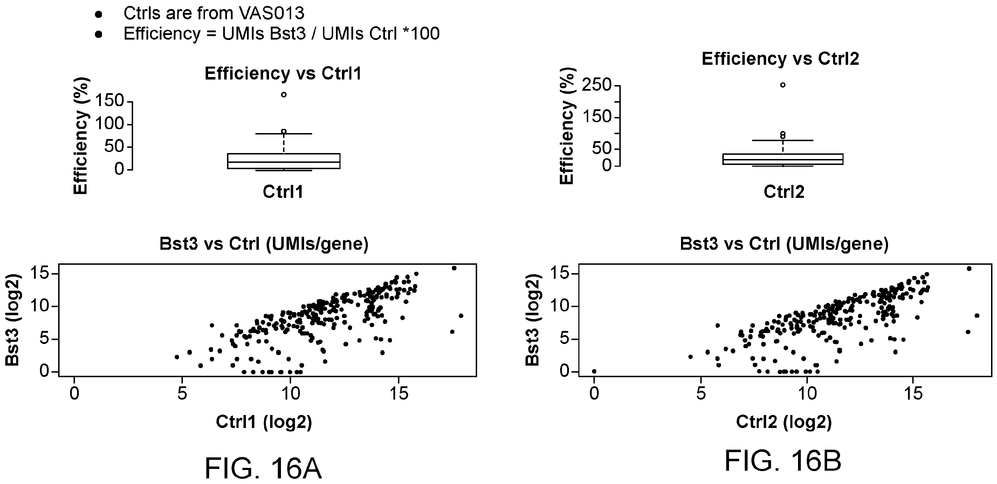

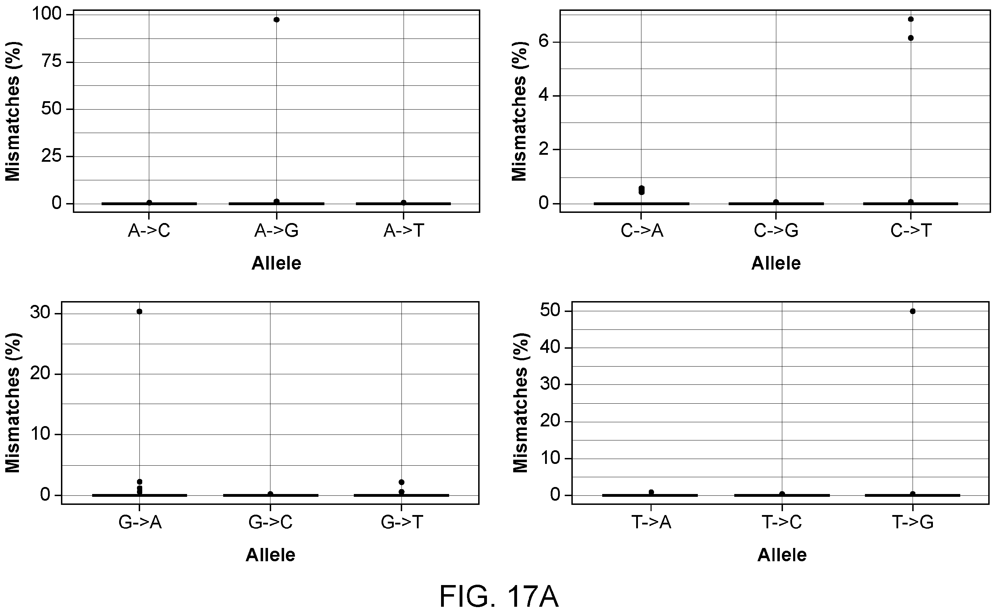

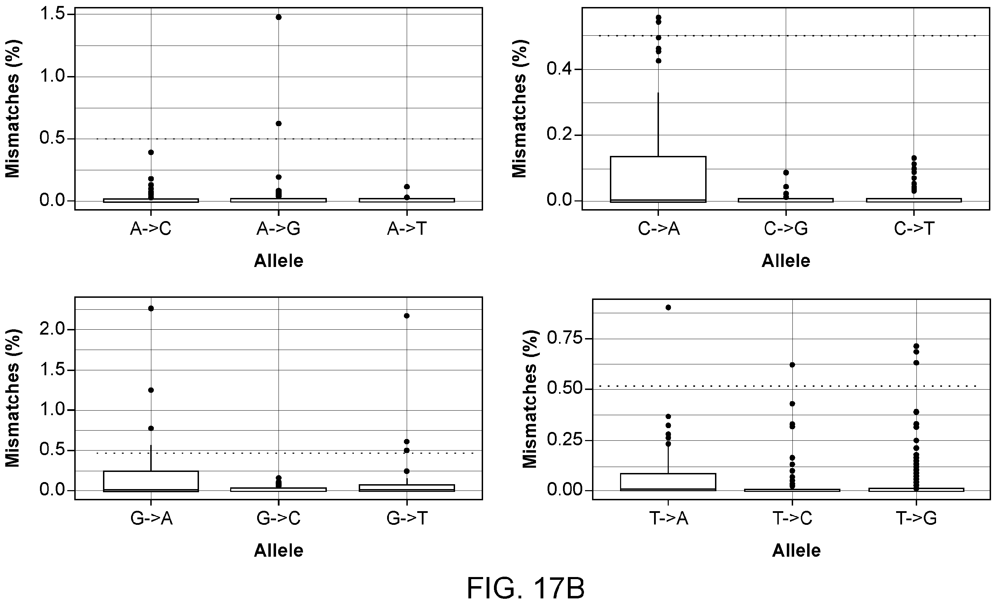

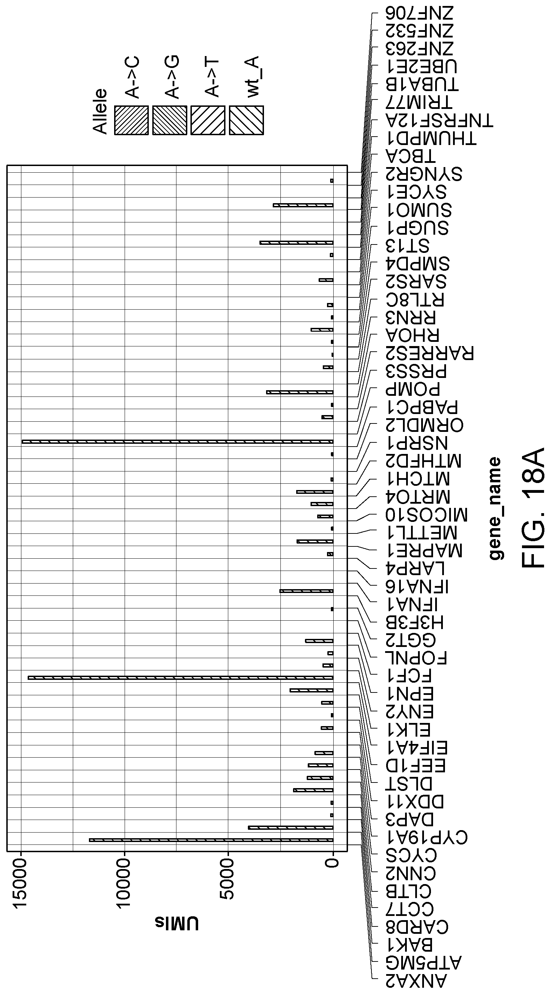

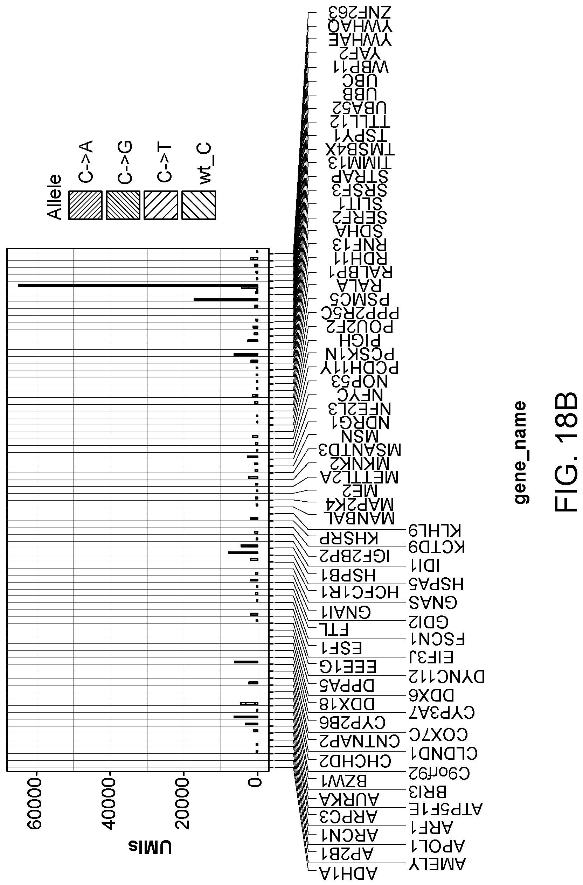

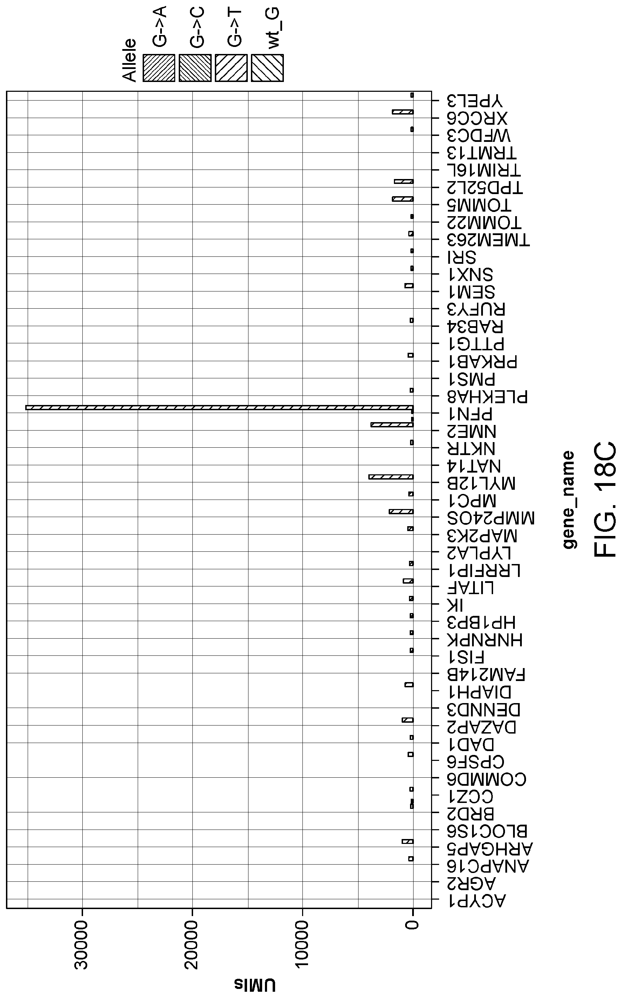

**FIGs. 16A-16B** are graphs showing unique molecular identifier capture efficiency with Bst 3 polymerase. Capture efficiency was calculated by the number of unique molecular identifiers identified with the method shown in **FIG. 12** (Bst 3) divided by the number of unique molecular identifiers by the standard templated ligation method (Ctrl1 and Ctrl2) (i.e., Efficiency = (UMIs Bst 3/UMIs Ctrl)*100). The data demonstrate that the method of **FIG. 12** was effective in capturing target nucleic acids. **FIGs. 17A-17B** are graphs showing the specificity of the method of **FIG. 12****.** The percentage of mismatched reads were minimal or zero. **FIGs. 18A-18C** are graphs showing detection of specific genes including detection of various alleles including wildtype alleles.

Collectively, the data demonstrate that the described method including extension of a first probe using Bst 3 polymerase and reversible terminator nucleotides, followed by ligation to a second probe has high specificity and can used to detect numerous genes with various alleles in a biological sample.

### Example 3. Genetic variant detection using spike-in mismatch probe set scenario.

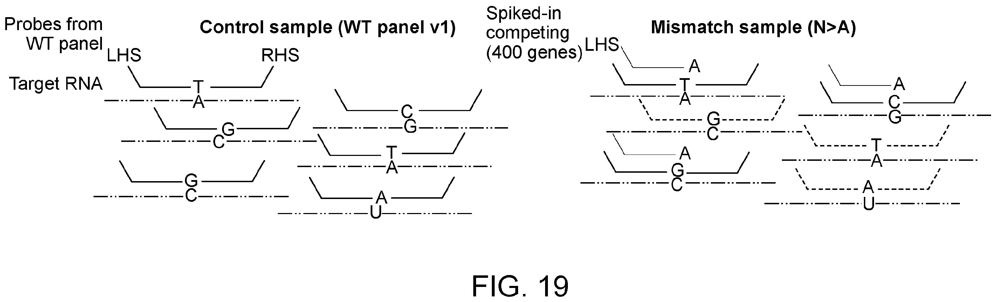

In this example, a spike-in probe set was used for detecting a genetic variant in a target nucleic acid in a biological sample. RNA templated ligation ("RTL") probe pairs (800 wildtype ("wt") probe pairs) were applied to the biological sample in conjunction with 400 RTL probe pairs in which the left-hand side (LHS) probes included a mismatch at the 3' end **(****FIG. 19****).** Each of the RTL probe pairs included a LHS probe and a right-hand side (RHS) probe, which can be ligated together. The LHS probes included either a mismatch (spike-in) or wt at the 3' end, and a functional sequence that is not complementary to the target nucleic acid (e.g., at the 5' end of the LHS probe). The RHS probe included a capture domain that is not complementary to the target nucleic acid (e.g., at the 3' end of the RHS probe), but instead complementary to a capture domain of a capture probe on a spatial array.

A section of FFPE pelleted MDA-MD-231 or Hs742.sk cells was placed on a tissue slide and then deparaffinized, H&E stained, and imaged. Following imaging, the tissue sample was destained and decrosslinked. The RTL probes, including the spike-in probe sets, were then added to the tissue sample and allowed to hybridize overnight to their target nucleic acids in the tissue sample. The two probe pairs were expected to target different locations on the target nucleic acid, for example, such that the probe pairs do not hybridize to overlapping sequences of the target nucleic acid. Following hybridization, the hybridized probes were ligated together using a ligase. After ligation, the tissue sample was washed, the ligation products were released from the target nucleic acid, hybridized to capture probes on the spatial array slide, and then extended using the capture probes as a template. Library preparation for sequencing of the ligation products was performed per manufacturer's user guide (Visium CytAssist Spatial Gene Expression Reagent Kits, CG000495). After sequencing of the ligation products, the data were analyzed using SpaceRanger, and specificity and efficiency of the spike-in mismatch scenario for identifying genetic variants was graphed.

The data were analyzed for specificity and efficiency of ligation (sensitivity). For specificity, (1-false ligation rate)x100=% specificity, such that highly specific is defined as when one probe does not hybridize and ligate to the wrong target. For efficiency (i.e. sensitivity), (normalized UMIs of match + mismatch probes/normalized UMIs for match probe of the control sample)x100=% efficiency, such that the amount of total signal detected in the spike-in mismatch scenario (correct and incorrect) is compared to the amount of total signal detected in a non-competing control experiment after normalization, where normalization was performed using all probes from the wt probes not participating in competition (or a subset of the non-competing probes). The efficiency control was set at 100% and is indicated by a dashed line at 100% in efficiency graphs.

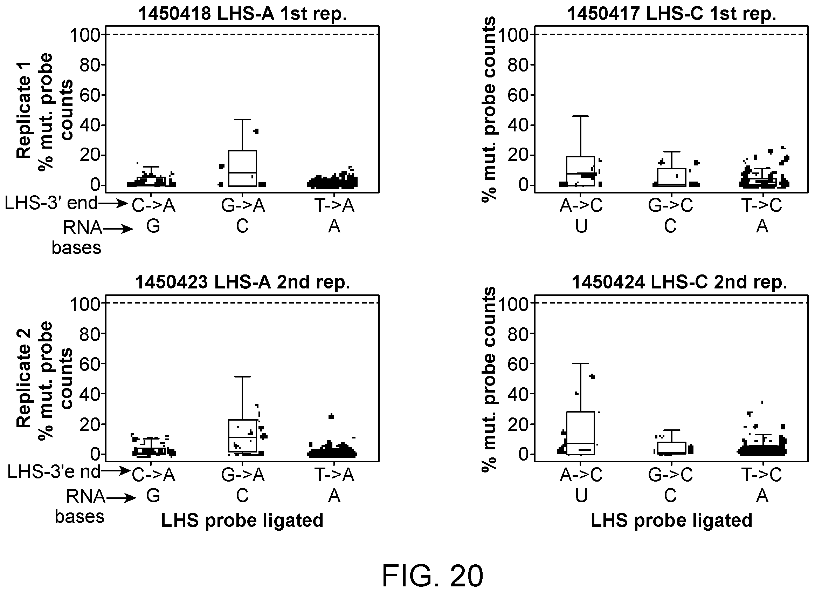

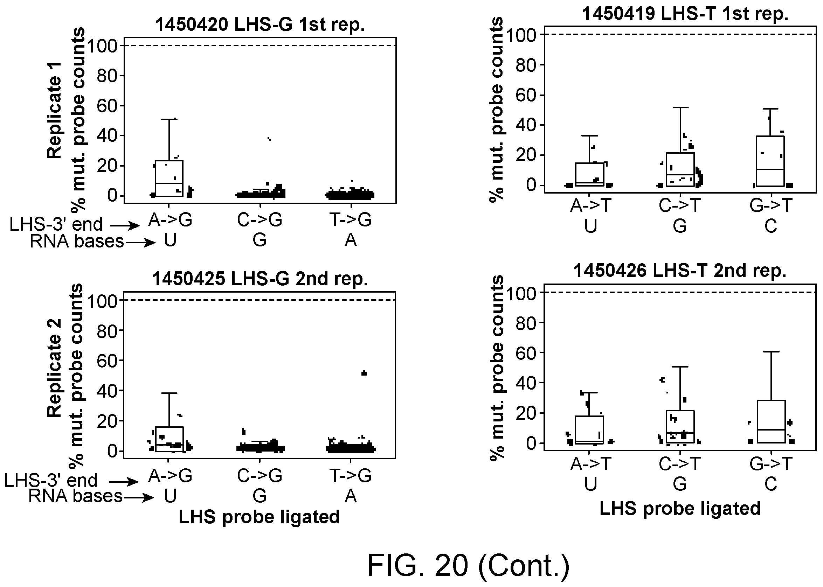

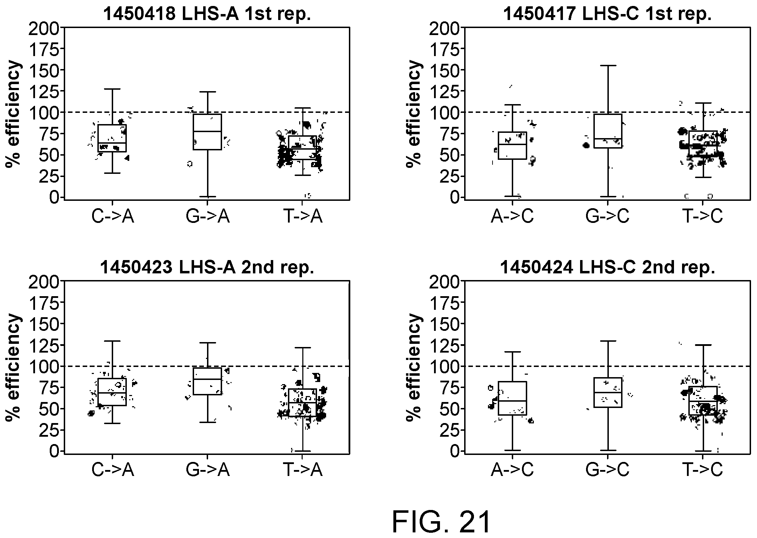

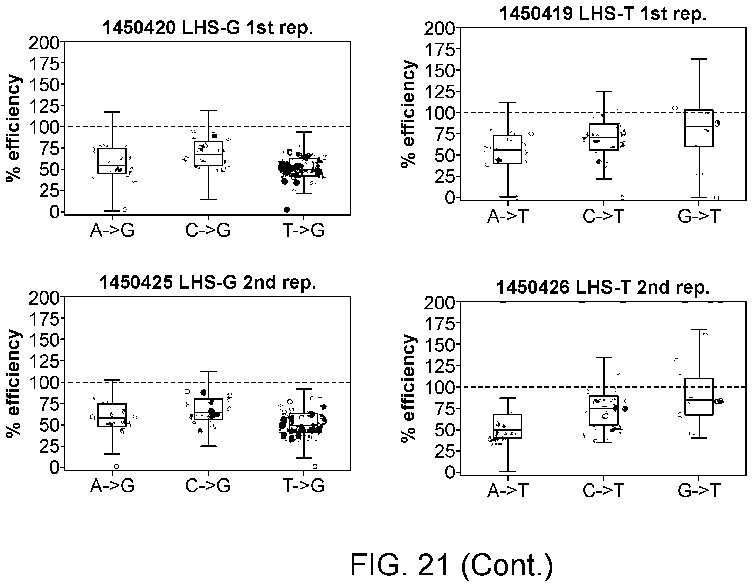

**FIG. 20** shows replicate (n=2) specificity graphs for the spike-in scenario. Probe specificity was lower than expected, but the resulting data from the replicates were consistent. Probe ligation efficiency or sensitivity was also lower when compared to control samples **(****FIG. 21****).** Some ligation events may have been hindered by non-specific hybridization, which can reduce the overall sensitivity. Some of the RTL probe sets did in fact overlap, even though that was not expected. As such, the specificity and efficiency data may have been negatively affected when overlap occurred.

### Example 4. Genetic variant detection using a two-step hybridization scenario.

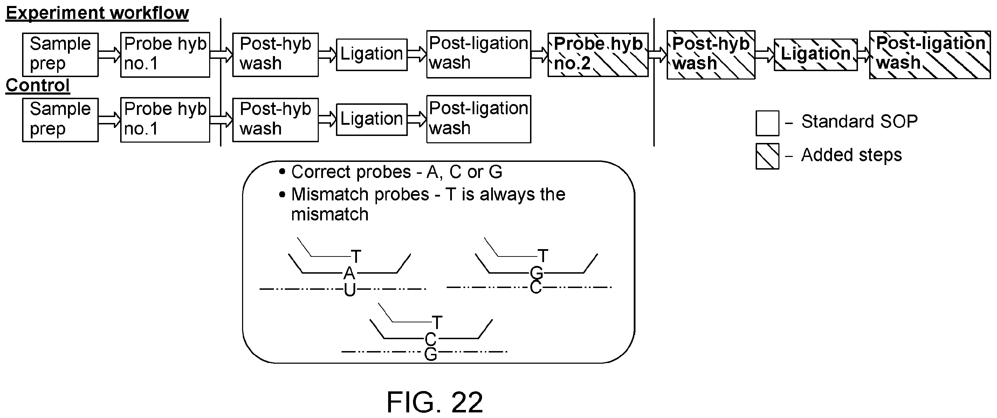

In this experiment, two sets of RTL probes were sequentially applied to a biological sample and hybridized with a wash step in between, in essence performing the probe hybridization twice instead of once with all the probes, including a 3' end mismatched or wt, present concurrently. In this example, two sets of RTL probes, one set having a mismatch T nucleotide at the 3' end of the LHS probe and a second set with the wt G nucleotide at the 3' end of the LHS probe, were used. In one set of experiments, the mismatch RTL probe set was first hybridized to the target nucleic acid and then the hybridized probes were ligated to generate ligation products. The sample was then washed to remove any non-hybridized/non-ligated probes. The second set of wt RTL probes were then hybridized to the target nucleic acid and ligated to generate a second set of ligation products. In one set of experiments, two post-ligation wash conditions were evaluated: one at 57°C and a second at 65°C. In a second set of experiments, the conditions remained the same except that the two probe sets were switched in sequence, such that the wt RTL probes were first applied to the biological sample to hybridize to the target nucleic acids and ligated, followed by a post-ligation wash (either at 57°C or 65°C), after which the mismatch RTL probes were then applied to the biological sample for target nucleic acid hybridization and ligation (see **FIG. 22****).**

The LHS and RHS probes hybridize to the target nucleic acid and are ligated together to generate a ligation product. The LHS probe, having either a mismatch at the 3' end or wt at the 3' end, further includes a functional domain that is not complementary to the target nucleic acid. The RHS probe includes a capture domain having a sequence that is not complementary to the target nucleic acid, but instead complementary to a capture domain of a capture probe on a spatial array.

A section of a FFPE MDA-MD-231 cell pellet tissue sample was placed on a slide and deparaffinized, eosin stained, and imaged. Following imaging, the tissue sample was destained and decrosslinked. The first RTL probe pairs were added to the tissue sample as previously described and were then allowed to hybridize overnight to their target nucleic acids in the tissue sample. Following hybridization, the probes were ligated together using SplintR ligase. After ligation, the tissue sample was washed (post-ligation wash) at either 57°C or 65°C, and the second RTL probe pairs were added to the tissue sample for hybridization and ligation, followed by second post-ligation wash at 57°C. Following the two-step hybridization workflow, the ligation products were released, hybridized to capture probes on a spatial array slide, and extension and library preparation for sequencing of the ligation products were performed per manufacturer's user guide (Visium CytAssist Spatial Gene Expression Reagent Kits, CG000495). After sequencing of the ligation products, the data was analyzed using SpaceRanger, and specificity and efficiency of the two-step hybridization probe scenario for identifying genetic variants was graphed.

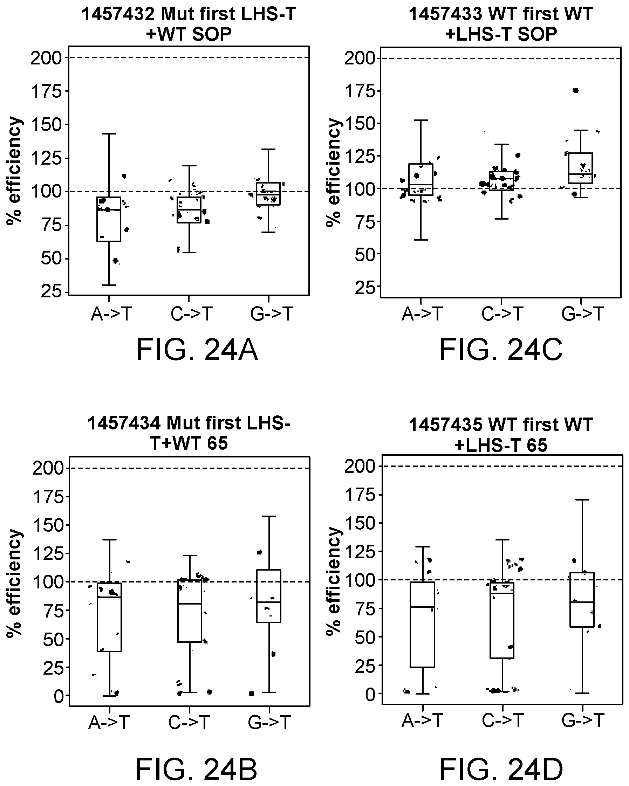

**FIG. 23** shows specificity graphs for the four different two-hybridization condition scenarios. Data show that hybridizing and ligating the wt RTL probes first followed by the mismatch RTL probes shows increased specificity **(****FIGs. 23C-23D****)** as compared to running the mismatch RTL probes first and the wt RTL probes second **(****FIGs. 23A-23B****).** However, the increase in temperature for the post-ligation wash increased specificity when running the mismatch RTL probes before the wt RTL probes **(****FIG. 23B** compared to **FIG. 23A****).** The data suggest that in some instances probes with lower melting temperatures might be dropping out when using the higher 65°C post-ligation wash. Efficiency was close to 100% as compared to control experiments (100% dotted line) where median UMI counts for all transcriptome probes were analyzed **(****FIGs. 24A-24D****).** Efficiency data were normalized using only probes with a Tₘ > 80°C to mitigate the drop out of lower Tₘ probes.

### Example 5. Genetic variant detection using an asymmetric probe scenario.

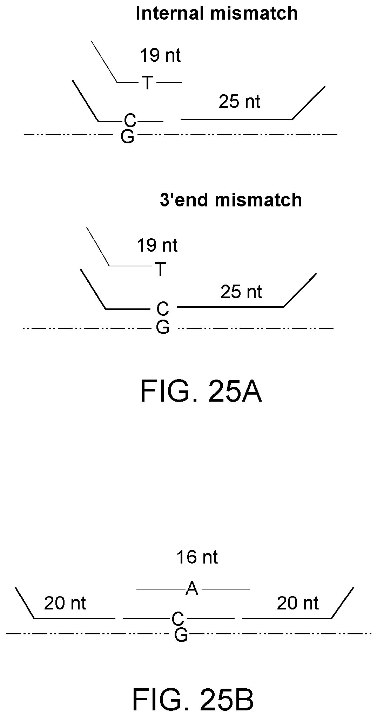

In this experiment, shorter length LHS probes and the RHS RTL probes were combined to create an asymmetric probe scenario. That is, a shorter length (e.g., 19nt) LHS probe having either a mismatch T nucleotide or a wt C nucleotide that is complementary to the target nucleic acid was combined with a longer length (e.g., 25nt) RHS probe. The LHS and RHS probes were hybridized to a target nucleic acid and then ligated together to generate a ligation product. As demonstrated in **FIG. 25A****,** the shorter LHS probes were 19nt in length and RHS RTL probes were 25nt in length. The LHS probe further included a functional sequence that is not complementary to the target nucleic acid. The RHS included a capture domain that is not complementary to the target nucleic acid, but is instead complementary to a capture domain of a capture probe on a spatial array.

A section of a FFPE Raji/Jurkat cell pellet tissue sample was placed on a slide and then deparaffinized, H&E stained, and imaged. Following imaging, the tissue sample was destained and decrosslinked. The shorter LHS probes (with either the mismatch or wt 3' end) and RHS probes were added to the tissue sample and allowed to hybridize overnight to their target nucleic acids in the tissue sample. Following hybridization, the probe pairs were ligated together using SplintR ligase. After the ligated probes were released from the target nucleic acid and hybridized to capture probes on the spatial array slide, extension and library preparation for sequencing of the ligation products were performed per manufacturer's user guide (Visium CytAssist Spatial Gene Expression Reagent Kits, CG000495). After sequencing of the ligation products, the data was analyzed using SpaceRanger, and specificity and efficiency of the asymmetric probe scenario for identifying genetic variants was graphed.

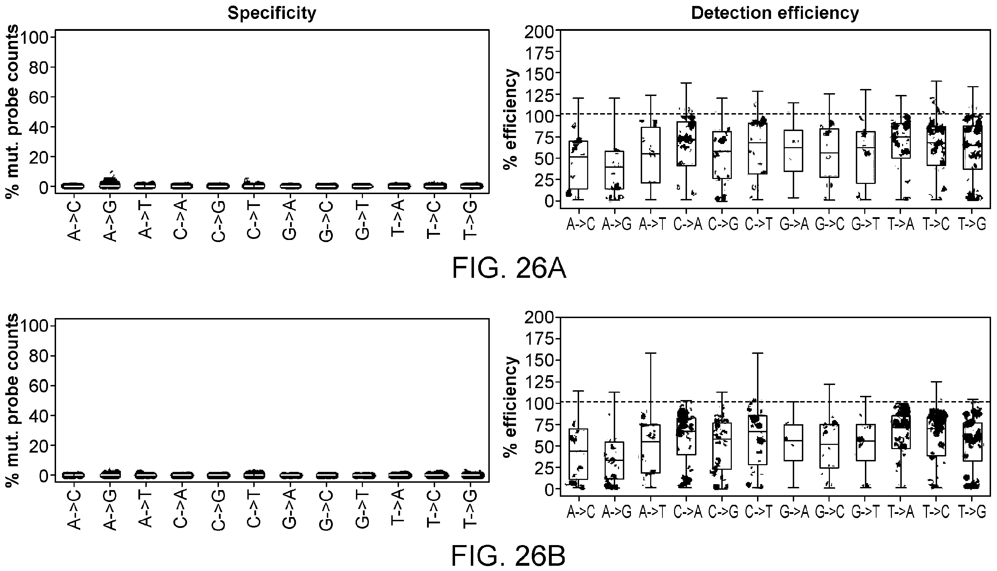

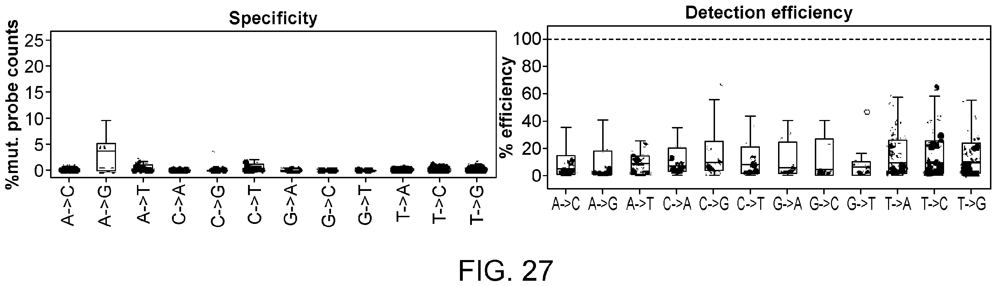

**FIGs. 26A-26B** show graphs for specificity and efficiency of two asymmetric probe scenarios: one with a mismatch at -9 of the shorter LHS probe **(****FIG. 26A****)** and a second with a mismatch at the 3' end of the shorter LHS probe **(****FIG. 26B****).** Regardless of the location of the mismatch on the shorter LHS probe, the graphs confirm specificity of the method, but at the cost of efficiency, but not to the extent as that seen when practicing the gap fill scenario for variant detection described in Example 6 below **(****FIG. 27****).** Efficiency was also variable and noisy, where some shorter LHS probes appear to hybridize better than others, while some of the shorter probes appear to have dropped out of the experiment. The variability might be a result of the shorter LHS probes having a lower Tₘ than the RHS probes.

### Example 6. Genetic variant detection using a gap fill/three-probe set scenario.

In this example, LHS and RHS RTL probes were combined with two sets of 16nt oligonucleotide probes: one oligonucleotide probe set having a mismatch A nucleotide, but otherwise fully complementary to a target nucleic acid, and the other oligonucleotide probe set having a wt C nucleotide that is complementary to the target nucleic acid. The LHS and RHS RTL probes were hybridized to the target nucleic acid leaving a gap of at least 16nt, such that the two 16nt oligonucleotide probe sets can compete to hybridize to the target nucleic acid region between the LHS and RHS probes, followed by ligation of the three hybridized probes (the LHS and RHS RTL probes and one of the 16nt oligonucleotide probes) into one ligation product. As demonstrated in **FIG. 25B****,** the LHS and RHS RTL probes were each 20nt long. The LHS probes further included a functional sequence on that is not complementary to the target nucleic acid. The RHS probes further included a capture domain that is not complementary to the target nucleic acid, but instead complementary to a capture domain of a capture probe on a spatial array.

A section of a FFPE Raji/Jurkat cell pellet tissue sample was placed on a slide and then deparaffinized, H&E stained, and imaged. Following imaging, the tissue sample was destained and decrosslinked. The decrosslinked tissue sample was then treated with DNase and washed multiple times prior to the addition of the target specific probes. The LHS and RHS RTL probes in combination with the two types of oligonucleotide probes (one with the A mismatch and the other with the wt C nucleotide) were added to the tissue sample and the RTL probes/oligonucleotide probes were allowed to hybridize overnight to their target nucleic acids in the tissue sample. Following probe/oligonucleotide hybridization, the three probes (LHS, oligonucleotide, and RHS) were ligated together using SplintR ligase. After the ligated probes were released from the target nucleic acid and hybridized to capture probes on the spatial array slide, extension and library preparation for sequencing of the ligation products were performed per manufacturer's user guide (Visium CytAssist Spatial Gene Expression Reagent Kits, CG000495). After sequencing of the ligation products, the data was analyzed using SpaceRanger, and specificity and efficiency of the gap fill scenario for identifying genetic variants was graphed.

**FIG. 27** shows exemplary graphs of an experiment using the three-probe experimental scenario to identify genetic variants. Specificity and efficiency for the three-probe experimental scenario were variable and lower compared with controls and as compared to specificity and efficiency when practicing the asymmetric probe scenario for genetic variant detection.

## Claims

1. A method for determining presence or absence of a genetic variant in a biological sample, the method comprising:
(a) providing an array comprising a plurality of capture probes, wherein a capture probe of the plurality of capture probes comprises: (i) a spatial barcode and (ii) a capture domain;
(b) contacting the biological sample with a plurality of first probes, wherein a first probe of the plurality of first probes comprises a sequence substantially complementary to a first sequence of a target nucleic acid in the biological sample;
(c) hybridizing the first probe to the target nucleic acid and extending the first probe using a reversible terminator nucleotide, thereby generating an extended first probe;
(d) contacting the biological sample with a plurality of second probes, wherein a second probe of the plurality of second probes comprises a sequence substantially complementary to a second sequence of the target nucleic acid in the biological sample;
(e) hybridizing the second probe to the target nucleic acid and ligating the extended first probe to the second probe, thereby generating a ligation product; and
(f) determining the presence or absence of the ligation product, thereby determining the presence or absence of the genetic variant in the biological sample.

2. The method of claim 1, wherein the reversible terminator nucleotide comprises a 3' blocking group, and optionally the 3' blocking group is a 3'-O-blocked reversible terminator nucleotide.

3. The method of claim 1, wherein the reversible terminator nucleotide comprises an unblocked reversible terminator nucleotide, and optionally the unblocked reversible terminator nucleotide comprises a 5-hydroxymethyl-2'-deoxy triphosphate derivative.

4. The method of any one of claims 1-3, wherein the target nucleic acid is DNA or RNA.

5. The method of claim 2, wherein the method further comprises removing the 3' blocking group after the extending in (c), and optionally the removing comprises reducing the 3' blocking group using a reducing agent or cleaving the 3' blocking group from the reversible terminator nucleotide.

6. The method of any one of claims 1-5, wherein the method further comprises removing the target nucleic acid from the ligation product.

7. The method of any one of claims 1-6 wherein the genetic variant is one or more single nucleotide polymorphisms, an insertion, or a deletion.

8. The method of any one of claims 1-7, wherein the determining in (f) comprises sequencing and/or fluorescent detection.

9. The method of any one of claims 1-8, wherein the capture probe comprises one or more functional domains, a cleavage domain, a unique molecular identifier (UMI), or a combination thereof.

10. The method of any one of claims 1-9, wherein the biological sample is disposed on the array.

11. The method of any one of claims 1-9, wherein the biological sample is disposed on a substrate and optionally, the method further comprises aligning the substrate comprising the biological sample with the array, such that at least a portion of the biological sample is aligned with at least a portion of the array.

12. The method of any one of claims 1-11, further comprising hybridizing the ligation product to the capture domain of the capture probe.

13. The method of any one of claims 1-12, further comprising determining (i) all or a portion of the sequence of the ligation product, or a complement thereof, and (ii) the sequence of the spatial barcode, or a complement thereof, and using the determined sequences of (i) and (ii) to determine a location of the genetic variant in the biological sample.

14. The method of any one of claims 1-13, wherein the method further comprises staining the biological sample and/or imaging the biological sample.

15. The method of any one of claims 1-14, wherein the biological sample is a tissue sample.

## Patentansprüche

1. Verfahren zum Bestimmen des Vorhandenseins oder Nichtvorhandenseins einer genetischen Variante in einer biologischen Probe, das Verfahren umfassend:
(a) Bereitstellen einer Anordnung umfassend eine Mehrzahl von Erfassungssonden, wobei eine Erfassungssonde der Mehrzahl von Erfassungssonden umfasst: (i) einen räumlichen Barcode und (ii) eine Erfassungsdomäne;
(b) Inkontaktbringen der biologischen Probe mit einer Mehrzahl von ersten Sonden, wobei eine erste Sonde der Mehrzahl von ersten Sonden eine Sequenz umfasst, die im Wesentlichen komplementär zu einer ersten Sequenz einer Zielnukleinsäure in der biologischen Probe ist;
(c) Hybridisieren der ersten Sonde an die Zielnukleinsäure und Verlängern der ersten Sonde unter Verwendung eines reversiblen Terminator-Nukleotids, wodurch eine verlängerte erste Sonde erzeugt wird;
(d) Inkontaktbringen der biologischen Probe mit einer Mehrzahl von zweiten Sonden, wobei eine zweite Sonde der Mehrzahl von zweiten Sonden eine Sequenz umfasst, die im Wesentlichen komplementär zu einer zweiten Sequenz der Zielnukleinsäure in der biologischen Probe ist;
(e) Hybridisieren der zweiten Sonde an die Zielnukleinsäure und Ligieren der verlängerten ersten Sonde an die zweite Sonde, wodurch ein Ligationsprodukt erzeugt wird; und
(f) Bestimmen des Vorhandenseins oder Nichtvorhandenseins des Ligationsprodukts, wodurch das Vorhandensein oder Nichtvorhandensein der genetischen Variante in der biologischen Probe bestimmt wird.

2. Verfahren nach Anspruch 1, wobei das reversible Terminator-Nukleotid eine 3'-Blockierungsgruppe umfasst und optional die 3'-Blockierungsgruppe ein 3'-O-blockiertes reversibles Terminator-Nukleotid ist.

3. Verfahren nach Anspruch 1, wobei das reversible Terminator-Nukleotid ein unblockiertes reversibles Terminator-Nukleotid umfasst und optional das unblockierte reversible Terminator-Nukleotid ein 5-Hydroxymethyl-2'-Desoxytriphosphat-Derivat umfasst.

4. Verfahren nach einem der Ansprüche 1 bis 3, wobei die Zielnukleinsäure DNA oder RNA ist.

5. Verfahren nach Anspruch 2, wobei das Verfahren ferner das Entfernen der 3'-Blockierungsgruppe nach dem Verlängern in (c) umfasst und optional das Entfernen das Reduzieren der 3'-Blockierungsgruppe unter Verwendung eines Reduktionsmittels oder das Spalten der 3'-Blockierungsgruppe von dem reversiblen Terminator-Nukleotid umfasst.

6. Verfahren nach einem der Ansprüche 1 bis 5, wobei das Verfahren ferner das Entfernen der Zielnukleinsäure aus dem Ligationsprodukt umfasst.

7. Verfahren nach einem der Ansprüche 1 bis 6, wobei die genetische Variante ein oder mehrere Einzelnukleotid-Polymorphismen, eine Insertion oder eine Deletion ist.

8. Verfahren nach einem der Ansprüche 1 bis 7, wobei das Bestimmen in (f) das Sequenzieren und/oder den Fluoreszenznachweis umfasst.

9. Verfahren nach einem der Ansprüche 1 bis 8, wobei die Erfassungssonde eine oder mehrere funktionelle Domänen, eine Spaltungsdomäne, eine eindeutige molekulare Kennung (UMI) oder Kombinationen davon umfasst.

10. Verfahren nach einem der Ansprüche 1 bis 9, wobei die biologische Probe auf dem Array angeordnet ist.

11. Verfahren nach einem der Ansprüche 1 bis 9, wobei die biologische Probe auf einem Substrat angeordnet ist und optional wobei das Verfahren ferner das Ausrichten des Substrats, das die biologische Probe umfasst, auf das Array umfasst, sodass mindestens ein Teil der biologischen Probe auf mindestens einen Teil des Arrays ausgerichtet ist.

12. Verfahren nach einem der Ansprüche 1 bis 11, ferner umfassend das Hybridisieren des Ligationsprodukts an die Erfassungsdomäne der Erfassungssonde.

13. Verfahren nach einem der Ansprüche 1 bis 12, ferner umfassend das Bestimmen (i) der gesamten oder eines Teils der Sequenz des Ligationsprodukts oder eines Komplements davon und (ii) der Sequenz des räumlichen Barcodes oder eines Komplements davon und das Verwenden der bestimmten Sequenzen von (i) und (ii) für ein Bestimmen einer Position der genetischen Variante in der biologischen Probe.

14. Verfahren nach einem der Ansprüche 1 bis 13, wobei das Verfahren ferner das Färben der biologischen Probe und/oder das Abbilden der biologischen Probe umfasst.

15. Verfahren nach einem der Ansprüche 1 bis 14, wobei die biologische Probe eine Gewebeprobe ist.

## Revendications

1. Procédé pour déterminer la présence ou l'absence d'un variant génétique dans un échantillon biologique, le procédé comprenant :
(a) la fourniture d'un réseau comprenant une pluralité de sondes de capture, dans lequel une sonde de capture de la pluralité de sondes de capture comprend : (i) un code à barres spatial et (ii) un domaine de capture ;
(b) la mise en contact de l'échantillon biologique avec une pluralité de premières sondes, dans lequel une première sonde de la pluralité de premières sondes comprend une séquence sensiblement complémentaire d'une première séquence d'un acide nucléique cible dans l'échantillon biologique ;
(c) l'hybridation de la première sonde à l'acide nucléique cible et l'extension de la première sonde en utilisant un nucléotide terminateur réversible, générant ainsi une première sonde étendue ;
(d) la mise en contact de l'échantillon biologique avec une pluralité de deuxièmes sondes, dans lequel une deuxième sonde de la pluralité de deuxièmes sondes comprend une séquence sensiblement complémentaire d'une deuxième séquence de l'acide nucléique cible dans l'échantillon biologique ;
(e) l'hybridation de la deuxième sonde à l'acide nucléique cible et la ligature de la première sonde étendue à la deuxième sonde, générant ainsi un produit de ligature ; et
(f) la détermination de la présence ou de l'absence du produit de ligature, déterminant ainsi la présence ou l'absence du variant génétique dans l'échantillon biologique.

2. Procédé selon la revendication 1, dans lequel le nucléotide terminateur réversible comprend un groupe de blocage en 3', et facultativement le groupe de blocage en 3' est un nucléotide terminateur réversible bloqué en 3'-O.

3. Procédé selon la revendication 1, dans lequel le nucléotide terminateur réversible comprend un nucléotide terminateur réversible non bloqué, et facultativement le nucléotide terminateur réversible non bloqué comprend un dérivé de 5-hydroxyméthyl-2'-désoxytriphosphate.

4. Procédé selon l'une quelconque des revendications 1 à 3, dans lequel l'acide nucléique cible est de l'ADN ou de l'ARN.

5. Procédé selon la revendication 2, dans lequel le procédé comprend en outre l'élimination du groupe de blocage en 3' après l'extension en (c), et facultativement l'élimination comprend la réduction du groupe de blocage en 3' en utilisant un agent réducteur ou le clivage du groupe de blocage en 3' du nucléotide terminateur réversible.

6. Procédé selon l'une quelconque des revendications 1 à 5, dans lequel le procédé comprend en outre l'élimination de l'acide nucléique cible du produit de ligature.

7. Procédé selon l'une quelconque des revendications 1 à 6, dans lequel le variant génétique est un ou plusieurs polymorphismes de nucléotide simple, une insertion ou une délétion.

8. Procédé selon l'une quelconque des revendications 1 à 7, dans lequel la détermination en (f) comprend un séquençage et/ou une détection par fluorescence.

9. Procédé selon l'une quelconque des revendications 1 à 8, dans lequel la sonde de capture comprend un ou plusieurs domaines fonctionnels, un domaine de clivage, un identifiant moléculaire unique (UMI), ou des combinaisons de ceux-ci.

10. Procédé selon l'une quelconque des revendications 1 à 9, dans lequel l'échantillon biologique est disposé sur un réseau.

11. Procédé selon l'une quelconque des revendications 1 à 9, dans lequel l'échantillon biologique est disposé sur un substrat, et facultativement le procédé comprend en outre l'alignement du substrat avec le réseau, de telle sorte qu'au moins une portion de l'échantillon biologique est alignée avec au moins une portion du réseau.

12. Procédé selon l'une quelconque des revendications 1 à 11, comprenant en outre l'hybridation du produit de ligature au domaine de capture de la sonde de capture.

13. Procédé selon l'une quelconque des revendications 1 à 12, comprenant en outre la détermination (i) de la totalité ou d'une portion de la séquence du produit de ligature ou d'un complément de celle-ci, et (ii) de la séquence du code à barres spatial ou d'un complément de celle-ci, et l'utilisation de la séquence déterminée de (i) et (ii) pour déterminer un emplacement du variant génétique dans l'échantillon biologique.

14. Procédé selon l'une quelconque des revendications 1 à 13, dans lequel le procédé comprend en outre la coloration de l'échantillon biologique et/ou l'imagerie de l'échantillon biologique.

15. Procédé selon l'une quelconque des revendications 1 à 14, dans lequel l'échantillon biologique est un échantillon tissulaire.
